# EUROPEAN PATENT APPLICATION

(11) **EP 2 088 190 A1**
(43) Date of publication of application: **12.08.2009**
(21) Application number: 07831542.1
(22) Date of filing: 09.11.2007
(51) Int. Cl.: C12N 5/06, A61L 27/00

(54) **METHOD FOR CULTURE AND PASSAGE OF PRIMATE EMBRYONIC STEM CELL, AND METHOD FOR INDUCTION OF DIFFERENTIATION OF THE EMBRYONIC STEM CELL**

(30) Priority: 09.11.2006 JP 2006303929
(71) Applicant: Japan as represented by President of International Medical Center of Japan, Tokyo 162-8655 (JP); Mitsubishi Tanabe Pharma Corporation, Chuo-ku Osaka-shi Osaka 541-8505 (JP)
(72) Inventor: YUO, Akira, Shinjuku-ku Tokyo 162-8655 (JP); TOBE, Kumiko, Shinjuku-ku Tokyo 162-8655 (JP); SAEKI, Koichi, Shinjuku-ku Tokyo 162-8655 (JP); NAKAHARA, Masako, Shinjuku-ku Tokyo 162-8655 (JP); NAKAMURA, Naoko, Shinjuku-ku Tokyo 162-8655 (JP); YOGISASHI, Yoshiko, Shinjuku-ku Tokyo 162-8655 (JP); MATSUYAMA, Satoko, Shinjuku-ku Tokyo 162-8655 (JP); YONEDA, Asako, Shinjuku-ku Tokyo 162-8655 (JP)
(74) Representative: HOFFMANN EITLE
(86) International application number: PCT/JP2007/071811
(87) International publication number: WO 2008/056779

(57) **Abstract**

The present invention provides a method for subculturing primate embryonic stem cells, and a method for inducing differentiation of the same cell into a vascular endothelial cell and a blood cell. The present invention provides a method comprising culturing primate embryonic stem cells in a medium containing a protein component without using feeder cells and cytokines in a container coated with an extracellular matrix, detaching colonies of the resulting embryonic stem cells in the presence of a cytodetachment agent, and plating the colonies in the similar medium, and a method comprising culturing primate embryonic stem cells in a serum-containing or not containing medium in the presence of cytokine, adhesion-culturing the resulting embryoid body or embroyid body-analogous cellular aggregate in the presence of a cytokine to obtain specific precursor cells, and separating non-adherent cells and adherent cells from the specific precursor cells to obtain blood cells and vascular endothelial precursor cells.

## Description

### Technical field

The present invention relates to a method for culturing and subculturing primate embryonic stem cells wherein said method keeps the primate embryonic stem cells in an undifferentiated state, a method for inducing differentiation of the primate embryonic stem cell into various cells such as a blood cell, a vascular endothelial precursor cell and the like, a method for expanded reproduction of the resulting cell, as well as a hemocyte, a vascular endothelial precursor cell and the like.

### Background Technique

An embryonic stem cell having a pluripotency, which can be differentiated into a variety of cells, was established in 1980's regarding a mouse. A method for culturing and subculturing it is already known. However, a method for continuously culturing and subculturing embryonic stem cells derived from a primate including a human, which keeps the cells undifferentiated, has not been established yet.
A human embryonic stem cell is extremely important for investigation in the field of biology and medicine, as well as for the clinical practice. Particularly, in the field of regeneration medicine and medical transplantation, stable supply of human embryonic stem cells as a fundamental material for preparing internal organs is demanded. For example, an internal organ and a tissue prepared from a human embryonic stem cell are promising because those could be applied to regeneration medicine and medical transplantation and further, those would enable a pre-clinical study using a human internal organ, implementation of which has been limited so far. In addition, if various internal organs can be prepared from a human embryonic stem cell, conducting a pharmacological test during the preparation could predict influence of a drug on a fetus. Because a toxicity test of a drug or the like on a fetus (pregnant mother's body) is not currently conducted from an ethical point of view and only accumulation of accidental cases gives drug information, a risky drug administration might be overlooked due to lack of information on drug safety. However, if various internal organs can be prepared from a human embryonic stem cell, conducting a pharmacological test during the preparation could avoid such risk.
As described above, establishment of a human embryonic stem cell and establishment of a safe method for culturing and subculturing it would promote research and development regarding a technique for preparing an internal organ etc, and are expected to greatly contribute to development of medical industry and perinatal care including research and application of medical care regarding transplantation/regeneration and drug discovery.

The human embryonic stem cell was firstly established by Thomson et al. in USA in 1998 and, thereafter, a culture method and induction of differentiation into a variety of tissues has been investigated. For example, Patent Document 1 (WO99/20741) describes a culture containing embryonic stem cells without substantially containing feeder cells, which is obtained by culturing primate embryonic stem cells using an extracellular matrix prepared from mouse embryonic fibroblasts (MEF). However, in the above-described established human embryonic stem cell, chromosome aberration is induced after subculturing for a short term (specifically, since accumulation of chromosome aberration is confirmed after ten or more times passages, the risk in clinical application is pointed out (Non-Patent Document 1 (Draper et al., Nature Biotechnology, Vol. 22, pp.53-54, (2004)))).

From the viewpoint of the clinical application to a human, it is not preferable to use feeder cells derived from a heterogeneous animal such as a mouse for culturing human embryonic stem cells. Then, in 2006, Ludwig et al. reported a feeder-free culture method in which a heterogeneous animal-derived component has been removed as much as possible. However, since this method uses a large amount of four kinds of expensive human-derived extracellular matrices in addition to a large amount of cocktail of synthetic cytokines (Non-Patent Document 2 (Ludwig et al., Nature Biotechnology, Vol. 24, pp.185-187, (2006))), it is not suitable for clinical application from an economical point of view.

Thereafter, a human embryonic stem cell line was established also in Australia and Sweden, and chromosome aberration is reported as an extremely rare case even though more than one year elapsed after the human embryonic stem cell line was established (Non-Patent Document 3 (Buzzard, Natural Biotechnology, Vol. 22, pp.381-382, (2004))). Although these human embryonic stem cells can be subcultured and maintained without a feeder cells, a special equipment and a special skill are required for culture/subculture/freeze preservation of the cells and thus handling of a large amount of cells is substantially difficult (Non-Patent Document 3 (Buzzard, Natural Biotechnology, Vol. 22, pp.381-382, (2004))). In addition, because use of a large amount of a synthetic cytokine is required for these feeder-free cultures in which a conditioned medium of cells derived from a heterogeneous animal is not used, great economical burden has to be involved in the research itself. Therefore, regarding a human embryonic stem cell, the technique of stable feeder-free culture has not been established yet.

In the light of the foregoing, development of a method for culturing and subculturing embryonic stem cells of a primate including a human, which satisfies the followings: not using a conditioned medium of a heterogeneous animal-derived cell, a feeder cell and a synthetic cytokine; being able to safely subculture the cells by means of a simple and basic culturing technique without inducing chromosome aberration for a long term; allowing freeze-thaw of the cells and high viability of the cells after the thaw; and being safe, low-cost and high feasibility, is a globally urgent task.
The aforementioned technique is expected to greatly contribute to promotion of a wide variety of researches, development of clinical medicine regarding transplantation/regeneration and development of perinatal care.

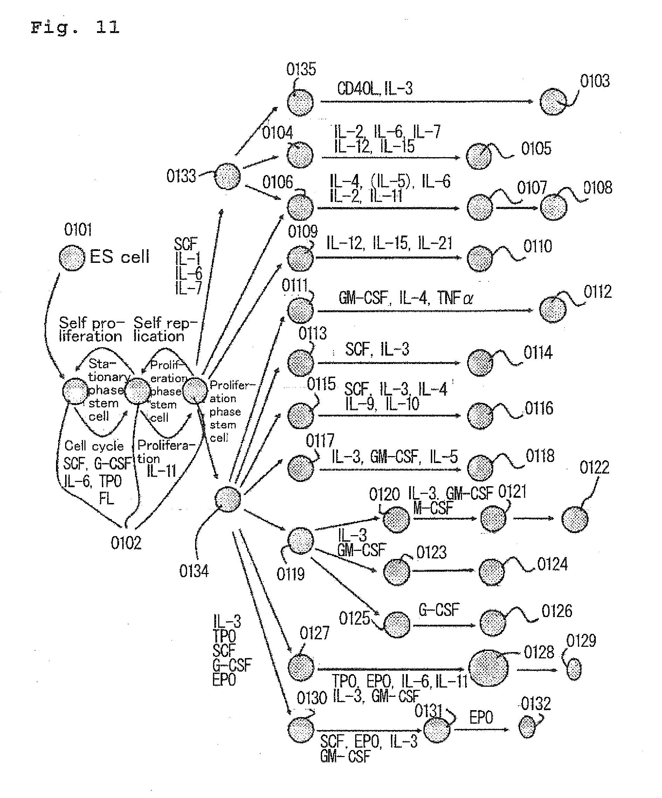

Since a suitable induction of differentiation of a primate embryonic stem cell enables to prepare a large amount of desired precursor cells and mature cells which are available for various applications, a method for effectively inducing a differentiation of an embryonic stem cell and further culturing the cell has been investigated. As one of differentiation sequences of an embryonic stem cell, a differentiation into blood-associated cells such as a vessel and a hemocyte is recognized (see Fig. 11).

A vascular endothelial cell is a fundamental element for constructing a vessel. A vessel is distributed in almost all living body tissues except for a few parts of tissues such as cartilage and sclera, and plays an extremely important role in supplying nutrients and removing waste products. Therefore, the vascular endothelial cell is useful for therapeutic angiogenesis of an occlusive vascular disorder accompanied with arteriosclerosis which is one disease of lifestyle-related diseases that tend to increase in recent years. In addition, it is known that in a process of regenerating a variety of tissues including a brain, guidance of the vascular endothelial cell is important for directing a tissue-specific stem cell (a neural stem cell etc.) to migrate to a suitable position. In addition, from a fetal stage, the vascular endothelial cell works as a precursor tissue of a blood cell and it plays an important role in hemocyte production. In an adult, it plays an important role as a "niche" (scaffold) of a hematopoietic stem cell. That is, the vascular endothelial cell is extremely important not only as a simple component of a vessel but also for regeneration of an entire tissue including a nerve and a blood cell, and thus, when regeneration medicine is generally considered, a regulated production of vascular endothelial cells is an important problem to be solved.

Therapeutic angiogenesis using a vascular endothelial precursor cell obtained from a living body tissue (peripheral blood, bone marrow blood) has been tried, but regeneration of the vascular endothelial cell from a transplanted cell has not been directly confirmed so far. In addition, a mature vascular endothelial cell obtained from a living body has already lost the proliferating ability and it is difficult to prepare a large amount of samples, regarding a primate. Thus, a basic research on the vascular endothelial cell is very delayed. Further, as described later, since primary cultured vascular endothelial cells obtained from a human living body (particularly, commercially available freeze-thawed primary cultured vascular endothelial cells) have often lost during in vitro expansion of the culture,an inherent nature possessed by a cell in living body, a drawback is the point that a nature of the vascular endothelial cell in a living body is not correctly reflected. Therefore, for the purpose of development of research and regeneration medicine regarding a vessel etc, a method of safely and effectively preparing vascular endothelial precursor cells and mature vascular endothelial cells which are obtained from human embryonic stem cells using feeder-free culture and a method for subculturing to maintain them are desired.

However, in the case of using conventional method, one problem is that an efficiency of differentiation of a primate embryonic stem cell into a vascular endothelial precursor cell is as extremely low as 2% or less (Non-Patent Document 4 (Sone et al., Circulation, Vol. 107, pp.2085-2088, (2003)), and Non-Patent Document 5 (Levenberg et al., Proceeding of Natural Academy of Science, USA, Vol. 99, pp.4391-4396, (2002))), while an efficiency of differentiation of a mouse embryonic stem cell into a vascular endothelial precursor cell is 90% or more. In addition, regarding not only a primate embryonic stem cell but also a mouse embryonic stem cell, a method for preparing vascular endothelial precursor cells which can be subcultured using feeder-free culture has not been established.

As described above, establishment of a technique that can use to effectively prepare, culture and produce a large amount of vascular endothelial precursor cells and mature vascular endothelial cells, which can be obtained from primate embryonic stem cells and stably subcultured using feeder-free culture, becomes a globally urgent problem to be solved for developing a new drug efficacy/toxicity test which is useful for making a progress in a regeneration/transplantation medicine, basic medical research regarding a vessel, and a pre-clinical trial. In addition, development of such technique is extremely useful for a basic medical research, development of clinical practice and healthcare industry (e.g. drug discovery).

On the other hand, a blood (hemocyte) cell plays an important role in an immune system. It exerts a resistance to invasion by a foreign substance, and it exerts an effect on a cancer etc. (NK cell) and an effect on leukemia etc. (hematopoietic stem cell etc.) and the like. Further, because, due to its tissue plasticity,a hematopoietic stem cell enables transdifferentiation into a cell necessary for a variety of diseases and a granulocyte/macrophage precursor cell is fused with a cell of an injured tissue to promotes tissue regeneration, they are very important for medical care.
However, since it is almost impossible to in vitro expand hematopoietic stem cells (bone marrow blood, umbilical blood etc.) obtained from a living body, an amount of the hematopoietic stem cells to be used for medical transplantation is limited (for example, a sample from one donor can be administered to only one patient), and use for basic medical research including a cell culture experiment is substantially impossible. In addition, regarding a mature hemocyte of a primate, because it is difficult to prepare a large amount of samples as compared with an experimental animal such as a mouse, fundamental research is much delayed.

Therefore, not only for application to regeneration medicine and treatment of a disease but also for research, a method for safely and effectively preparing from a human embryonic stem cell, blood cells or hematopoietic stem cells using a feeder-free culture, and a method for subculturing and maintaining the prepared cells are desired.
The resulting blood cells such as erythrocytes are free from contamination with AIDS or hepatitis C virus, and are useful for improvement in safety of treatment. Further, a problem of hospital infection can be also overcome by transfusing leukocytes containing a neutrophil or the like derived from an embryonic stem cell for the purpose of strengthening an immune system, the function of which has been reduced by chemical therapy in a cancer. In addition, since a blood cell leads to strengthening of a natural curing force, preparation of a blood cell from an embryonic stem cell is thought to provide much profit to medical care.
Further, a hematopoietic stem cell can be utilized per se not only for transplantation but also for preparation of blood cells. However, in the case of using feeder free culture, an efficacy of inducing differentiation of an embryonic stem cell of a primate including a human into a hemocyte is not sufficiently high, and an amount of prepared hemocytes is limited. Particularly, a preparation efficacy of a hematopoietic stem cell is very low (preparation efficacy of hematopoietic stem cell is around 5%) (Non-Patent Document 6 (Chadwick et al., Blood, Vol. 102, pp.906-915, (2003))). Moreover, in order to maintain a sufficient amount of hemocytes, it is necessary to conduct an experiment by repeating it tens times or hundreds times, but induction of differentiation using a conventional method is transiently effective, and it is impossible to actually apply these methods to regeneration/transplantation medicine.

That is, establishment of the technique that allows safe induction of differentiation of a primate embryonic stem cell into a blood cell (hematopoietic stem cell and mature hemocyte), maintenance of the blood cell, and preparation of a large amount of blood cells is becoming an important problem to be solved not only for medical application but also for basic medical research and further for pharmacological research such as drug efficacy analysis. Development of such technique is extremely useful for application to clinical practice, basic medical research, and healthcare industry (e.g. drug discovery).

In addition, a stroma cell is indispensible for hematopoiesis in a living body, but use of stroma cells for transplantation/reproduction medicine is very limited and it is also substantially impossible to culture stroma cells in vitro because a hematopoietic stroma cell obtained from a living body (fetal liver, adult bone marrow etc.) rapidly loses the function due to subculturing or freeze-thaw. Therefore, for development of transplantation medicine regarding a blood disease, and development of basic medical research on a hematopoietic stroma cell, it is essential to establish the technique which allows preparation of hematopoietic stroma cells from an embryonic stem cell. However, preparation of hematopoietic stroma cells from an embryonic stem cell of any species including a mouse has not been successful yet.

Further, blood-associated cells (blood cells, hematopoietic stem cells etc.) are useful for analyzing the "hematopoietic mechanism" of a primate including a human. Even now, there is lack of information regarding the hematopoietic mechanism, particularly, initial hematopoiesis during development. For example, the presence of "hemangioblast" which is a common precursor cell regarding a vascular endothelial cell and a blood cell was finally verified regarding fish (zebrafish) very recently (2006, September) (Non-Patent Document 7), although the presense has been suggested for a long time regarding development of a mouse (Non-Patent Document 7 (Vogeli et al., Nature, Vol. 443, pp.337-339, (2006))). If the technique allowing to prepare this "hemangioblast" from a human embryonic stem cell, which has possibility of correctly mimicking initial hematopoiesis in a living body of human is established, it is thought that the hematopoietic mechanism is understood better, and the technique is useful for development of research and medical care.

In the light of the foregoing, a method for making/preparing from an embryonic stem cell of a primate including a human, blood-associated cells (e.g.hematopoietic stem cell, mature hemocyte, "hemangioblast" which is a common precursor cell of vascular endothelial cell and blood cell, a vascular endothelial cell, hematopoietic stroma cell etc.) which can be very efficiently and stably subcultured, maintained, expanded reproduced, and freeze-thawed using feeder-free culture is generally desired for making a progress in regeneration/transplantation medicine, fundamental research regarding the hematopoietic mechanism, and development of a new drug efficacy/toxicity test which could be useful for pre-clinical trial.
[Patent Document 1] Pamphlet of International Publication No. WO 99/20741
[Non-Patent Document 1] Draper et al., Nature Biotechnology, Vol. 22, pp.53-54, (2004)
[Non-Patent Document 2] Ludwig et al., Nature Biotechnology, Vol. 24, pp.185-187, (2006)
[Non-Patent Document 3] Buzzard, Natural Biotechnology, Vol. 22, pp.381-382, (2004)
[Non-Patent Document 4] Sone et al., Circulation, Vol. 107, pp.2085-2088, (2003)
[Non-Patent Document 5] Levenberg et al., Proceeding of Natural Academy of Science, USA, Vol. 99, pp.4391-4396, (2002)
[Non-Patent Document 6] Chadwick et al., Blood, Vol. 102, pp.906-915, (2003)
[Non-Patent Document 7] Vogeli et al., Nature, Vol. 443, pp.337-339, (2006)

### Disclosure of the Invention

### Problems to be Solved by the Invention

An object of the present invention is to provide a method for subculturing primate embryonic stem cells wherein said method safely keeps said primate embryonic stem cells in undifferentiated state for a long term without inducing chromosome aberration or the like.
Another object of the present invention is to provide a method for efficiently and safely inducing differentiation of a primate embryonic stem cell into a vascular endothelial cell, a vascular endothelial precursor cell, a blood cell, a myeloid lineage cell, a hematopoietic stem cell or the like.
A further object of the present invention is to provide a vascular endothelial cell, a blood cell, a myeloid lineage cell, a hematopoietic stem cell or the like obtained by the aforementioned method.
Other objects of the present invention will be appreciated throughout the specification.

### Means for Solving the Problems

The present inventors has made an intensive research for the puropose of establishing a method for supplying a sufficient amount of primate embryonic stem cells suitable for clinical application and, at the same time, for the purpose of efficiently and safely preparing a variety of cells or internal organs from a primate embryonic stem cell, and then, found out that under the constant conditions, a primate embryonic stem cell is continued to be maintained stably, which is induced to suitably differentiate into a variety of cells. Finally, they reached a completion of the present invention.
That is, the overview of the present invention includes the followings.
[1] A method for culturing and subculturing primate embryonic stem cells, comprising:
   (A) a step of culturing primate embryonic stem cells in a medium containing a protein component in a container coated with an extracellular matrix without using feeder cells and cytokines,
   (B) a step of detaching colonies of the embryonic stem cells prepared by the step (A) in the presence of a cytodetachment agent, and
   (C) a step of plating the colonies of the embryonic stem cells prepared by the step (B) in a container coated with an extracellular matrix in a medium containing a protein component without using feeder cells and cytokines.
[2] The method for culturing and subculturing according to [1], wherein in the step (A), the primate embryonic stem cells are cultured until the size of the colony of the primate embryonic stem cells becomes about 2-4 times larger.
[3] The method for culturing and subculturing according to [1] or [2], wherein the protein component in the step (A) is serum albumin.
[4] The method for culturing and subculturing according to any one of [1] to [3], wherein the cytodetachment agent in the step (B) is at least one selected from the group consisting of trypsin, collagenase, and dispase.
[5] The method for culturing and subculturing according to any one of [1] to [4], wherein the extracellular matrix in the step (A) is at least one selected from the group consisting of human collagen, human laminin, human vitronectin, human fibronectin, human serum, a degradation product thereof and a synthetic peptide thereof.
[6] A method for preparing blood cells and/or vascular endothelial precursor cells from a primate embryonic stem cell, comprising:
   (A) a step of suspension-culturing primate embryonic stem cells in a medium containing serum or a serum-free medium containing a serum substitute in the presence of a cytokine to prepare an embryoid body or an embryoid body-analogous cellular aggregate,
   (B) a step of adhesion-culturing the embryoid body or embryoid body-analogous cellular aggregate prepared by the step (A) in the presence of the cytokine to prepare specific precursor cells containing non-adherent cells and adherent cells, and
   (C) a step of separating the non-adherent cells and the adherent cells from the specific precursor cells prepared by the step (B).
[7] The method for preparing vascular endothelial precursor cells and/or blood cells from a primate embryonic stem cell according to [6], wherein culturing of the step (A) is performed until an embryoid body-like cellular aggregate is formed.
[8] The method for preparing vascular endothelial precursor cells and/or blood cells from a primate embryonic stem cell according to [6] or [7], wherein the cytokine is at least one selected from the group consisting of vascular endothelial growth factor (VEGF), bone morphogenetic protein 4 (BMP4), stem cell factor (SCF), Flt3-ligand (FL), interleukin 6 (IL6), interleukin 3 (IL3), glanulocyte colony stimulating factor (G-CSF), megakaryocyte proliferation factor (TPO), oncostatin M (OSM), fibroblast growth factor 2 (FGF2) and granulocyte macrophage colony stimulating factor (GM-CSF).
[9] The method for preparing vascular endothelial precursor cells and/or blood cells from a primate embryonic stem cell according to any one of [6] to [8], wherein a cytodetachment agent is used for separating an adherent cell of a specific precursor cell in the step (C).
[10] The method for preparing vascular endothelial precursor cells and/or blood cells from a primate embryonic stem cell according to [9], wherein the cytodetachment agent is at least one selected from the group consisting of trypsin, collagenase, and dispase.
[11] A method for preparing blood cells, myeloid lineage cells, hematopoietic stroma cells and/or hematopoietic stem cells from a primate embryonic stem cell, comprising:
   (A) a step of suspension-culturing primate embryonic stem cells in a medium containing serum or a serum-free medium containing a serum substitute in the presence of cytokine to prepare an embryoid body or an embryoid body-analogous cellular aggregate,
   (B) a step of adhesion-culturing the embryoid body or the embryoid body-analogous cellular aggregate prepared by the step (A) in the presence of a cytokine to prepare specific precursor cells containing non-adherent cells and an adherent cells, and
   (C) a step of culturing the specific precursor cells prepared by the step (B) together with separating the non-adherent cells.
[12] The method for preparing blood cells, myeloid lineage cells, hematopoietic stroma cells and/or hematopoietic stem cells from a primate embryonic stem cell according to [11], wherein culturing of the step (A) is performed until an embryoid body is formed.
[13] The method for preparing blood cells, myeloid lineage cells, hematopoietic stroma cells and/or hematopoietic stem cells from a primate embryonic stem cell according to [11] or [12], wherein the cytokine is at least one selected from the group consisting of vascular endothelial growth factor (VEGF), bone morphogenetic protein 4 (BMP4), stem cell factor (SCF), Flt3-ligand (FL), interleukin 6 (IL6), interleukin 3 (IL3), glanulocyte colony stimulating factor (G-CSF), megakaryocyte proliferation factor (TPO), oncostatin M (OSM), fibroblast growth factor 2 (FGF2) and granulocyte macrophage colony stimulating factor (GM-CSF).
[14] The method for preparing blood cells, myeloid lineage cells, hematopoietic stroma cells and/or hematopoietic stem cells from a primate embryonic stem cell according to any one of [11] to [13], wherein a cytodetachment agent is used for separating an adherent cell of a specific precursor cell, in the step (C).
[15] The method for preparing blood cells, myeloid lineage cells, hematopoietic stroma cells and/or hematopoietic stem cells from a primate embryonic stem cell according to [14], wherein the cytodetachment agent is at least one selected from the group consisting of trypsin, collagenase, and dispase.
[16] A substantially isolated vascular endothelial precursor cell, which is induced to differentiate from a primate embryonic stem cell by the method according to any one of [6] to [10].
[17] A substantially isolated blood cell, which is induced to be differentiated from a primate embryonic stem cell by the method according to any one of [6] to [14].
[18] A substantially isolated hematopoietic stroma cell, which is induced to be differentiated from a primate embryonic stem cell by the method according to any one of [11] to [14].
[19] A substantially isolated hematopoietic stem cell, which is induced to be differentiated from a primate embryonic stem cell by the method according to any one of [11] to [14].
[20] A substantially isolated myeloid lineage cell, which is induced to be differentiated from a primate embryonic stem cell by the method according to any one of [11] to [14].
[21] A composition comprising the substantially isolated vascular endothelial precursor cell, blood cell, hematopoietic stroma cell, or hematopoietic stem cell accroding to any one of [16] to [19].

### Effect of the Invention

According to the method for culturing and subculturing primate embryonic stem cells of the present invention, primate embryonic stem cells can be safely cultured with keeping the cells in the undifferentiated state, using a simple device and a method without inducing a cellular disorder such as chromosome aberration and the like. In addition, according to the present method, primate embryonic stem cells can be cultured to be maintained in an undifferentiation state at low cost, and it is possible to generally meet to a demand in regeneration medicine and research field.
According to the present invention, a "specific precursor cell" which is identified as a common precursor cell regarding a vascular endothelial cell, a blood cell and the like can be safely and very efficiently prepared.
In addition, according to the present invention, it is possible to prepare in large quantity, vascular endothelial cells, blood cells, hematopoietic stem cells and myeloid lineage cells, each of which have high re-productivity and allow stable subculture and freeze-thaw.
Further, according to the present invention, various cells can be easily supplied as a material for safe and secure blood products for transfusion (including hematopoietic stem cell transplantation, glanulocyte transfusion, myeloid lineage cell administration), as a material for a treatment of a vascular damage and improvement of a topical blood stream or as a material suitable for clinical use in medical care for the purpose of promoting regeneration of other various tissues.
Further, according to the present invention, it firstly enables to supply in a large quantity, a cell population having a nature which correctly mimics a living body tissue of a primate, especially a human. Since these cells can be also adequately used for a test for efficacy or toxicity of a drug, they can greatly contribute to development of not only clinical practice but also medical industry.

### BRIEF DESCRIPTION OF THE DRAWINGS

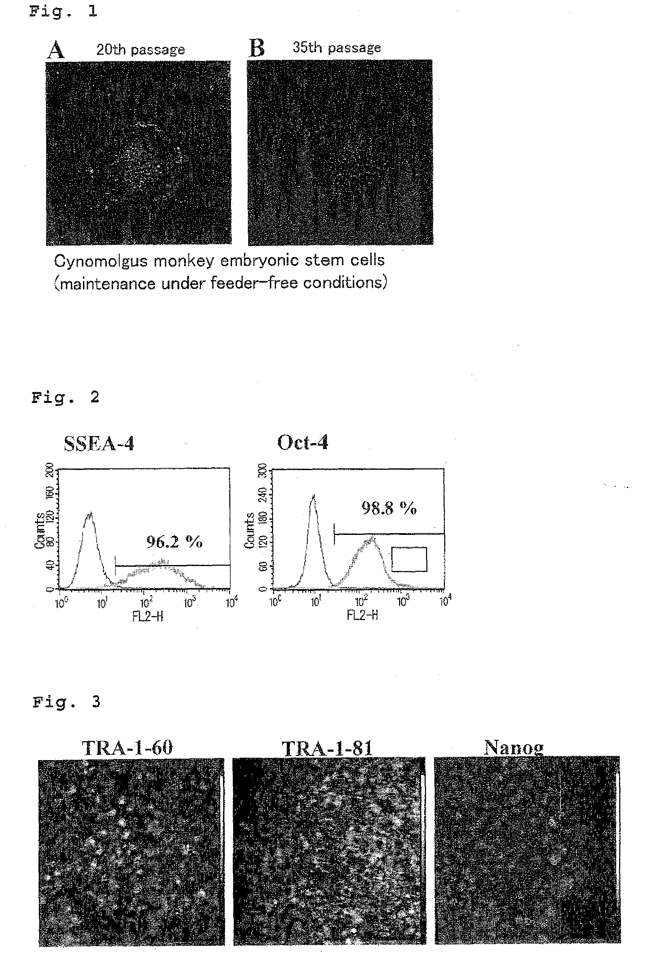

Fig. 1 shows photographs of a colony of cynomolgus embryonic stem cells cultured using a culture dish coated with a Matrigel^{®} matrix according to "a method for subculturing with keeping the undifferentiation state without using feeder cells and cytokines" of the present invention, which is shown in Example 1. Cells shown in the photographs are obtained by subculture of thawed cells frozen at 20th passage (A) and 35th passage (B). A scale bar indicates 100 µm.
Fig. 2 shows results regarding expression of SSEA-4 and Oct-4, each of which is an undifferentiation state marker, which were obtained by flow cytometry analysis of 20th passage cynomolgus monkey embryonic stem cells cultured on a culture dish coated with a Matrigel^{®} matrix according to "a method for subculturing with keeping the undifferentiation state without using feeder cells and cytokines" of the present invention, which is shown in Example 1. Very high expression of both of them (> 95%) is confirmed.
Fig. 3 shows photographs indicating expressions of Tra-1-60, Tra-1-81, and Nanog, each of which is an undifferentiation state marker, determined by immunostaining of 20th passage cynomolgus monkey embryonic stem cells cultured on a culture dish coated with a Matrigel^{®} matrix according to "a method for subculturing with keeping the undifferentiation state without using feeder cells and cytokines" of the present invention, which is shown in Example 1. On almost all cells, expression of each marker is confirmed. A scale bar indicates 100 µm.

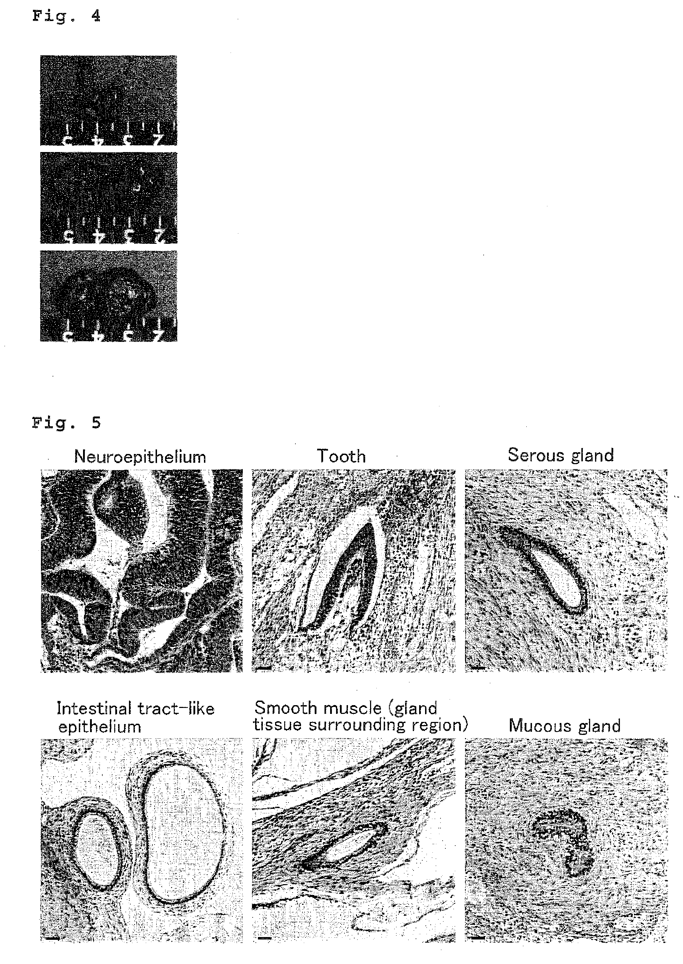

Fig. 4 shows photographs of a testis obtained two months after 21st passage cynomolgus monkey embryonic stem cells, which were cultured on a culture dish coated with a Matrigel^{®} matrix according to "a method for subculturing with keeping the undifferentiation state without using feeder cells and cytokines" of the present invention shown in Example 1, were grafted under a testis membrane of three immunodeficient mice (SCID mice). As shown in Fig. 4, formation of a tumor was confirmed in all of three animals.
Fig. 5 shows a tissue specimen of the above-described tumor (hematoxylin/eosin staining). As described, a neuroepithelium, a tooth, a secretion gland, an intestinal tract-like epithelium, and a smooth muscle are confirmed.

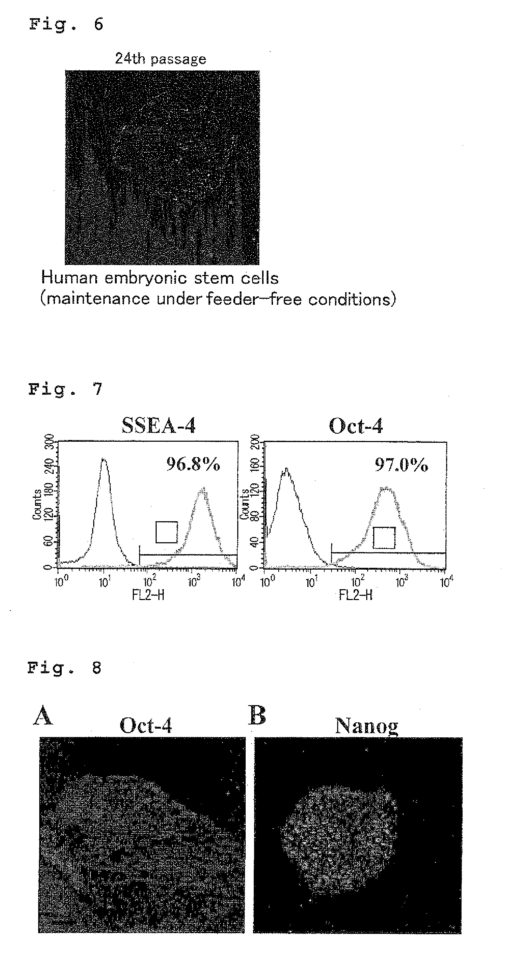

Fig. 6 shows a phase-contrast micrograph of a colony of 24th passage human embryonic stem cells, which were cultured on a culture dish coated with a Matrigel^{®} matrix according to "a method for subculturing with keeping the undifferentiation state without using feeder cells and cytokines" of the present invention shown in Example 2. A scale bar indicates 100 µm.
Fig. 7 shows expressions of SSEA-4 and Oct-4, each of which is an undifferentiation state marker, which were determined by flow cytometry analysis of 20th passage human embryonic stem cells cultured on a culture dish coated with a Matrigel^{®} matrix according to "a method for subculturing with keeping the undifferentiation state without using feeder cells and cytokines" of the present invention, which is shown in Example 2. Very high expressions of both of them (> 95%) are confirmed.
Fig. 8 shows photographs indicating expressions of Oct-4 (A) and Nanog (B), each of which is an undifferentiation state marker, determined by immunostaining of 25th passage human embryonic stem cells cultured on a culture dish coated with a Matrigel^{®} matrix according to "a method for subculturing with keeping the undifferentiation state without using feeder cells and cytokines" of the present invention, which is shown in Example 2. It is confirmed that both proteins were expressed on almost all cells. A scale bar indicates 100 µm.

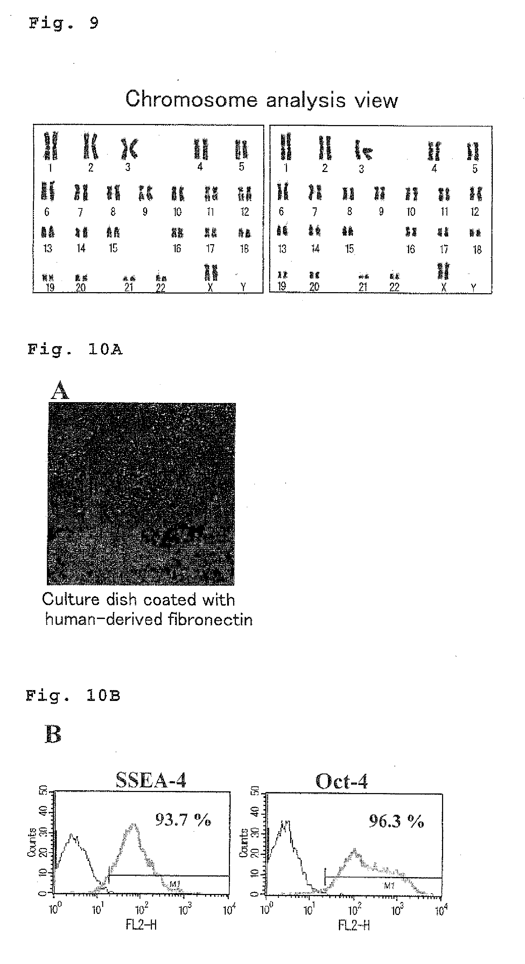

Fig. 9 shows a chromosome analysis view of a human embryonic stem cell (G band method). The left figure shows a result obtained in maintenance of the cell according to a conventional method (i.e. co-culture using fetal mouse fibroblast as feeder cell) as recommended by the institution which established the cell, while right figure shows a result obtained after 20th passage according to "a method for culturing without using feeder cells and cytokines" of the present invention. It was confirmed that no chromosome aberration occurred.
Fig. 10A shows a phase-contrast micrograph of 4th passage human embryonic stem cells cultured on a culture dish coated only with human-derived fibronectin (5 µg/cm²) according to "a method for subculturing with keeping the undifferentiation state without using feeder cells and cytokines" of the present invention, which is shown in Example 3. It can be understood that the undifferentiated morphology is retained.
Fig. 10B shows results regarding expression of SSEA-4 and Oct-4 each of which is an undifferentiation state marker, obtained by flow cytometry analysis of the cells of Fig. 10A. High expression of both markers was confirmed.

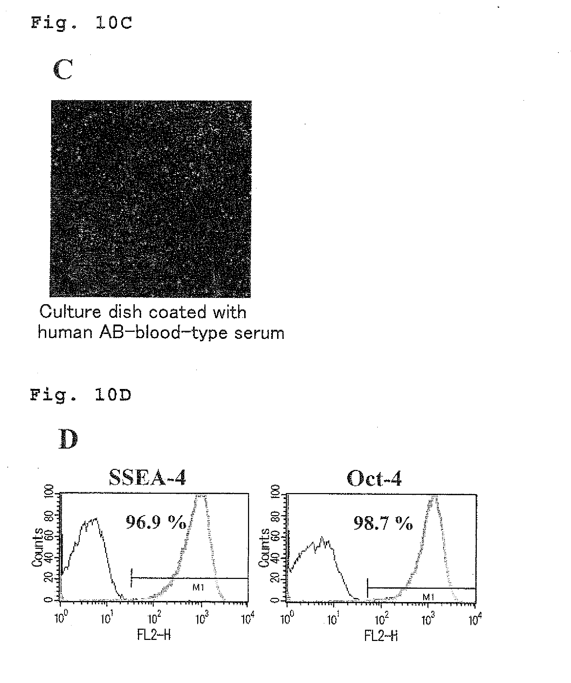

Fig. 10C shows a phase-contrast micrograph of 4th passage human embryonic stem cells cultured on a culture dish coated only with human AB-blood type serum according to "a method for subculturing with keeping the undifferentiation state without using feeder cells" of the present invention. It can be understood that the undifferentiated morphology is retained.
Fig. 10D shows results regarding expressions of SSEA-4 and Oct-4, each of which is an undifferentiation state marker, obtained by flow cytometry analysis of the cell of Fig. 10C. High expressions of both markers were confirmed.
Fig. 11 shows a differentiation tree from a hematopoietic stem cell to a blood cell.

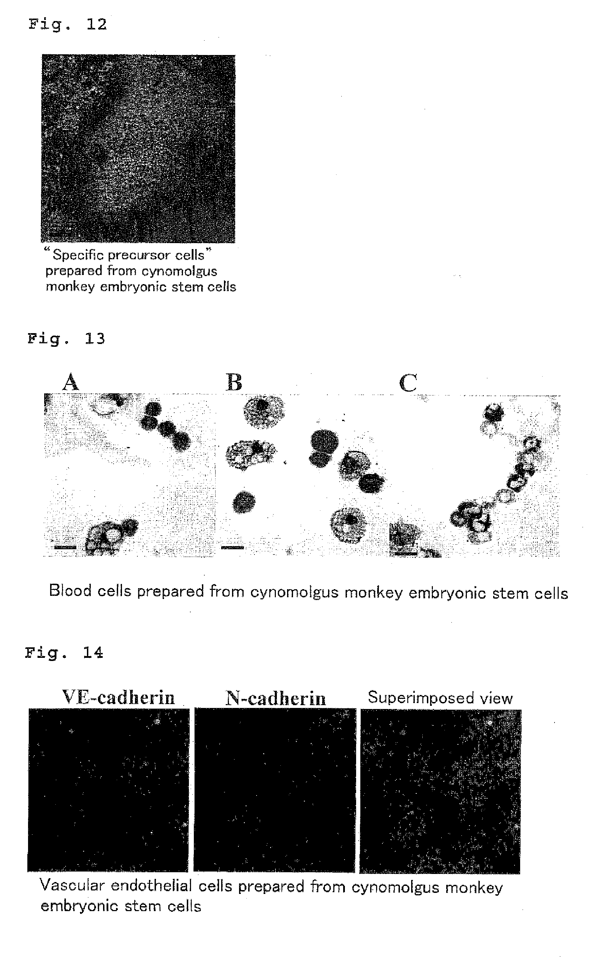

Fig. 12 shows a "specific precursor cell" (organization consisting of sac-like structure and spherical cell population), which is common precursor of both a vascular endothelial precursor cell and a blood cell, prepared from a cynomolgus monkey embryonic stem cell in the presence of bovine fetal serum according to the technique of the present invention relating to inducing differentiation of vascular endothelial cell/blood cell without using feeder cells and expanded reproduction thereof. A scale bar indicates 100 µm.
Fig. 13 shows Wright-Giemsa staining (A) and special staining (myeloperoxidase staining (B) and esterase double staining (C)) of mature hemocytes prepared from spherical cells contained in the "specific precursor cells" as described above. Various myeloid lineage cells, that is, cells which are in each of various differentiation stages ranging from bone-marrow blast to mature hemocyte (e.g. neutrophil and macrophage), are observed. A scale bar indicates 20 µm.
Fig. 14 shows an immunostaining study on expression of VE-cadherin, which is a vascular endothelial cell-specific marker, and expression of N-cadherin, which is one of vascular endothelial cell markers, regarding vascular endothelial cells prepared from the "specific precursor cells" as described above. Regarding almost all cells, expressions of VE-cadherin and N-cadherin are confirmed. A scale bar indicates 50 µm.

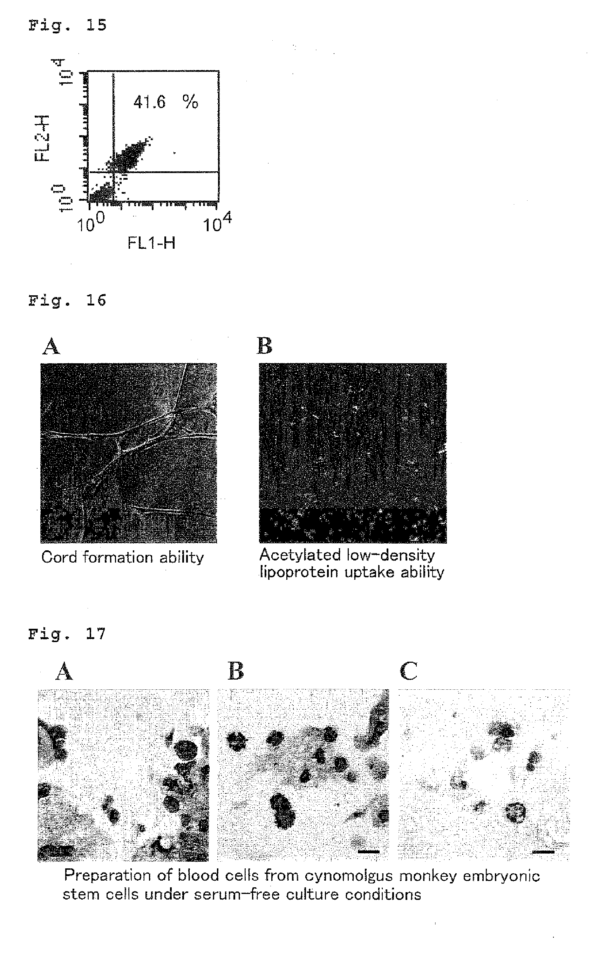

Fig. 15 shows expression of PECAM1 which is a mature vascular endothelial cell marker on vascular endothelial cells prepared from the "specifc precursor cells" as described above, confirmed by flow cytometry analysis using double staining with VE-cadherin which is a pan-vascular endothelial cell marker and a vascular endothelial cell-specific marker. An axis of abscissas represents an intensity of PECAM1 expression, and an axis of ordinate represents an intensity of VE-cadherin expression. Expressions of both of them are confirmed in 40% or more of the cells.
Fig. 16 shows the cord formation ability (A) and the acetylated low-density lipoprotein (AC-LDL) uptake ability (B), which were investigated so as to confirm the mature function of vascular endothelial cells prepared from the "specifc precursor cells" as described above. In any case, acquisition of the mature function is confirmed at high efficiency.
Fig. 17 shows Wright-Giemsa staining (A) and special staining (myeloperoxidase staining (B) and esterase stainining (C)) of mature hemocytes prepared from cynomolgus monkey embryonic stem cells using a serum-free culture (using KNOCKOUT^{®} SR) according to "a method for inducing differentiation into vascular endothelial cell and blood cell without using feeder cells" of the present invention, shown in Example 4. Various myeloid lineage cells, that is, cells which are in each of various differentiation stages ranging from a bone-marrow blast to a mature hemocyte (neutrophil and macrophage) are observed. A scale bar indicates 20 µm.

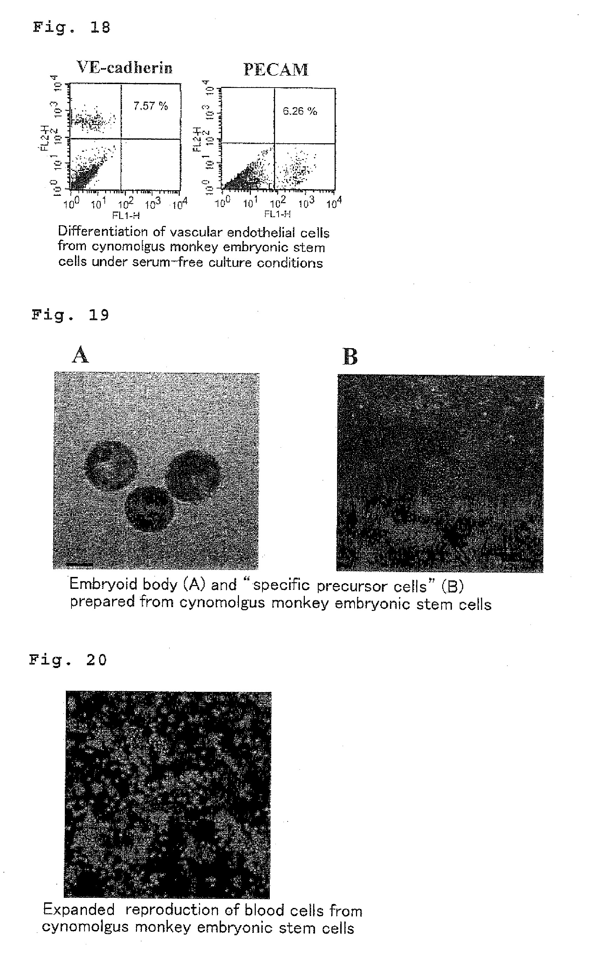

Fig. 18 shows expressions of VE-cadherin and PECAM1 on vascular endothelial cells prepared from cynomolgus monkey embryonic stem cells using the serum-free culture (using KNOCKOUT^{®} SR) according to "a method for inducing differentiation into vascular endothelial cell and blood cell without using feeder cells" of the present invention, shown in Example 5. Although previously, it was believed that induction of differentiation of an endothelial cell could not be achieved without using serum, expressions of VE-cadherin and PECAM1 on cell membrane are confirmed at efficiency of a few % or more.
Fig. 19 shows a phase-contrast micrograph of an embryoid body cell (A) prepared from a cynomolgus monkey embryonic stem cell in the presence of bovine fetal serum, according to "a method for inducing differentiation into vascular endothelial cell and blood cell without using feeder cells" of the present invention, and a "specific precursor cell" (organization consisting of sac-like structure and spherical cell population (B), which is common precursor of both a vascular endothelial precursor cell and a blood cell, obtained by adhesion culture of an embryoid body, shown in Example 6. A scale bar indicates 100 µm.
Fig. 20 shows a phase-contrast micrograph showing a circumstance in which expanded reproduction of blood cells obtained by culturing the "specific precursor cell" was achieved. Both a hematopoietic stroma cell (adherent cell) and a hemocyte produced therefrom (non-adherent cell) are confirmed. A scale bar indicates 100 µm.

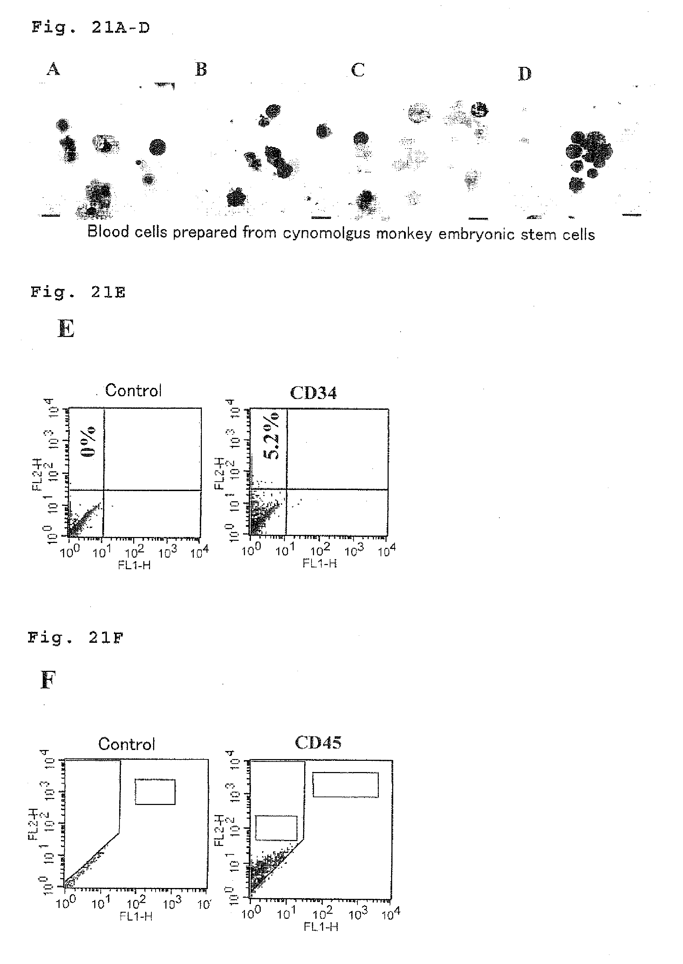

Figs. 21A-D show Wright-Giemsa staining image (A) and special staining (myeloperoxidase staining (B), esterase double staining (C), neutrophilic alkaline phosphatase staining (D)) of hemocytes (non-adherent cell) recovered in a process of the expanded reproduction of blood cells as described above. Various myeloid lineage cells, that is, cells which are in each of various differentiation stages ranging from bone-marrow blast to mature hemocyte (e.g. neutrophil and macrophage) are observed. A scale bar indicates 20 µm.
Fig. 21E shows results confirmed by flow cytometry, regaridng expression of CD34, which is a hematopoietic stem cell marker, on hemocytes (non-adherent cells) shown in Figures 21A-D.
Fig. 21F shows results confirmed by flow cytometry, regarding expression of CD45 (F), which is a pan-hematopoietic cell marker, on a hemocyte (non-adherent cell) shown in Figures. 21A-D.

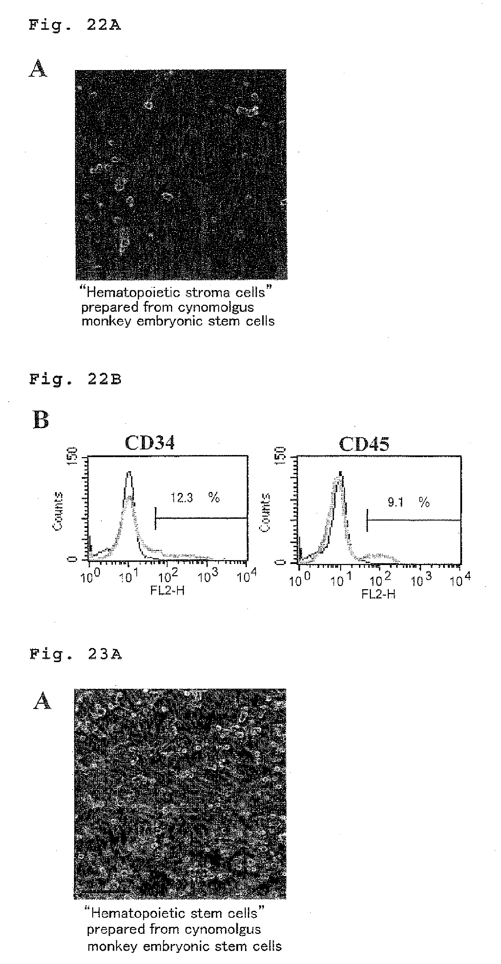

Fig. 22A shows a phase-contrast micrograph (scale bar indicates 100 µm) of subculturable "hematopoietic stroma cells" prepared from the "specific precursor cell" as described above.
Fig. 22B shows results of flow cytometry analysis regarding expressions of CD34 and CD45 on subculturable "hematopoietic stroma cells" prepared from the "specific precursor cell" as described above. Both CD45 which is a pan-hematopoietic cell marker and CD34 which is a marker of a hematopoietic stem cell are almost negative (it is thought that a small amount of a positive cell is caused by contamination of a few hematopoietic stem cells adhered to hematopoietic stroma cells).
Fig. 23A shows a phase-contrast micrograph of "CD34-positive and CD45-positive cells" prepared by long term culture (> 100 days) of "subculture-capable hematopoietic stem/precursor cells" which were prepared from the "specific precursor cell" as described above. Non-adherent cells and adherent cells are present as an admixture, but both can be interchangeable and thus it is thought that both are eqivalent cell populations (i.e. hematopoietic stem cells and equivalent thereof). A scale bar indicates 100 µm.

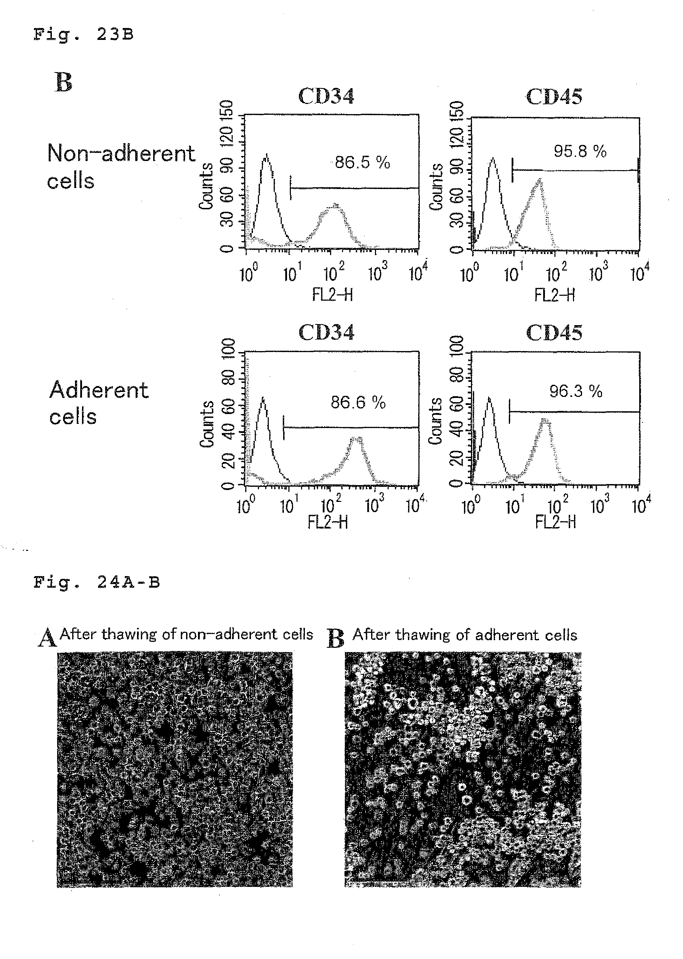

Fig. 23B shows results confirmed by flow cytometry, regarding expression of CD34 and CD54 on non-adherent cells and adherent cells of "CD34-positive and CD45-positive cells" prepared by long term culture (> 100 days) of "subculturable hematopoietic stem/precursor cells" which were prepared from the "specific precursor cell" as described above.
Fig. 24 shows phase-contrast micrographs showing circumstances of "expanded reproduction of blood cells", where each of non-adherent cells and adherent cells were freeze-thawed to restart the culture respectively. In each case, the presence of a hematopoietic stroma cell (adherent cell) and a hemocyte produced therefrom (non-adherent cell) was confirmed like the culture before freezing. A scale bar indicates 100 µm.

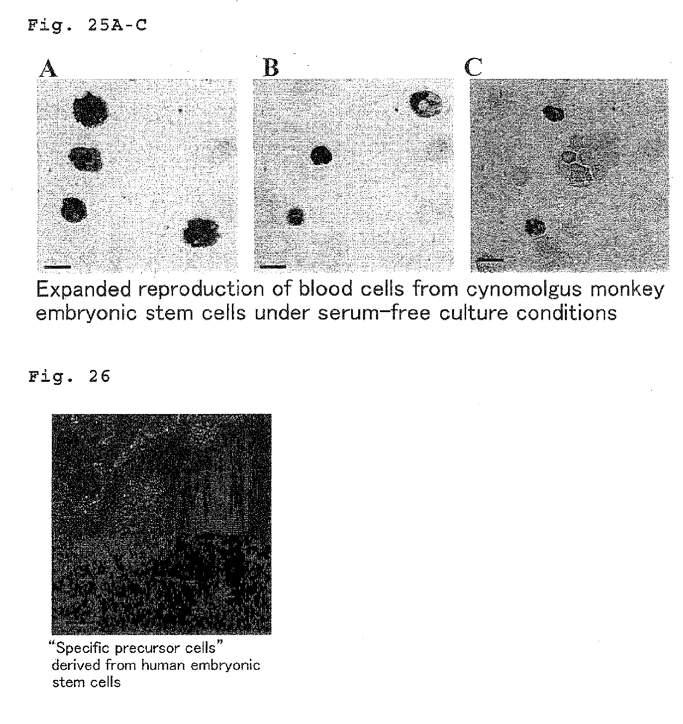

Fig. 25 shows Wright-Giema staining image (A) and special staining (myeloperoxidase staining (B), esterase double staining (C)) of hemocytes prepared from a cynomolgus monkey embryonic stem cells under the serum-free condition (using KNOCKOUT^{®} SR) according to "method for inducing differentiation into a vascular endothelial cell and a hemocyte without using feeder cells" of the present invention, shown in Example 7. Various myeloid lineage cells are observed. A scale bar indicates 20 µm.
Fig. 26 shows a "specific precursor cell" (organization consisiting of sac-like structure and spherical cell population) of both a vascular endothelial precursor cell and a blood cell, prepared from human embryonic stem cells according to "method for inducing differentiation into a vascular endothelial cell and a hemocyte without using feeder cells" of the present invention, shown in Example 8. A scale bar indicates 100 µm.

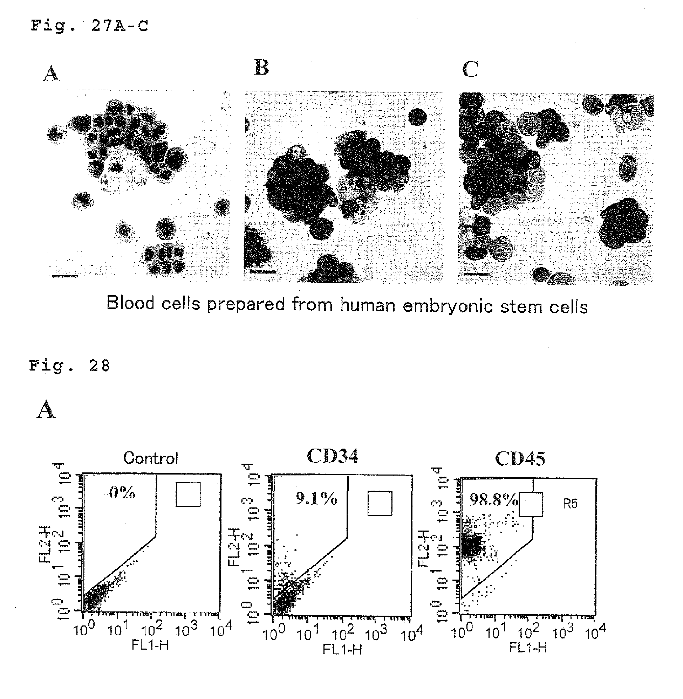

Fig. 27 shows Wright-Giema staining image (A) and special staining (esterase double staining (B), neutrophilic alkaline phosphatase staining (C)) of mature hemocytes (non-adherent cells) prepared from the "specific precursor cell" as described above. Various myeloid lineage cells, that is, cells are in each of various differentiation stages ranging from bone-marrow blast to mature hemocyte (neutrophil and macrophage) are observed. A scale bar indicates 20 µm.
Fig. 28 shows results confirmed by flow cytometry, regarding expressions of CD34 and CD45 on non-adherent cells prepared from human embryonic stem cells in the presence of bovine fetal serum according to "method of inducing of differentiation into a vascular endothelial cell and a hemocyte without using feeder cells" of the present invention, shown in Example 8. On almost all cells, CD45 which is a pan-hematopoientic cell marker is expressed, and it can be understood that hemocyte differentiation is induced at the very high efficiency. In addition, since about 10% of CD34-positive cells are detected, it is confirmed that a hematopoietic stem cell is also present.

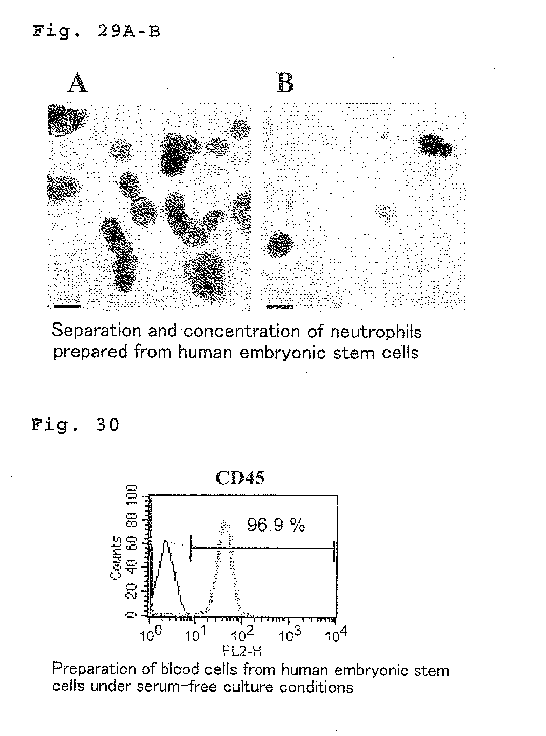

Fig. 29 shows results of concentration of neutrophils prepared from hemocytes according to "method of inducing differentiation into a vascular endothelial cell and a hemocyte without using feeder cells" of the present invention, shown in Example 8, by means of a density gradient centrifugation using Lymphoprep^{®} (manufactured by Sekisui Medical Co., Lid.). Herein, neutrophil is bluepurple-stained by neutrophilic alkaline phosphatase staining, and almost all cells are confirmed as a neutrophil after concentration (B) as compared with before concentration (A).
Fig. 30 shows a result confirmed by flow cytometry, regarding expression of CD45 on non-adherent cells prepared from human embryonic stem cells under the serum-free condition (using KNOCKOUT^{®} SR) according to "method for inducing differentiation into a vascular endothelial cell and a hemocyte without using feeder cells" of the present invention, shown in Example 9. On almost all cells, CD45 which is a pan-hematopoietic cell marker is expressed, and it can be understood that hemocyte differentiation is induced at the very high efficiency.

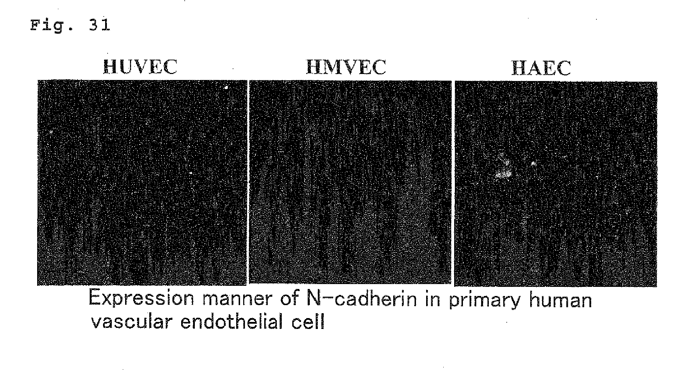

Fig. 31 shows immunostaining study on an intracellular expression manner of N-cadherin in three kinds of human primary cultured vascular endothelial cells (human umbilical vein endothelial cell (HUVAC), human microvessel enthothelial cell (HMVEC), human aorta endothelial cell (HAEC). It can be understood that cell membrane localization of N-cadherin is clearly found in vascular endothelial cells prepared from primate embryonic stem cells (shown in Fig. 14), while cell membrane localization of N-cadherin has been already lost in human primary cultured vascular endothelial cells. A scale bar indicates 50 µm.

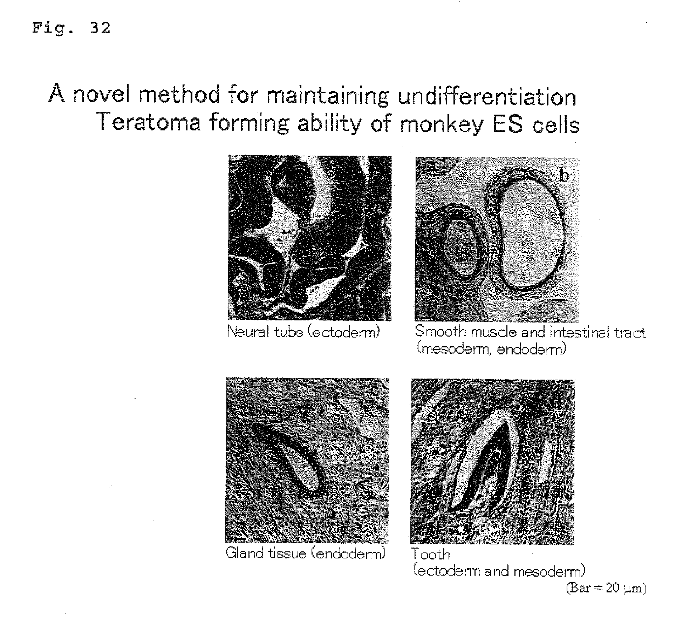

Fig. 32 shows tissue specimens of tumor (hematoxylin/eosin staining) shown in Example 10. As described, tridermic components of an ectoderm component (neuroepithelial cell; Fig. a, tooth enamel epithelium; Fig. d), mesoderm component (smooth muscle; Fig. b, tooth dentine; Fig. d), and endoderm component (intestinal tract epithelium; Fig. b, secretory gland tissue; Fig. c) can be recognized.

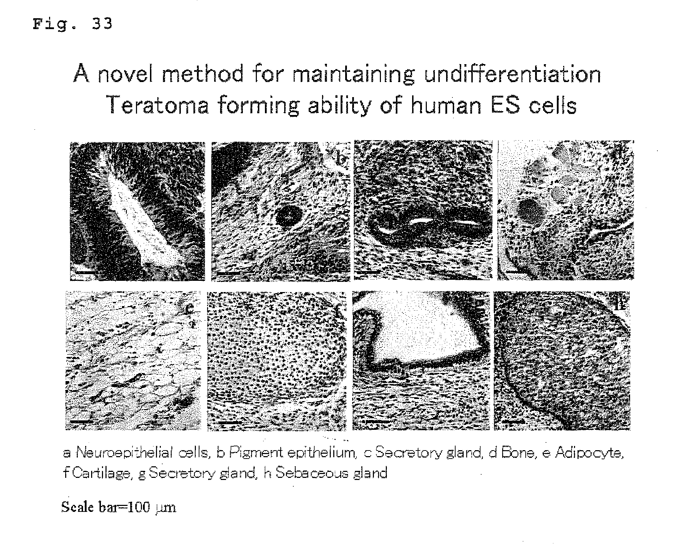

Fig. 33 shows tissue specimens of tumor (hematoxylin/eosin staining) shown in Example 11. As described, tridermic components of an ectoderm component (neuroepithelial cell; Fig. a, pigment epithelium; Fig. b, sebaceous gland: Fig. h), mesoderm component (bone; Fig. d, adipocyte Fig. e cartilage; Fig. f), and endoderm component (secretory gland; Fig. c and Fig. g) can be recognized.

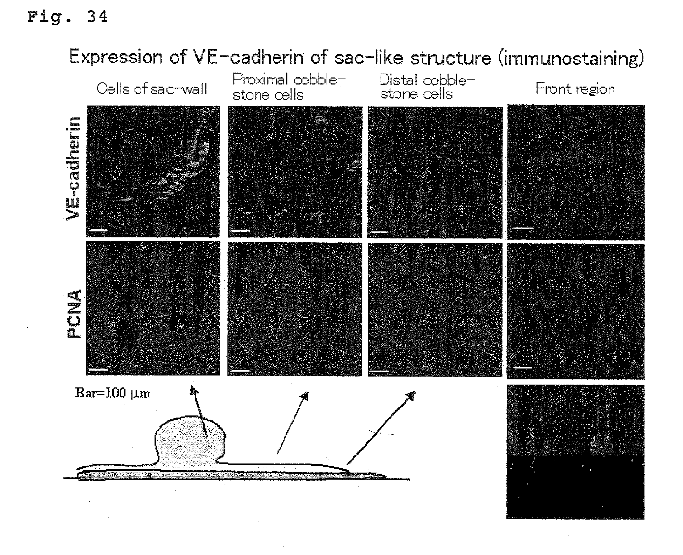

Fig. 34 shows immunostaining study on VE-cadherin expression regarding a sac-like structure which was fixed with an acetone/methanol mixture, shown in Example 12. Localization of VE-cadherin at the boundary between cells is clear in "sac wall cells" and "proximal cobblestone cells" spreading at the periphery of "sac wall cells". As the cobblestone cell becomes distal and the motility becomes increased, the cell membrane localization of VE-cadherin becomes unclear. In a front region having the highest motility, VE-cadherin is expressed mainly in a cell. Although all of these VE-cadherin-positive cells are PCNA positive, which is a proliferation marker, it can be understood that VE-cadherin-negative large-size cells are PCNA negative (arrow) and not proliferating. A scale bar indicates 100 µm.

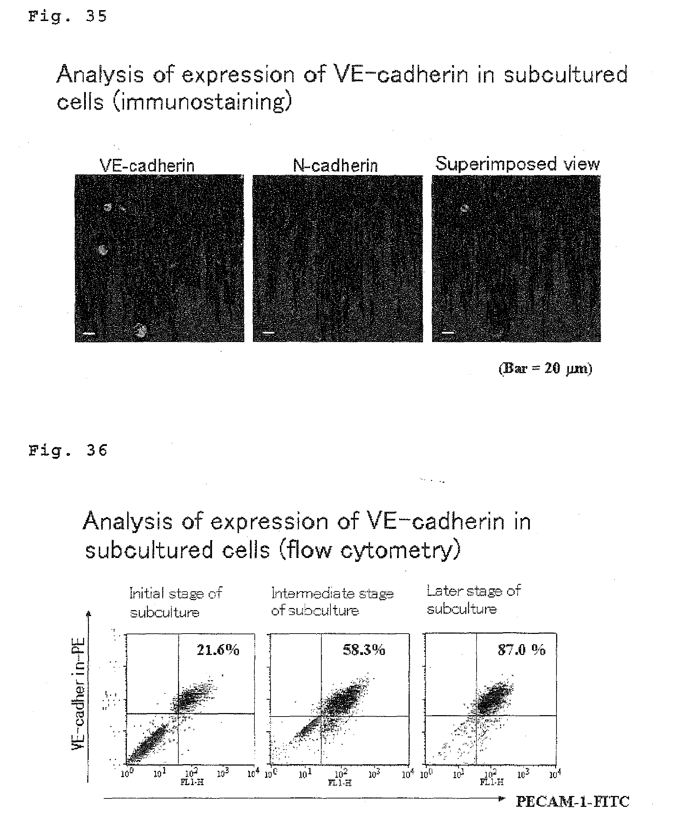

Fig. 35 shows immunostaining study as shown in Example 12, on expression of VE-cadherin regarding cells which were obtained by detaching and recovering the structure as an agglomerate shown in Fig. 34 and subculturing it. Herein, regarding all the cells, intracellular expression of VE-cadherin is at least confirmed. A scale bar indicates 20 µm.
Fig. 36 shows flow cytometry analysis of expressions of VE-cadherin and PECAM1 on the sac-like structure and the cobblestone cell which were subcultured for the indicated time, shown in Example 12. Although the VE-cadherin/PECAM1 positive rate is around 10 to 20% at initial subculturing, it can be understood that as passage number is increased, the positive rate is increased.

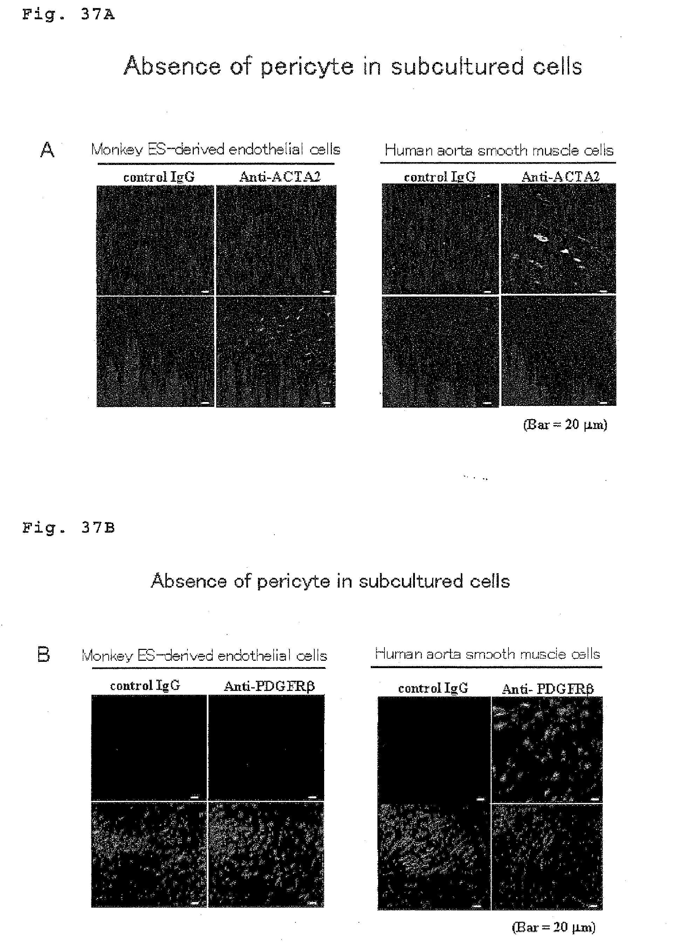

Fig. 37A shows immunostaining of monkey embryonic stem cell-derived endothelial cells and human aorta smooth muscle cells using an anti-smooth muscle actin (ACTA2) antibody or a control IgG, shown in Example 12 (Fig. 37A upper column) . Fig. 37A lower column shows differential interference images of cells. The monkey embryonic stem cell-derived endothelial cell population was ACTA2-negative, and thus it was confirmed that pericyte did not contaminate it. A scale bar indicates 20 µm.
Fig. 37B shows immunostaining of monkey embryonic stem cell-derived endothelial cells and human aorta smooth muscle cells using an anti-platelet-derived growth factor receptor β (PDGFR β) antibody or a control IgG, shown in Example 12 (Fig. 37B upper column). Fig. 37B lower column shows differential interference images of cells. A monkey embryonic stem cell-derived endothelial cell population was PDGRFβ-negative and thus it was confirmed that a pericyte did not contaminate it. A scale bar indicates 20 µm.

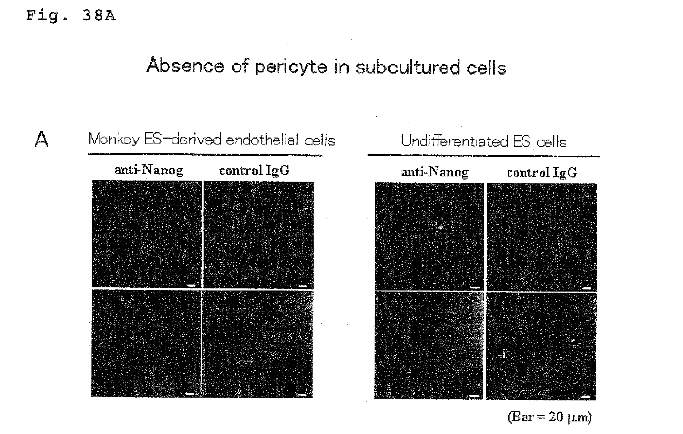

Fig. 38A shows immunostaining of monkey embryonic stem cell-derived endothelial cells and undifferentiated embryonic stem cells using anti-human Nanog antibody (Reprocell Incorporation) or a control IgG, shown in Example 12 (Fig. 38A upper column). Fig. 38A lower column shows differential interference images of cells. A monkey embryonic stem cell-derived endothelial cell population was Nanog-negative, and thus it was confirmed that the undifferentiated embryonic stem cell did not contaminate it. A scale bar indicates 20 µm.

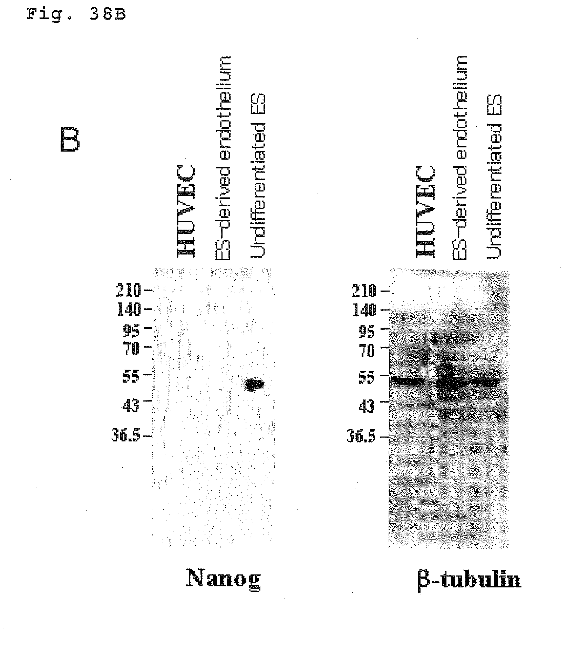

Fig. 38B shows results of Western blotting of a lysate of human umbilical vein endothelial cells (HUVEC), monkey embryonic stem-derived endothelial cells and undifferentiated embryonic stem cells, using an anti-human Nanog antibody (left) and an anti-β-tubulin antibody (right) shown in Example 12. A monkey embryonic stem-derived endothelial cell population was Nanog-negative, and thus it was confirmed that the undifferentiated embryonic stem cell did not contaminate it.

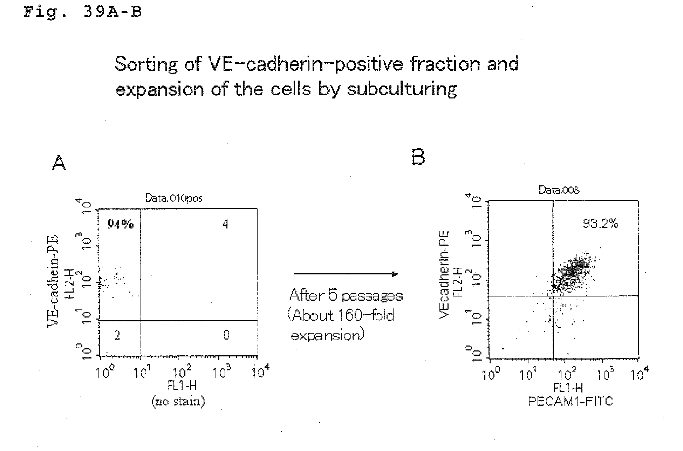

Figs. 39A-B show results of sorting a sac-like structure which was subcultured once, into a VE-cadherin-positive fraction and negative-fraction using an anti-VE-cadherin antibody (Beckman Coulter, Clone TE A 1.31) by means of FACSAria (BD Bioscience), shown in Example 12. Even if the VE-cadherin-positive fraction (Fig. 39A) was subcultured, expression of VE-cadherin was stably maintained (Fig. 39B), and about 160-fold expansion of the cells was achievd by five times passages.

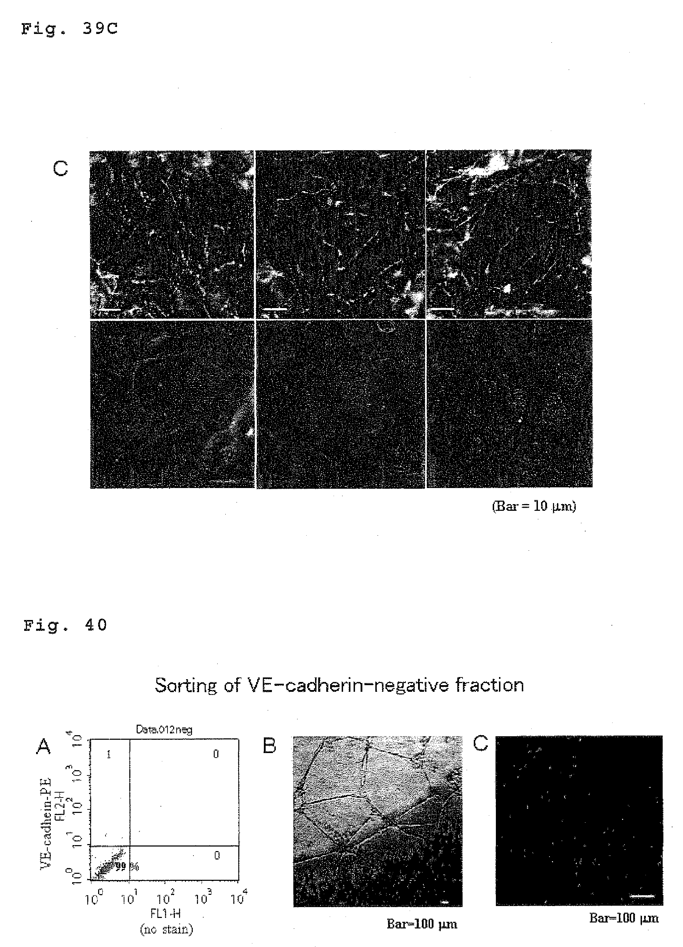

Fig. 39C shows immunostaining study on expressions of VE-cadherin using an anti-VE-cadherin antibody (BD, clone 75) after subculturing a VE-cadherin-positive fraction, shown in Example 12. Localization of VE-cadherin was confirmed at adhesion sites between all the cells. A scale bar indicates 10 µm.
Fig. 40 shows results of sorting a sac-like structure which was subcultured once, into a VE-cadherin-positive fraction and a negative-fraction by means of FACSAria (BD Bioscience), shown in Example 12. The VE-cadherin-negative fraction did not express VE-cadherin on a cell surface (Fig. A), but VE-cadherin is expressed within the cells, and the cells have the cord formation ability (Fig. B) and the acetylated low-density lipoprotein (Ac-LDL) uptake ability (Dil-Ac-LDL; fluorescence labeled Ac-LDL; LDL is non-labeled LDL as a negative control) (Fig. C). It was confirmed that the cell was committed to being a vascular endothelial cell. A scale bar indicates 100 µm.

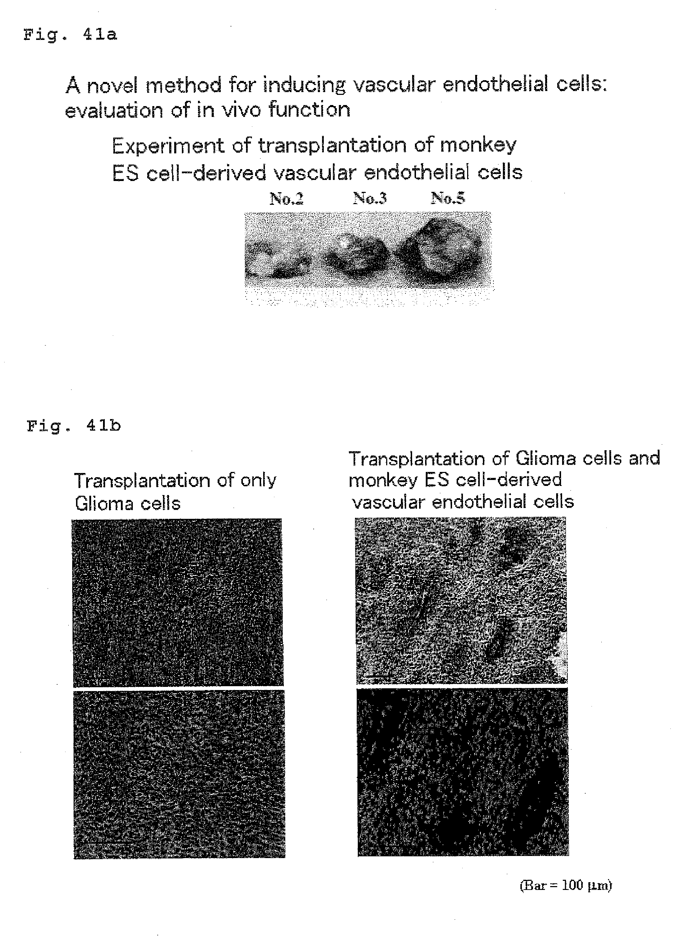

Fig. 41a shows tumor tissue specimens of rat glioma which was transplanted into a mouse, shown in Example 13. Regarding a mouse cotransplanted with cynomolgus monkey embryonic stem cell-derived vascular endothelial cells, it can be understood that a tumor is large, and a vessel is rich, and the mouse is easily-bleeding.
Fig. 41b shows histological study using hematoxylin/eosin staining (HE staining) of thin section of the tumor tissue of Fig.41a which was fixed with formalin. Regarding the mouse cotransplanted with cynomolgus monkey embryonic stem cell-derived vascular endothelial cells, a vessel rich structure is seen. A scale bar of 41b indicates 100 µm.

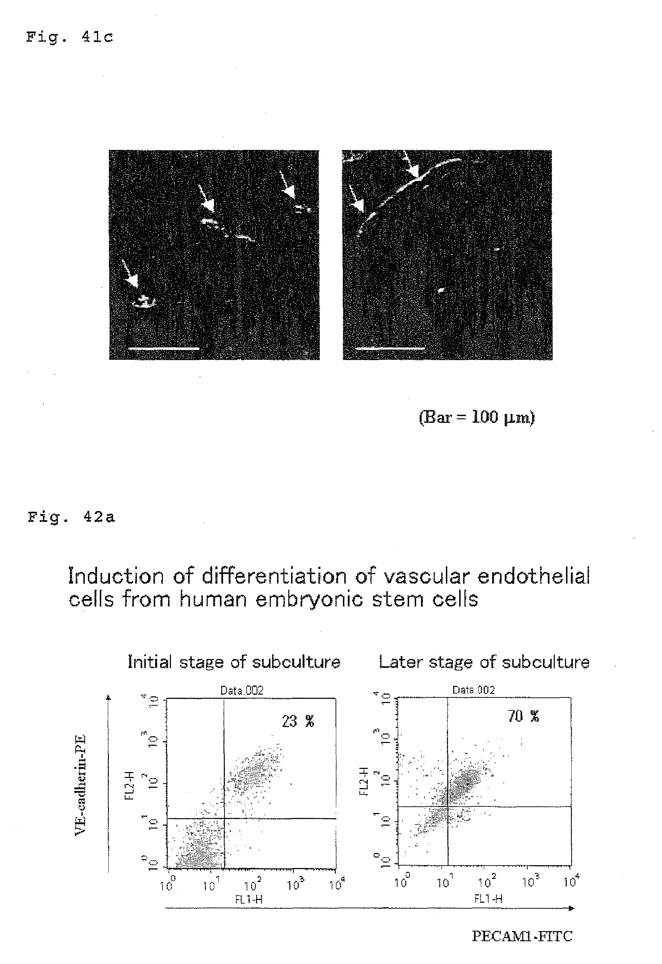

Fig. 41c shows results of immunostaining of tumor cells formed by cotransplantation of rat glioma cells and cynomolgus monkey embryonic stem cell-derived vascular endothelial cells, using anti-human HLA-A, B, C antibodies. The endothelial cells supporting neovascular vessels in the tumor are derived from a primate, that is, derived from cynomolgus monkey embryonic stem cell-derived vascular endothelial cells.
Fig. 42a shows flow cytometer analysis of expressions of VE-cadherin and PECAM1 on the cell surface of vascular endothelial cells which were induced to differentiate from human embryonic stem cells, shown in Example 14. It can be understood that at the initial stage of the subculture, the ratio of a VE-cadherin/PECAM1 double positive cell is around 20%, while the ratio reaches 70% at the later stage of the subculture.

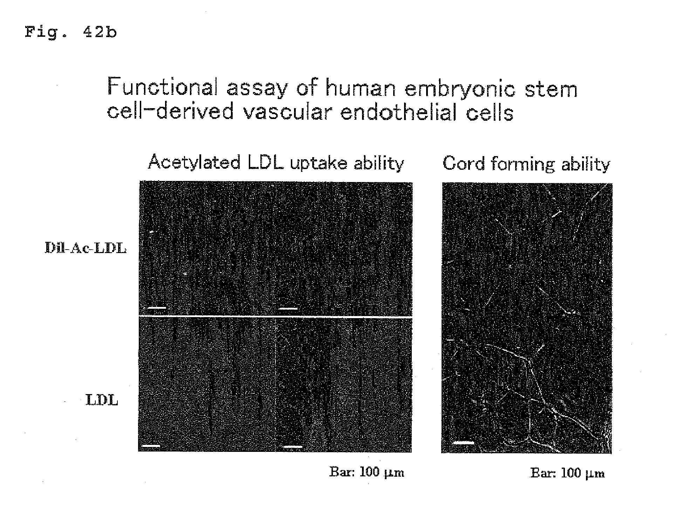

Fig. 42b shows an evaluation of in vitro function of human embryonic stem cell-derived vascular endothelial cells shown in Example 14. It can be understood that both the acetylated low-density lipoprotein uptake ability and the cord formation ability are positive. A scale bar indicates 100 µm.

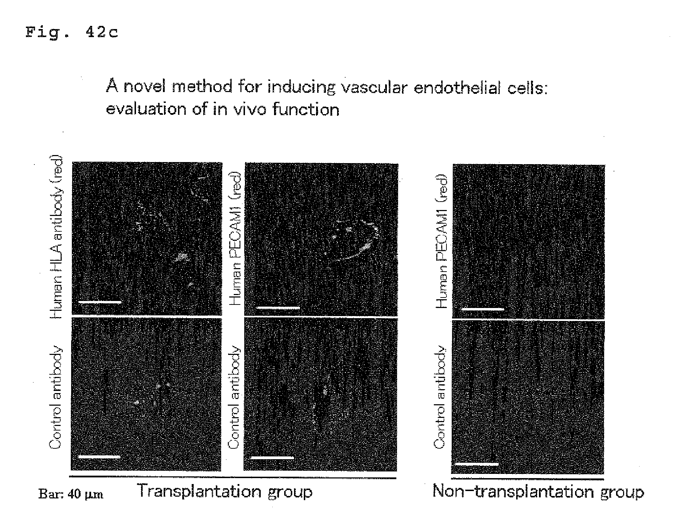

Fig. 42c shows results of plug assay for the purpose of evaluating in vivo function of human embryonic stem cell-derived vascular endothelial cells, shown in Example 14. A collagen plug into which human embryonic stem cell-derived vascular endothelial cells had been transplanted was recovered, followed by fixation with formalin, and immunostaining was conducted using human HLA-A, B, C antibodies and a human PECAM1 antibody. It can be understood that neovascular vessel formed in the plug is derived from a primate, that is, derived from human embryonic stem cell-derived vascular endothelial cells. A scale bar indicates 40 µm.

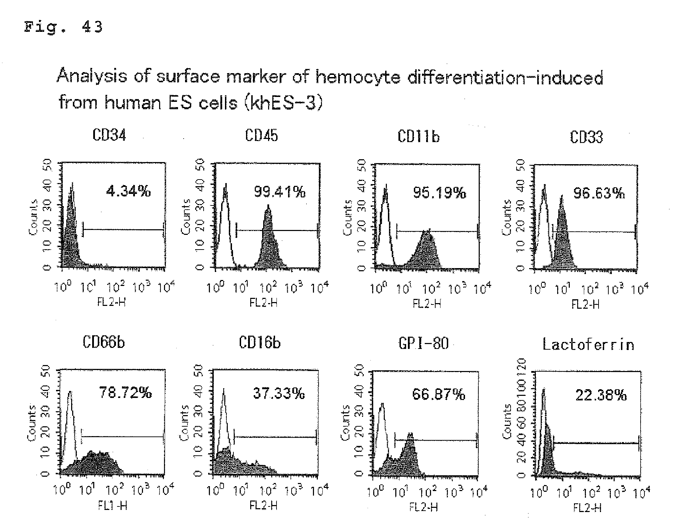

Fig. 43 shows flow cytometry analysis of expression of a hemocyte marker mainly relating to a neutophil, shown in Example 15.

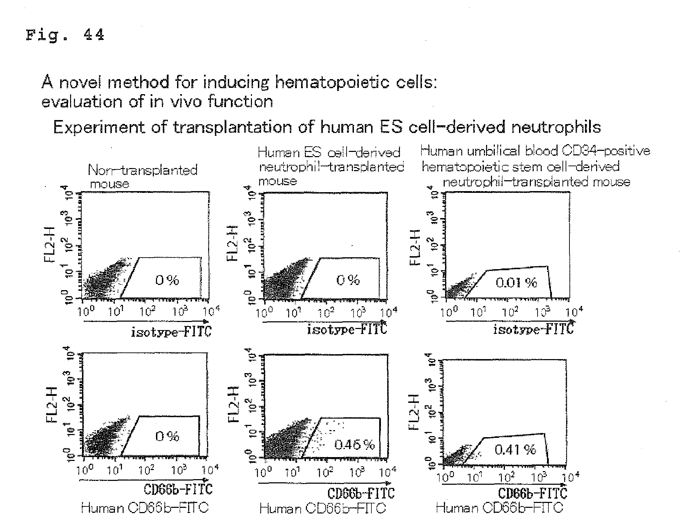

Fig. 44 shows flow cytometry measurement of the positive rate of a human-derived neutrophil using an anti-human CD66b antibody, shown in Example 16.

### [Explanation of symbols]

- 0101: Embryonic stem cell
- 0102: Hematopoietic stem cell
- 0103: Dendritic cell
- 0104: T lymphocyte precursor cell
- 0105: T cell
- 0106: B lymphocyte precursor cell
- 0107: B cell
- 0108: Plasma cell
- 0109: NK precursor cell
- 0110: NK cell
- 0111: Dendritic cell precursor cell
- 0112: Dendritic cell
- 0113: Mast cell precursor cell
- 0114: Mast cell
- 0115: basophil precursor cell
- 0116: Basocyte
- 0117: Eosinophil line precursor cell
- 0118: Eosinophil
- 0119: Glanurocyte macrophage precursor cell
- 0120: Macrophage precursor cell
- 0121: Monocyte
- 0122: Macrophage
- 0123: Osteoclast precursor cell
- 0124: Osteoclast
- 0125: Neutrophil precursor cell
- 0126: Neutrophil
- 0127: Megakaryocyte precursor cell
- 0128: Megakaryocyte
- 0129: Platelet
- 0130: Early erythroblast precursor cell
- 0131: Late erythroblast precursor cell
- 0132: Erythrocyte
- 0133: lymphoid stem cell
- 0134: Myeloid stem cell
- 0135: Dendritic cell precursor cell

### Best Mode for Carrying Out the Invention

The present invention was basically achieved by the present inventors as a result of trying to reduce stress on primate embryonic stem cells when they are cultured and subcultured and finding a suitable condition for allowing the inherently regulated differentiation of primate embryonic stem cells.
Specifically, the present inventors, as described later, found out the conditions for reducing and relieving a stress from primate embryonic stem cells, differentiated precursor cells and differentiated mature cells and the like, and thereby achieved continuous stable maintenance of embryonic stem cells and, at the same time, completed a method for safely and efficiently preparing desired cells from embryonic stem cells.

### I. Method for culturing and subculturing primate embryonic stem cells

In a first aspect, the present invention provides a method for culturing and subculturing primate embryonic stem cells, wherein the cells are maintained in the undifferentiated state, comprises the following steps:
(A) a step of culturing primate embryonic stem cells in a medium containing a protein component without using feeder cells and cytokines in a container coated with an extracellular matrix,
(B) a step of detaching colonies of the embryonic stem cells formed in the step (A) in the presence of a cytodetachment agent, and
(C) a step of plating the colonies of the embryonic stem cells obtained in the step (B) in a medium containing a protein component without using feeder cells and cytokines in a container coated with an extracellular matrix.
   The method of the present invention is based on the inventor's experimental finding that a primate embryonic stem cell has the ability to be maintained in the undifferentiated state without supplementing any particular exogenous factors (e.g. factor secreted by feeder cells, synthetic cytokine). Based on such finding, the inventors selected a suitable medium and employed an adequate technique for culturing and subculturing primate embryonic stem cells, and finally, they were able to maintain primate embryonic stem cells in the undifferentiated state without using any feeder cells and cytokines, and stably keep continuous subculture of primate embryonic stem cells for long periods (e.g. more than several ten times-passages) without inducing chromosome aberration.

Throughout the present specification, drawings and claims, "primate embryonic stem cell" related to the present invention means an embryonic stem cell derived from any primate. The primate embryonic stem cell and a method for the preparation thereof are known as exemplified by cynomolgus monkey embryonic stem cell [Suemori, H. et al., "Establishment of embryonic stem cell lines from cynomolgus monkey blastcysts produced by IVF or ICSI.", Dev. Dynamics, Vol. 222, pp.273-279 (2001)], a rhesus monkey embryonic stem cell [Tomson, J.A. et al., "Isolation of a primate embryonic stem cell line.", Proc. Natl. Acad. Sci, USA, Vol. 92, pp.7844-7848 (1995)], a marmoset embryonic stem cell [Tomson, J.A. et al., "Pluripotent cell lines derived from common marmoset blastcysts.", Biolol. Reprod., Vol. 55, pp.254-259 (1996)], a human embryonic stem cell [Tomson, J. A. et al., "Embryonic stem cell lines derived from human blastcysts", Science, Vol. 282, pp.1145-1147 (1998); Reubinoff. B. E. et al., "Embryonic stem cell lines from a human blastcysts: somatic differentiation in vitro.", Nat. Biotech., pp.399-404 (2000)].
In addition, if not otherwise specified, the term "primate embryonic stem cell" in the context of the present invention means an undifferentiated primate embryonic stem cell.

To confirm that a primate embryonic stem cell is undifferentiated, any known assay can be conducted. For example, a method known to a person skilled in the art including confirmation of expression of a molecular marker (e.g. determination of expression of SSEA-4, Oct-4 using flow cytometry, immunostaining of Oct-4, Nanog etc.), confirmation of pluripotent differentiation by in vitro experiment, and confirmation of teratoma formation by transplantation into an immunodeficient mouse etc., may be employed.

For culturing and subculturing primate embryonic stem cells, a medium which is usually used for maintaining primate embryonic stem cells (including no cytokines) can be used. Specifically, examples of the medium include an Iscove's-modified Dulbecco's medium (IMDM/Ham's F-12). Embryonic stem cells are plated in a medium according to the method as described later. It is not necessary to use a same medium throughout a series of subculture, and any different media may be employed as far as the embryonic stem cell can be maintained in the undifferentiated state. For example, media used in the step (A) and the step (C) may be the same or different.

The protein component used in the "a method for subculturing primate embryonic stem cells with keeping the undifferentiation state without using feeder cells and cytokines" as described above may be "other than animal serum" used for maintaining the primate embryonic stem cells, including serum albumin, human AB-blood-type serum. Alternatively, a commercially available serum-free additive, which is suitable for maintaining and proliferating embryonic stem cells, such as KNOCKOUT^{®} SR (manufactured by Invitrogen) and the like may be also used.

Examples of the "extracellular matrix" as described above include the extracellular matrix component secreted by a cell (Matrigel^{®} matrix (manufactured by BD) etc.), other component which is extracellularly secreted and enhances cell adhesion, living body(including a human body)-derived collagen, laminin, fibronectin, vitronectin, hyaluronic acid and an artificial synthetic substance of these proteins or polysaccharides (including degradation product and fragment), living body(including a human body)-derived serum, plasma and products separated or purified therefrom. In addition, culture container may be coated with the extracellular matrix by the conventional method. Preferably, the extracellular matrix may be derived from a primate including a human. In addition, when the extracellular matrix is obtained from both of a heterogeneous animal and a human and the latter is not less effective than the former, it is preferable to use the latter.

Primate embryonic stem cells may be separated from the culture container coated with an extracellular matrix preferably by a method which induces as little stress as possible, and a method described later is suitable. For example, when a culture dish coated with a Matrigel^{®} matrix is used, dispase is preferablely used. The method includes, but not limited to, a method that was confirmed to induce no stress or a sufficiently low stress on the cells.
In addition, it is preferable to omit operation or treatment including pipetting, which has the risk of evoking a cell damage as much as possible.

During an operation of culturing primate embryonic stem cells, determination of a stress received by a cell is simply conducted by measuring the number of dead cells or a proliferation rate of a cell. However, it is necessary to conduct a chromosome test (determination by a G band staining method etc.) periodically (every half a year etc.) and confirm that chromosomal aberration is not induced.

The "culture container" as described above may be a container which is normally used for culturing cell.
A cell density, when the primate embryonic stem cells are plated on a culture container, is selected so that a stress on a cell is minimized. Control of the cell density is achieved by suitably selecting the size and the number of a colony of primate embryonic stem cells.
The size of a colony, when the primate embryonic stem cells are plated on a culture container, is confirmed simply and highly effectively by observation using a microscope (inverted phase microscope etc.), and may be confirmed by other method (visual observation, measurement of side scattered light amount, measurement of solution turbidity etc.). An optimal size of a colony is dependent on individual case of primate embryonic stem cells. For example, in the case of a cynomolgus monkey embryonic stem cell or a human embryonic stem cell, the diameter is about 100 µm to about 2,000 µm, preferably about 300 µm to about 1,000 µm, more preferably around 500 µm.

Control of the size of the colony, when the primate embryonic stem cells are plated on the culture container, is achieved by an adequate cytodetachment operation.
It is preferable that a cell is detached under such condition that a stress on a cell is reduced as much as possible. As the cytodetachment agent, at least one selected from the group consisting of trypsin, collagenase, and dispase is preferable, and dispase alone or a combination of dispase and other cytodetachment agent is preferable. Alternatively, a commercially available cytodetachment agent (cytodetachment solution for primate embryonic stem cell (Reprocell Corporation)) and the like can be also used.
A suitable cytodetachment method (time, temperature, kind of cytodetachment agent etc.) may be determined for every individual primate embryonic stem cells. For example, a method shown in Example 1 described later is recommended. For example, in the case of feeder free culture of cynomolgus monkey embryonic stem cells, dispase which degrades only extracellular matrices is preferably used rather than collagenase solution (which may degrade cell membrane protein) for detaching cells when they are subcultured, because the cell stress can be considerably suppressed. However, case is not limited to this example.

The number of a colony of primate embryonic stem cells which are plated on a culture container is simply confirmed by observation using a microscope (inverted phase microscope etc.). A cell number measuring apparatus (hemocytometer etc.) may be used.
The optimal number (density) of a colony of the primate embryonic stem cell which are plated on the culture container is determined for every individual primate embryonic stem cells, and the density is necessary to be lower than a density which never induces fusion of colonies during the culture. For example, in the case of a cynomolgus monkey embryonic stem cell or a human embryonic stem cell, a method shown in Examples described later (particularly, Examples 1 to 3) is recommended.

A subculturing frequency (timing) of the primate embryonic stem cell is determined for every individual primate embryonic stem cells. As a rough guide, the timing is a time when a diameter of the colony reaches about 2-fold larger compared with the colony at the plating. For example, in the case of a cynomolgus monkey embryonic stem cell or a human embryonic stem cell, a method shown in Examples described later (particularly, Examples 1 to 3) is recommended. That is, when a size of the colony reaches about 200 µm to about 4,000 µm, preferably about 600 µm to about 2,000 µm, more preferably about 1,000 µm, cells are detached and plated again. When cytodetachment operation is conducted according to the method shown in Examples 1 to 3, colonies, each of which has an averege diameter of about 500 µm, are uniformly dispersed.

A primate embryonic stem cell is maintained in an undifferentiated state, when the medium is exchanged using a size of the colony as a guide of a timing for the exchange, as described above. The specific frequency is different depending on an origin of the embryonic stem cell, the culture condition and the like, and it is adequately adjusted. For example, in the case of a cynomolgus monkey embryonic stem cell or a human embryonic stem cell, the good undifferentiated state is maintained by performing medium exchange 4 times or more, preferably 5 times or more, more preferably 6 times or more a week (7 days).

The culture condition of primate embryonic stem cells may be a condition suitable for culturing the embryonic stem cells, including a condition of 37°C and 5 vol% CO₂. Optionally, the oxygen concentration may be adequately changed.

The primate embryonic stem cell which is undifferentiation-maintained according to "I. Method for culturing and subculturing primate embryonic stem cells" of the present invention can be freeze-thawed by the conventional method. For example, in the case of a cynomolgus monkey embryonic stem cell or a human embryonic stem cell, a method shown in Example 1 described later is recommended.

A cell obtained by culturing and subculturing primate embryonic stem cells by the method of the invention is normal after it is freeze-thawed, and can be also further subcultured. In addition, as described in Examples described later, chromosome aberration is not detected at all after at least 27 passages (as described later in Example 2). This shows that the present method is the considerably excellent culture technique as compared with the prior art (chromosome aberration is evoked after ten and a few times more passages; see Non-Patent Document 1).
In addition, according to the present method, since an additive such as a synthetic cytokine and the like is unnecessary, a culture system is simplified, and analysis at a molecular level becomes easy, and further great benefits are given to development of basic research. In addition, a cost of culture can be considerably reduced.
In addition, since handling of the culture according to the present method is extremely simple and does not need special equipment or special skill, the method can be immediately conducted in any facility around the world. That is, the method for culturing primate embryonic stem cells with keeping the cells undifferentiated of the present invention is generally applicable to the three fields of "clinical medicine", "medical industry" and "basic medical biology research", and may greatly contribute to development of them.

The present invention related to the method for culturing and subculturing primate embryonic stem cells with keeping the cells in the undifferentiated state includes a medium for maintaining undifferentiation, a method for coating a culturing equipment, a method for detaching cells at subculturing, a method for controlling a cell density at culturing, a method for controlling a frequency (timing) of subculture, and a method for freeze-thaw, regarding a primate embryonic stem cell, and also includes provision of the culture technique (publication, guidance), provision of information for preparing a medium or the like, and provision of a medium or the like.

### II. Method for inducing differentiation of a primate embryonic stem cell without using feeder cells

In other aspect, the present invention relates to a method for preparing blood cells, myeloid lineage cells, vascular endothelial precursor cells, stroma cells, hematopoietic stem cells or the like from a primate embryonic stem cell without using feeder cell.
(1) A method for preparing blood cells and/or vascular endothelial precursor cells without using feeder cells of the present invention comprises the following steps:
   (A) a step of suspension-culturing primate embryonic stem cells in a medium containing serum or a serum-free medium containing a serum substitute in the presence of a cytokine to prepare an embryoid body or an embryoid body-analogous cellular aggregate,
   (B) a step of adhesion-culturing the embryoid body or the embryoid body-analogous cellular aggregate obtained in the step (A) in the presence of a cytokine to prepare specific precursor cells containing non-adherent cells and adherent cells, and
   (C) a step of separating the non-adherent cells and the adherent cells from the specific precursor cells obtained in the step (B).
(2) In addition, a method for preparing blood cells, hematopoietic stroma cells and/or hematopoietic stem cells without using feeder cells of the present invention comprises the following steps:
   (A) a step of suspension-culturing primate embryonic stem cells in a medium containing serum or a serum-free medium containing a serum substitute in the presence of a cytokine to prepare an embryoid body or an embryoid body-analogous cellular aggregate,
   (B) a step of adhesion-culturing the embryoid body or the embryoid body-analogous cellular aggregate obtained in the step (A) in the presence of a cytokine to prepare specific precursor cells containing non-adherent cells and an adherent cells, and
   (C) a step of culturing the specific precursor cells obtained in the step (B) while the non-adherent cells are separated.

The "suspension culture" relating to the method of the present invention is to culture cells with keeping them in suspension using low absorbable culture container etc. (low-attachment plates etc.). In addition, the "adhesion culture" relating to the method of the present invention is to culture cells with keeping the adhesiveness of the cells to a culture container using a normal container designed for cell culture.

The methods (1) and (2) of the present invention are fundamentally based on the present inventor's finding as described below: "when an embryoid body or a cell aggregate similar thereto is formed from an embryonic stem cell and further adhesion culture is conducted using a suitable technique in a suitable differentiation medium containing a cytokine without using feeder cells, control of differentiation into a desired lineage of cells is achieved". Based on the finding, it becomes possible to prepare and expanded reproduce blood cells, vascular endothelial precursor cells, stroma cells, hematopoietic stem cells, myeloid lineage cells or the like from a primate embryonic stem cell, although it was previously impossible or difficult to do so.

It is known that the embryonic stem cell is differentiated into a vascular endothelial cell and a blood cell via an undifferentiated mesoderm and a common precursor cell of a vascular endothelial cell/ a blood cell (e.g. Jun Yamashita, Inflammation/Regeneration, Vol. 22, pp.509, 2002).
The blood cell in the context of the present method refers to a whole blood cell. Specifically, examples of the blood cell include, as shown in Fig.11 indicating differentiation cladogram from the embryonic stem cell to the blood cell, hematopoietic stem cell 0102, lymphoid stem cell 0133, lymphoid dendritic cell precursor cell 0135, lymphoid dendritic cell 0103, T lymphocyte precursor cell 0104, T cell 0105, B lymphocyte precursor cell 0106, B cell 0107, plasma cell 0108, NK precursor cell 0109, NK cell 0110, myeloid stem cell 0134, myeloid dendritic cell 0111, myeloid dendritic cell 0112, mast cell precursor cell 0113, mast cell 0114, basophil precursor cell 0115, basophil 0116, eosinophil precursor cell 0117, eosinophil 0118, granulocyte macrophage precursor cell 0119, macrophage precursor cell 0120, monocyte 0121, macrophage 0122, osteoclast precursor cell 0123, osteoclast 0124, neutrophil precursor cell 00125, neutrophil 0126, megakaryocyte precursor cell 0127, megakaryocyte 0128, platelet 0129, early erythroblast precursor cell 0130, late erythroblast precursor cell 0131, erythrocyte 0132.
In addition, the "blood cell" as described above includes a precursor cell of a hematopoietic stem cell; all morphologies of the blood cells which are present in all differentiation processes from a hematopoietic stem cell to final peripheral blood.
In addition, example of the "myeloid lineage cell" includes myeloblast, promyelocyte, myelocyte, metamyelocyte, neutrophil, monocyte, and macrophage.

A primate embryonic stem cell used as a material and a method for culturing said cell and a differentiated precursor cell or a mature cell used in the "II. Method for inducing differentiation of a primate embryonic stem cell without using feeder cells" related to the present invention is, in principle, the same as that described in the "I. Method for culturing and subculturing primate embryonic stem cells" for the purpose of culturing and maintaining the primate embryonic stem cells in an undifferentiated state. That is, conditions relating to a culture container, a culture medium, an additive such as a protein component and the like, an extracellular matrix, detaching of a cell from a medium, a method for subculture, a timing of subculture and the like are pursuant to the aforementioned conditions.
An origin of a primate embryonic stem cell which is a starting material is not particularly limited. A primate embryonic stem cell which is subcultured and maintained according to the method described in the "I" can be used. If necessary, a primate embryonic stem cell which has been frozen for preservation can be also used.

The culturing technique used in the "II. Method for inducing differentiation of a primate embryonic stem cell without using feeder cells" comprises a step of forming an embryoid body or an embryoid body-analogous cellular aggregate by suspension-culturing of primate embryonic stem cells, and a step regarding adhesion-culturing of an embryoid body or an embryonic body-analogous cellular aggregate. In this context, the "embryoid body-analogous cellular aggregate" means a cell aggregate which is present in the process of forming an embryoid body from an embryonic stem cell.
Examples of a method for forming an embryoid body or an embroyid body-analogous cellular aggregate includes a conventional hanging/dropping method, a conventional culture using a nonadhesive culture dish, and a conventional culture using a semi-solid medium. The method is not limited to them as far as an embryoid body or an embroyid body-analogous cellular aggregate is formed.

Although a term of suspension-culture for preparing an embryoid body or an embroyid body-analogous cellular aggregate is different depending on a cell, the culture condition and an objective product, it is usually about 2 days to 2 weeks. As the term becomes shorter, the ratio of the cell aggregate relative to an embryoid body becomes increased. In the case where an embryoid body or an embroyid body-analogous cellular aggregate is used as a starting material of the method for inducing differentiation into a blood cell and/or a vascular endothelial cell as described in (1), it is preferable to culture a cell until a cell aggregate is sufficiently formed. Specifically, regarding a cynomolgus monkey embryonic stem cell or a human embryonic stem cell, a method shown in example 4 or 5 is recommended. On the other hand, in the case where an embryoid body or an embroyid body-analogous cellular aggregate is used as a starting material of the method for inducing differentiation into a blood cell, a hematopoietic stroma cell, a bone marrow-associated cell, and/or a hematopoietic stem cell as described in the (2), a cell aggregate may be sufficiently formed for the purpose of inducing differentiation into only a mature blood cell (see Examples 8 and 9), while it is preferable to culture the cell until a sufficient embryonic body is formed for the purpose of inducing differentiation into a hematopoietic stroma cell, a bone marrow-associated cell, and/or a hematopoietic stem cell (see Examples 6 and 7).

In the "method for inducing differentiation of a primate embryonic stem cell without using feeder cells" as described above, an embryoid body or an analogous cell aggregate is adhesion-cultured with keeping the form in an optimized differentiation medium. The present method is epoch-making in that differentiation can be induced simply, safely and at the high efficiency unlike the previous method in which "precursor cell population" that is differentiating into a desired cell is isolated and purified after an embryoid body or an analogous cell aggregate is dissociated into a single cell by treatment of an enzyme.
When an embryoid body cell is adhesion-cultured without dissociating the cells using an "optimized differentiation medium" according to the present method, control of differentiation into a desired lineage is achieved at the extremely high efficiency (about 100% efficiency in both of vascular endothelial cell and hemocyte). This demonstrates that maintenance of "controlled differentiation" as is seen in a developmental process of an animal individual has been achieved.

The "differentiation medium" means a medium in which at least one kind cytokine is added to the medium for culturing primate embryonic stem cells with keeping undifferentiated state. The medium optionally contains other suitable additive as far as maintenance and differentiation of a cell are not adversely influenced.

The "cytokine" as described above, can be adequately selected depending on the purpose, and such cytokine is known to a person skilled in the art. The cytokine usable for the present invention is not particularly limited, as far as it is a factor for differentiating an embryonic stem cell into a blood cell and/or a vascular endothelial cell, and examples of the "cytokine" include stem cell factor (SCF), granulocyte colony stimulating factor (G-CSF, granulocyte macrophage colony stimulating factor (GM-CSF), colony stimulating factor (M-CSF), erythropoietin (EPO), thrombopoietin (TPO), Flt3 ligand (FL), interleukin (IL) (e.g. interleukin-3, interleukin-6, interleukin-15, interleukin-11 etc.), vascular endothelial growth factor (VEGF), bone morphogenetic protein (BMP; e.g. BMP-4 etc), oncostatin M, acidic and basic fibroblast growth factor (acidic FGF, basic FGF), angiopoietin family (e.g. Angiopoietin-1 and Angiopoietin-2) and the like. The G-CSF as described above, has the function of strengthening production of neutrophil. In addition, EPO (erythropoietin) induces production of erythrocyte having the oxygen transporting activity. In addition, TPO (thrombopoietin) induces amplification of an embryonic stem cell, and production of magakaryocyte and platelet having the hemostasis activity (activity of coagulating blood to stop breeding). In addition, interleukin 15 induces a natural killer cell (NK cell) which attacks a cancer cell.

A fundamental culture component used in the "II. Method for inducing differentiation of a primate embryonic stem cell without using feeder cells" of the present invention may be a medium suitable for inducing differentiation of a primate embryonic stem cell into a vascular endothelial cell/a hemocyte as described above, and specifically, examples of the medium include an Iscove's-modified Dulbecco's medium (IMDM).

A protein component, which may be added to the fundamental culturing component used in the "II. Method for inducing differentiation of a primate embryonic stem cell without using feeder cells", may be a protein component suitable for inducing differentiation of a primate embryonic stem cell into a vascular endothelial cell/a hemocyte and, specifically, examples of the protein component include bovine fetal serum, human serum (it is preferable to use AB-blood type serum having a low risk of inducing immunological rejection) and KNOCKOUT^{®} SR (manufactured by Invitrogen).

A coating component of a culture dish used in the "II. Method for inducing differentiation of a primate embryonic stem cell without using feeder cells" may be a component suitable for inducing differentiation of a primate embryonic stem cell into a vascular endothelial cell/a hemocyte and, specifically, examples of the coating component include gelatin.

The culture container as described above may be a container which is normally used for culturing cells.
The culture condition of a primate embryonic stem cell may be a condition suitable for culturing embryonic stem cells, examples of the condition include a condition of 37°C and 5 vol% CO₂, and the oxygen concentration may be adequately changed.
The culture condition used in the "method for inducing differentiation of primate embryonic stem cells without using feeder cells" of the present invention can be adequately determined depending on a kind of a primate embryonic stem cell used, and examples of the condition include a condition of 37°C and 5 vol% CO₂.

First of all, adhesion-culture is conducted until a "specific precursor cell" is formed. Medium change and cytodetachment procedure are conducted according to the description of the "I. Method for inducing differentiation of a primate embryonic stem cell without using feeder cells".
The "specific precursor cell" means a precursor cell differentiated from an embryoid body or an embroyid body-analogous cellular aggregate, comprising a non-adherent cell (cell having a spherical or near spherical shape and a nature of floating in a culture solution) and an adherent cell (cell adhering to a culture container). This may sometimes form a sac-like structure comprising an adherent cell and a spherical cell population consisting of a non-adherent cell, wherein the sac-like structure contains spherical cell population in the inside thereof, but the sac-like structure is not always formed. As described in Examples later, in the case of culturing an embryoid body or an embroyid body-analogous cellular aggregate in the presence or the absence of serum under the suitable condition, a specific precursor cell containing or not containing the sac-like structure can be formed. The non-adherent cell (spherical cell), when the sac-like structure is formed, is present not only in the structure but also in a culture solution to which the cell is released. On the other hand, when the sac-like structure is not formed, it is suspended in a culture solution. Therefore, in the present specification, the term "spherical cell" or "non-adherent cell" means a cell having both forms in abroad sense.
A blood cell (hematopoietic precursor cell committed to myeloid lineage cell, and mature hemocyte) can be mainly induced from a non-adherent cell (spherical cell) of a specific precursor cell, while a vascular endothelial precursor cell and a hematopoietic stroma cell can be mainly induced from an adherent cell of a specific precursor cell. In addition, a hematopoietic stem cell is induced from both the non-adherent cell and the adherent cell.

It is preferable to select a "precursor cell population" which is being differentiated to a specific lineage based on observation of a histological form of a cell using a phase microscope. "Correctly controlled and effective induction of differentiation" can be achieved not by a conventional method for selecting a cell using a "cell separating apparatus" including a cell sorter and an antibody specific to a molecular marker but by a method based on a histological morphology. That is, in the case of the conventional method based on a molecular marker, there is a problem that specificity is not always high and a cell is damaged by use of a cell separating apparatus. On the other hand, in the case of the method for selecting a precursor cell population of the present invention based on the histological morphology, adhesive culture of an embryoid body is maintained on a new culture dish without breaking the embryoid body, and the selection is determined based on observation of the various histological morphologies under a phase contrast microscope. As the result of the selection, it was clear that the "sac-like structure" and a spherical cell containing therein (non-adherent cell) as shown in Fig. 12, Fig. 19 and Fig. 26 correspond to a precursor of a vascular endothelial cell and a blood cell, respectively.
Identification of a precursor cell population based on observation under a microscope has the advantage of high feasibility and being immediately implementable at many facilities due to unnecessity of using a special equipment and an expensive reagent, in addition to advantages as follows: (1) the situation of cell differentiation can be checked at real time without invading a cell, and (2) by micropipette operation under a microscope, a desired precursor cell population can be picked out (manipulation) without giving a damage to the population.

In the method for preparing blood cells and/or vascular endothelial precursor cells without using feeder cells as described in (1), related to the method for inducing differentiation of a primate embryonic stem cell without using feeder cells of the present invention, non-adherent cells and adherent cells are separated respectively, from specific precursor cells containing the non-adherent cells and the adherent cells, and blood cells and vascular endothelial precursor cells are prepared from each of them.
A non-adherent cell in a culture solution and a spherical cell in a sac-like structure are separated by centrifugation etc. Separation of a spherical cell from the sac-like structure is conducted by providing an opening in the sac-like structure by means of a suitable method and releasing the contained spherical cells to be suspended. It is preferable that this operation is conducted before complete filling of the sac-like structure with cells. Usually, the opening of the sac-like structure is closed again while it is cultured, and the inside thereof becomes filled with a spherical cell.
When the sac-like structure is not formed, the non-adherent cell and the adherent cell are adequately separated.

In addition, the method for preparing blood cells, myeloid lineage cells, hematopoietic stroma cells and/or hematopoietic stem cells without using feeder cells, as described in (2), consists of adequately separating non-adherent cells released into a culture solution to obtain blood cells, and continuously culturing specific precursor cells of a sac-like structure containing non-adherent cells and adherent cells without modification.
Separation of the non-adherent cells is conducted by the centrifugation etc. On the other hand, blood cells and stroma cells can be obtained by continuously culturing a mixture of an adherent cell population and a non-adherent cell population containing the sac-like structure without separating spherical cells from the sac-like structure but with adequately releasing the non-adherent cells. If subculture is further continued, hematopoietic stem cells are obtained. Formation of the prepared stroma cells or hematopoietic stem cells can be confirmed by detecting each cell marker. Such marker is generally known to a person skilled in the art.

A hemocyte can be expanded reproduced by adhesion-culturing a precursor cell population separated and purified from cells comprised in embryoid body as described above. That is, the present method includes a culture method for expanded reproducing hemocytes. In said method, hemocyte precursor cells (spherical cells etc. which are present in sac-like structure) prepared by adhesion culture of embryoid body cells is further subcultured using adhesive culture, and resulting cell population, which is mixture of an adherent cell (hemocyte precursor cell and a hematopoietic stroma cell) and a non-adherent cell (hemocyte), is subcultured with keeping the mixture state to be expanded.
The present invention firstly provides a method for preparing a "hematopoietic stroma cell" from an embryonic stem cell. In addition, it became clear that the hematopoietic stem cell prepared by the present invention has not only a nature of a non-adherent cell and but also a nature of an adherent cell (see Fig. 23).

The vascular endothelial cell, the blood cell, the myeloid lineage cell, the hematopoietic stroma cell, the hematopoietic stem cell and the like of the present invention are maintained, for example, in a medium such as a solution designed for freeze preservation of cells such as Banbanker (manufactured by Nippon Genetics Co., Ltd.) under the frozen state using nitrogen gas. In addition, regarding at least the blood cell and the hematopoietic stroma cell, they have the advantage as follows: even after freeze-thawed, the blood cell and the hematopoietic stroma cell have an excellent reproductive ability and can be continuously obtained for a long time like before freezed.

The vascular endothelial cell, the blood cell, the myeloid lineage cell, the hematopoietic stroma cell, the hematopoietic stem cell and the like prepared by the present method exhibit an excellent nature as follows: they are substantially neither contaminated with a heterogeneous animal cell nor infected with a heterogeneous animal-derived virus. In addition, the vascular endothelial cell, the blood cell, and the hematopoietic stroma cell according to the present invention all exhibit high purity and a uniform nature. Therefore, the vascular endothelial cell according to the present invention can be used as a material for treating vessel damage or improving a topical blood stream, a material for transplantation, for manufacturing these materials and as a material used in a basic research concerning the development/differentiation mechanism of a blood endothelial cell. In addition, the blood cell according to the present invention can be used for a blood for transfusion, for preparation of a blood for transfusion and as a material in a basic research concerning a hematopoiesis mechanism. Further, the hematopoietic stroma cell according to the present invention can be utilized as a material for medical transplantation regarding a hematopoietic disorder, and as a material for basic research concerning a hematopoiesis mechanism. In addition, it is thought that the myeloid lineage cell is useful for the treatment of a bone-marrow damage.

According to the method for preparing vascular endothelial cells without feeder cells of the present invention, a cell population having more maturation tendency can be separated from a vascular endothelial precursor cell as a double positive population of VE-cadherin-positive and PECAM1-positive on the cell membrane surface, using a cell sorter or a bead precipitation method. Specifically, for example, the vascular endothelial cell can be separated by means of cell sorting by flow cytometry using a specific antibody to a marker such as VE-cadherin, PECAM1 and the like, cell sorting using magnetic beads having the antibody, or the like.

Alternatively, a steric vessel structure can be also obtained, for example, by culturing the vascular endothelial cells of the present invention in a collagen gel. Alternatively, the constructed vessel structure is transplanted into an animal, and thereby, a new vessel network can be also formed in a living body.

Using a hemocyte (including hematopoietic stem cell to mature hemocytes of a variety of lineages) prepared by the method for preparing hemocytes without using feeder cells of the present invention, only a specific lineage hemocyte such as a hematopoietic stem cell, a neutrophil, a monocyte, and a lymphocyte can be separated and concentrated by cell sorting with flow cytometry using an antibody to a lineage-specific marker or using magnetic beads having the antibody. Specifically, for example, a hematopoietic stem cell is separated and concentrated by recovering a CD34 and CD45 double positive cell fraction using a CD34-antibody and a CD45-antibody.

Examples of the means of separating and concentrating a specific lineage cell without giving a cell damage, among the aforementioned hemocytes (including from hematopoietic stem cell to mature hemocytes of a variety of lineages) include a density gradient centrifugation method and a separation method using counter-streaming centrifugation method (using elutriator (manufactured by Hitachi Ltd.)). By these methods, only a specific lineage hemocyte such as neutrophil, monocyte and lymphocyte can be separated. For example, as shown in Fig. 28, by a density gradient centrifugation method using Lymphoprep^{®} (manufactured by Daiichi Pure Chemicals Co., Ltd.), it is possible to effectively separate and concentrate neutrophil prepared from a human embryonic stem cell.

In the method for preparing a blood cell of the present invention, depending on the kind of an objective blood cell or the like, the hematopoietic stem cell/ precursor cell may be further differentiated under the suitable condition, or a cytokine in a medium for feeder free differentiation may be adequately changed. In the method for preparing a blood cell of the present invention, examples of preparation of the blood cell using various cytokines include differentiation into a granulocyte using G-CSF and GM-CSF, differentiation into a monocyte/macrophage using GM-CSF and M-CSF, differentiation into an NK cell using IL-15, differentiation into erythrocyte using EPO, differentiation into a megakaryocyte/a platelet using TPO, differentiation into a dendritic cell using IL-4 and GM-CSF, and the like.

In addition, the method for subculturing primate embryonic stem cells with maintaining the undifferentiation state without using feeder cells and cytokines and the method for inducing differentiation into a vascular endothelial cell, a blood cell or the like without using feeder cells of the present invention is not limited to use for an embryonic stem cell. However, by making attempts including some modification of the culture condition, the methods can be applied to the technique of differentiating into various cells (cell group having pluripotency such as a testis stem cell and an adult stem cell).

As described above, according to the present invention, a vascular endothelial cell which can be subcultured and maintained, a hemocyte which can be expanded reproduced (including from hematopoietic stem cell to mature hematocyte), a hematopoietic stroma cell and the like can be prepared from a primate embryonic stem cell with very high efficiency (approximately near 100%) by a simple culture method using only an inexpensive culture equipment. The prepared cells are not substantially accompanied with being contaminated with a heterogeneous animal cell and being infected with a heterogeneous animal-derived virus, or the like.
Therefore, since the "method for inducing differentiation into a vascular endothelial cell and a blood cell without using feeder cells" according to the present invention has a very high differentiation efficiency, is not accompanied with a cell damage, and has high feasibility, the method is rapidly implemented worldwide, and its profit is remarkably high.

The present invention will be explained in detail based on Examples below, but the present invention is not limited by these Examples.

### Example 1

### Undifferentiation-maintenance culture of cynomolgus monkey embryonic stem cells without using feeder cells and cytokines

### (1) Preparation of undifferentiation-maintenance culture solution

Cynomolgus monkey embryonic stem cells were cultured at 37°C and 5 vol% CO₂ in a CO₂ incubator on a 10 cm culture dish or a 78 cm² culture dish which was coated at room temperature for around 15 minutes to 30 minutes with Matrigel^{®} matrix [manufactured by BS (BD Biosciences)] diluted by 30-fold with an undifferentiation-maintenance culture solution 1-1 (composition: DMEM/Ham' S F-12 [manufactured by Kohjinbio Co., Ltd.], 20 vol% KNOCKOUT^{®} SR [manufactured by Invitrogen Corp.], 1 mM L-glutamine [manufactured by Invitrogen Corp.], 2 mM non-essential amino acid solution [manufactured by Invitrogen Corp.], 1 mM sodium pyruvate [manufactured by Invitrogen Corp.], final concentration 100 U/ml of penicillin [manufactured by Invitrogen Corp.], final concentration 100 µg/ml of streptomycin [manufactured by Invitrogen Corp.]).

### (2) Technique of undifferentiation-maintenance culture

Cynomolgus monkey embryonic stem cells are plated at the density of 1 to 2 cells within the microscope's field of view using a phase-contrast microscope equipped with 4×objective lens and 10×ocular lens in the condition that the colony is relatively uniformed in size and has the diameter of 500 µm. Since on the next day, the size of the colony becomes about 1,000 µm, the cells are detached using dispase [manufactured by BD (BD Biosciences)], and are subcultured in a new culture container coated with a Matrigel^{®}. The dispase treatment was conducted as follows: after the culture solution is removed, cynomolgus monkey embryonic stem cells are impregnated with a dispase solution, followed by immediately sucking the solution and performing the reaction at 37°C for 5 minutes, and then, DMEM/Ham's F-12 is added thereto, followed by collecting the cells to transfer them into a centrifugation tube with great care to avoid pipetting as much as possible and then, the supernatant is centrifuged (1,000 rpm, 5 min, 4°C) to precipitate the cells. By the above-described procedure, the colonies of the cynomolgus monkey embryonic stem cells are dispersed so that the size of the colony having the diameter of 500 µm is relatively uniformed. Repeating above-described subculture procedure every two days makes it possible to avoid fusion of the colonies and expand the culture which is properly maintained in the undifferentiated state in the above-described medium containing no feeder cells and synthetic cytokines.

### (3) Cell morphology

After at least 43 passages, the undifferentiated state was properly maintained. Specifically, cell morphology desired as the undifferentiated state (see Fig. 1), high expression of SSEA-4, Oct-4, Nanog, Tra-1-60, and Tra-1-81 which are an undifferentiation-maintenance marker (see Fig. 2 and Fig. 3) and tumor formation in an immunodeficient mouse (SCID mouse) (see Fig. 4 and Fig. 5) were confirmed.

Fig. 1 shows the colony of the cynomolgus monkey embryonic stem cell cultured by the aforementioned method. "A" indicates cells which were subcultured after freeze-thaw of 20th passage cells and "B" indicates cells which were subcultured after freeze-thaw of 35th passage cells.
Fig. 2 is the result of flow cytemetry measurement of expressions of SSEA-4 and Oct-4 which are an undifferentiation maintenance marker on the 20th passage cynomolgus monkey embryonic stem cells cultured by the aforementioned method. Very high expressions of them(> 95%) are confirmed.
Fig. 3 shows immunostaining analysis on expressions of Tra-1-60, Tra-1-81, and Nanog which are an undifferentiation maintenance marker on the 20th passage cynomolgus monkey embryonic stem cells cultured by the aforementioned method. On almost all cells, expressions of any marker are confirmed.
Fig. 4 shows a photograph of testis obtained from three immunodeficient mice (SCID mice) two months after transplantation of the 21st cynomolgus monkey embryonic stem cells cultured by the aforementioned method under a testis membrane of said mice. In all of three animals, formation of a teratoma was confirmed. Fig. 5 is a tissue specimen of the above-described tumor (hematoxylin/eosin staining). As indicated, a nerve epithelium, a tooth, secretion gland, an intestine tube-like epithelium, and a smooth muscle are recognized.
As shown in Example 4 to Example 7, the present invention makes it possible to prepare vascular endothelial cells and expanded reproduce hemocytes and hematopoietic stroma cells from the cynomolgus monkey embryonic stem cell which is undifferentiation-maintained.

### (4) Freeze preservation of cynomolgus monkey embryonic stem cells

The cynomolgus monkey embryonic stem cells subcultured by the aforementioned undifferentiation-maintenance culture method can be frozen in liquid nitrogen for preservation using a freeze preservation solution 1 (2 M DMSO, 1 M Acetamide, 3 M Propylene glycol/medium for human ES cell) or a commercially available freeze preservation solution for a primate embryonic stem cell (Reprocell Incorporation). First of all, a frozen stock of cells is prepared by the following procedure:
the precipitated cynomolgus monkey embryonic stem cells are recovered by the aforementioned method;
200 µl of a freeze preservation solution cooled on an ice is added;
the cells are gently suspended and transferred to a tube for freeze preservation as rapidly as possible (within 15 seconds);
the tube is soaked in liquid nitrogen; and
the tube is frozen in liquid nitrogen for 30 seconds to 1 minute to completely freeze up to the inside, and is transferred to a liquid nitrogen storage container.

### (5) Thaw of frozen cynomolgus monkey embryonic stem cells

Thaw of frozen and stored cells is performed by the following procedure:
1 ml of an undifferentiation-maintenance medium pre-warmed to 37°C is added to a frozen tube containing the cynomolgus monkey embryonic stem cells;
the cells are rapidly thawed by pipetting and 15 ml of a cell suspension is transferred to a 15 ml conical tube followed by recovering the cells by centrifugation (1,000 rpm, 5 minutes, 4°C); and
after the cells are suspended in an undifferentiation-maintenance medium, the state of the cell is confirmed with a microscope, and the cells are cultured at 37°C and 5 vol% CO₂ in a CO₂ incubator in a culture container coated with a Matrigel^{®} matrix.
By the aforementioned procedure, undifferentiation-maintained cynomolgus monkey embryonic stem cells are proliferating even after freeze-thaw with properly maintaining the undifferentiated state.

### Example 2

### Undifferentiation-maintenance culture of human embryonic stem cells without using feeder cells and cytokines (method using culture container coated with Matrigel^{®} matrix)

### (1) Preparation of undifferentiation-maintenance culture solution

Cynomolgus monkey embryonic stem cells were cultured at 37°C and 5 vol% CO₂ in a CO₂ incubator on a 10 cm culture dish or a 78 cm² culture dish coated at room temperature for around 15 minutes to 30 minutes, with Matrigel^{®} matrix [manufactured by BS (BD Biosciences)] diluted by 30-fold with an undifferentiation maintenance culturing solution 1-1 (composition: DMEM/Ham' S F-12 [manufactured by Kohjinbio Co., Ltd.], 20 vol% KNOCKOUT^{®} SR [manufactured by Invitrogen Corp.], 1 mM L-glutamine [manufactured by Invitrogen Corp.], 2 mM non-essential amino acid solution [manufactured by Invitrogen Corp.], 0.1 µM 2-mercaptoethanol [manufactured by Sigma Chemical Co.], final concentration 100 U/ml of penicillin [manufactured by Invitrogen Corp.], final concentration 100 µg/ml of streptomycin [manufactured by Invitrogen Cop.]).

### (2) Technique of undifferentiation-maintenance culture

The human embryonic stem cells are plated at the density of about 2 to 3 within the microscope's field of view using a phase-contrast microscope equipped with 4×objective lens and 10×ocular lens in the condition that the colony is relatively uniformed in size to have the diameter of 500 µm and, thereafter, medium exchange is performed every day. Since the size of the colony becomes about 1,000 µm after 3 to 4 days, the cells are detached using a cytodetachment solution 1 (composition: 0.25% trypsin solution [manufactured by Invitrogen Corp.], 1 mg/ml collagenase IV [manufactured by Invitrogen Corp.], 1% KNOCKOUT^{®} SR [manufactured by invitrogen Corp.], and 1 mM calcium chloride [manufactured by Sigma Chemical Co.] the solution is prepared based on phosphate buffer) or a cytodetachment solution for a primate embryonic stem cell (Reprocell Incorporation), and the resulting cells are subcultured in a new culture container coated with a Matrigel^{®} matrix. A specific procedure of detachment is as follows:
after the culture solution is removed, human embryonic stem cells are impregnated with a cytodetachment solution to perform the reaction at 37°C for 5 minutes followed by sucking the cytodetachment solution;
DMEM/Ham'S F-12 is added to further perform the reaction at 37°C for 10 minutes;
thereafter, the cells are detached by tapping a culture container to allow the cells to be suspended; and
the cells are suspended two times using a 1,000 µl pipette tube, and recovered in a centrifugation tube followed by a centrifugation (1,000 rpm, 5 minutes, 4°C) to precipitate the cells.
By the aforementioned procedure, the colonies of the human embryonic stem cell are dispersed into the relatively uniform size of the diameter of 500 µm. By performing the aforementioned procedure two times every week, the human embryonic stem cells can be subcultured and properly maintained in the undifferentiated state in the culture solution to which neither a feeder nor a synthetic cytokine is added.

### (3) Cell morphology

Even after 25 times passages, the undifferentiation maintenance state was properly retained. Specifically, the cell morphology desired as the undifferentiated state, and high expression of SSEA-4, Oct-4, and Nanog which are an undifferentiation-maintenance marker was confirmed. In addition, in chromosome analysis of the cells after 27 passages, maintenance of a normal chromosome karyotype was confirmed (see Fig. 6, Fig. 7, Fig. 8 and Fig. 9).

Fig. 6 shows a phase-contrast micrograph of a colony of 24th passage human embryonic stem cells cultured by the aforementioned method.
Fig. 7 is the result of flow cytometry measurement of expressions of SSEA-4 and Oct-4 which are undifferentiation-maintenance markers on 20th passage human embryonic stem cells cultured by the aforementioned method. In both of them, very high expressions (> 95%) are confirmed.
Fig. 8 shows immunostaining analysis on expressions of Oct-4 (A) and Nanog (B) which are undifferentiation-maintenance markers on 25th passage human embryonic stem cells cultured by the aforementioned method, On almost all cells, expressions of both proteins are confirmed.
Fig. 9 is a chromosome analysis view of a human embryonic stem cell (G band method). The left figure shows a result obtained in maintenance of the cell according to a conventional method (i.e. co-culture using fetal mouse fibroblast as feeder cells) as recommended by the institution which established the cell, while right figure shows a result obtained after 20th passage according to "a method for culturing without using feeder cells and cytokines" of the present invention. It was confirmed that no chromosome aberration occurred.

### (4) Freeze preservation and thaw of human embryonic stem cells

The human embryonic stem cells subcultured by the aforementioned undifferentiation-maintenance culture method can be frozen in liquid nitrogen for the preservetion and thawed by the method described in Example 1, using a freeze preservation solution 1 or a commercially available freeze preservation solution for a primate embryonic stem cell (Reprocell Incorporation). By the above procedure, the human embryonic stem cells which were maintained in undifferentiated state, as shown in Example 2, are also proliferating with properly keeping the undifferentiated state even after the freeze-thaw.

### Example 3

### Undifferentiation-maintenance culture of human embryonic stem cells without using feeder cells and cytokines (method using a culture container coated with one kind of human-derived protein component)

### (1) Preparation of undifferentiation-maintenance culture solution

The medium as described in Example 2 was used.

### (2) Technique of undifferentiation-maintenance culture

The human embryonic stem cells are plated at the density of about 2 to 3 within the microscope's field of view using a phase-contrast microscope equipped with 4×objective lens and 10×ocular lens in the condition that the colony is relatively uniformed in size to have the diameter of 500 µm, and thereafter, medium exchange is performed every day. Since the size of the colony becomes about 1,000 µm after 3 to 4 days, the cells are detached using a cytodetachment solution 1 (composition: 0.25% trypsin solution [manufactured by Invitrogen Corp.], mg/ml collagenase IV [manufactured by Invitrogen Corp.], 1% KNOCKOUT^{®} SR [manufactured by Invitrogen Corp.], and 1 mM calcium chloride [manufactured by Sigma Chemical Co.] the solution are prepared based on phosphate buffer) or a cytodetachment solution for a primate embryonic stem cell (Reprocell Incorporation), and the cells are subcultured in a new culture container coated with 5 µg/cm² fibronectin obtained from human plasma (manufactured by BD), in a new culture container coated with human AB-blood type serum, in a new culture container coated with 5 µg/cm² laminin obtained from human placenta (manufactured by Sigma), in a new culture container coated with 0.2 µg/cm² vitronectin obtained from human plasma (manufactured by BD) or in a new culture container coated with 5 µg/cm² collagen type IV obtained from human placenta (manufactured by BD). A specific procedure of the detachment is as follows:
after the culture solution is removed, human embryonic stem cells are impregnated with a cytodetachment solution to perform the reaction at 37°C for 5 minutes followed by sucking the detachment solution;
DMEM/Ham'S F-12 is added to further perform the reaction at 37°C for 10 minutes;
thereafter, the cells are detached by tapping a culture container to allow them to be suspended;
the cells are suspended two times using a 1,000 µl pippet tube and recovered in a centrifugation tube; and
a centrifugation procedure (1,000 rpm, 5 minutes, 4°C) is performed to precipitate the cells.
By the aforementioned procedure, the colonies of the human embryonic stem cells are dispersed into the relatively uniform size of the diameter of 500 µm. By performing the above procedure two times every week, the human embryonic stem cells can be subcultured to properly maintain the undifferentiated state in the culture solution as described in Example 2, to which neither a feeder nor a synthetic cytokine is added.

Even after fourth passage, the undifferentiated state is properly retained. The cell morphology desired as the undifferentiated state, and high expression of SSEA-4, and Oct-4 which are undifferentiation-maintenance markers were confirmed (See Fig. 10).
Fig. 10 shows a phase-contrast micrograph of a fourth passage human embryonic stem cells cultured using a culture dish coated only with human-derived fibronectin (5 µg/cm²) (A) or a culture dish only coated with human type AB serum, as prepared by the aforementioned method. It can be understood that any of them retains the undifferentiated morphology. In addition, when expressions of SSEA-4 and Oct-4 which are undifferentiation-maintenance markers were measured on these cells by flow cytometry, high expressions of both markers were confirmed in any of them (B, D).

### Example 4

### Induction of differentiation of a Cynomolgus monkey embryonic stem cell into a vascular endothelial cell/a blood cell without using feeder cells (method using bovine fetal serum)

### (1) Preparation of differentiation medium

For a cynomolgus monkey embryonic stem cell, cytokines consisting of a final concentration 20 ng/ml of vascular endothelial growth factor (VEGF), a final concentration 20 ng/ml of bone morphorgenetic protein-4 (BMP-4), 20 ng of stem cell factor (SCF), a final concentration 10 ng/ml of Flt3-ligand, a final concentration 20 ng/ml of interleukin 3 (IL 3) and a final concentration 10 ng/ml of interleukin 6 (IL 6), were added to a differentiation medium 1-1 {composition: Iscove's-modified Dulbecco's medium (IMDM) [manufactured by Sigma Chemical Co.], 15% by weight of heat-inactivated bovine fetal serum [PPA Laboratories GmbH], 1 mM β-mercaptoethanol [manufactured by Sigma Chemical Co.], and 2 mM L-glutamine [manufactured by Invitrogen Corp.]}.

### (2) Technique of inducing differentiation

Embroyid body-analogous cellular aggregate cells are prepared by the Hanging/Dropping method using the differentiation medium 1-1 (with addition of cytokine). Specifically, cynomolgus monkey embryonic stem cells were recovered using a cytodetachment solution followed by treatment with a 0.25% trypsin solution [manufactured by Invitrogen Corp.] at 37°C for 5 minutes to allow the cells to be dispersed and individually separated from each other. 3,000 cynomolgus monkey embryonic stem cells are suspended in 30 µl of the differentiation medium 1-1 (with addition of cytokine), and a drop of the suspension was placed on a back side of a lid of a culture dish having the diameter of 10 cm using a micropipette (it is possible to place about 20 to 30 drops on one culture dish). The cells were suspension-cultured at 37°C and 5 vol% CO₂ for 3 days in a CO₂ incubator which was filled with water to prevent the culture from being dried up. Since formation of a cellular aggregate can be visually confirmed after three days, the aggregate was recovered by rinsing a lid surface of the culture dish, and adhesion-cultured at 37°C and 5 vol% CO₂ in a CO₂ incubator using the differentiation medium 1-1 (with addition of cytokine) on a culture dish (diameter 10 cm or 6 cm) coated with 0.1% gelatin [manufactured by Sigma Chemical Co.]. Thereafter, the medium was exchanged every 3 to 4 days. The aggregate of cynomolgus monkey embryonic stem cells continued to grow with spreading transversally and, after about 2 weeks, a specific precursor cell (organization consisting of sac-like structure and spherical cell population) was formed from the area around a center of the place where the aggregate was originally placed (one was formed per aggregate) (see Fig. 12). Fig. 12 indicates specific precursor cells (organization consisting of sac-like structure and spherical cell population) which are common precursors of vascular endothelial precursor cells and blood cells, and were prepared from the cynomolgus monkey embryonic stem cells in the presence of bovine fetal serum, by means of the technique of inducing differentiation and expanded reproduction of vascular endothelial cells/ hemocytes without using feeder cells.

Before the spherical cells were completely filled into the sac-like structure, by making a slight notch around a bottom of the sac-like structure without destroying a structure of the sac-like structure itself using a microknife (Stem cell knife, manufactured by SweMed), the spherical cells in the inside of the sac-like structure were slowly released into a culture solution. If the spherical cells are completely filled into the sac-like structure, viability of the spherical cell is decreased. These spherical cells were recovered by centrifuging the culture supernatant. On the other hand, the sac-like structure and cells expanding therefrom were detached and recovered by treatment with trypsin/EDTA solution [manufactured by Invitrogen Corp.] at 37°C for 5 minutes.

Hemocyte preparation was confirmed by conducting a hematopoietic colony assay of the recovered spherical cell using a colony assay kit (Methocult^{®} GF⁺H4535 (Stemcell Technologies Inc.)) equipped with a semi-solid medium containing methylcellulose (see Fig. 13). Fig. 13 shows Wright-Giemsa staining (A) and special staining (myeloperoxidase staining (B) and esterase double staining (C)) of mature hemocytes prepared from the spherical cells. A variety of myeloid lineage cells, that is, cells which are present in each differentiation stage ranging from a myeloblast to a mature hemocyte (neutrophil and macrophage) were observed.

The recovered sac-like structures and cells expanding at the periphery thereof were cultured using a differentiation medium 1-1 (with cytokine added thereto), in a new culture dish (diameter 10 cm or 6 cm) coated with 0.1% gelatin [manufactured by Sigma Chemical Corp.]. Thereafter, the cells were detached using a trypsin/EDTA solution every 3 to 4 days, and about one-third of the detached cells were used for the subculture. The subculture was conducted eight times (see Fig. 14 to Fig. 16).
Fig. 14 is the results of immunostaining study on expressions of VE-cadherin which is a vascular endothelial cell-specific marker, and N-cadherin which is one of vascular endothelial cell markers, on vascular endothelial cells prepared from the "specific precursor cell" as described above. As shown in Fig. 14, expression of VE-cadherin which is a vascular endothelial cell-specific marker was confirmed on almost all cells after 2 passages. Further, as shown in this figure, N-cadherin which is an adhesion factor known to be expressed regarding a vascular endothelial cell was expressed on almost all cells as measured by immunostaining, and it was shown that clear cell membrane localization was recognized. This is a nature that a primary vascular endothelial cell obtained from a living body has been lost as shown in Fig. 31, and is the very interesting finding (compare and see Fig. 14 and Fig. 31).
Fig. 31 is the results of immunostaining study on an intracellular expression manner of N-cadherin in there kinds of commercially available human primary cultured vascular endothelial cells (human umbilical vein endothelial cell (HUVEC), human microvessel endothelial cell (HMVEC) and human aorta endothelial cell (HAEC)). From Fig. 31, it can be understood that cell membrane localization of N-cadherin has been lost in the commercially available human primary cultured vascular endothelial cell. On the other hand, the vascular endothelial cell prepared by the present method shows the clear cell membrane localization, and correctly reflects a nature of a vascular endothelial cell in a living body (in Fig. 14).

Fig. 15 shows expression of PECAM1 which is a mature vascular endothelial cell marker on a vascular endothelial cells prepared from the "specific precursor cell", confirmed by flow cytometry analysis using double staining with VE-cadherin which is a pan-vascular endothelial cell marker and vascular endothelial-specific marker. An axis of abscissa represents an intensity of PECAM1 expression, and an axis of ordinate represents intensity of VE-cadherin expression. As shown in Fig. 15, expressions of both proteins of VE-cadherin which is a pan-vascular endothelial cell marker and vascular endothelial cell-specific marker and PECAM1 which is a mature vascular endothelial cell marker were confirmed in 40% or more of the cells.
Fig. 16 shows the cord formation ability (A) and the acetylated low-density lipoprotein uptake ability (B), which were investigated so as to confirm the mature function of vascular endothelial cells prepared from the "specific precursor cells". In any case, acquisition of the mature function is confirmed at high efficiency. In the light of the forgoing, expressions of VE-cadherin and PECAM1 were retained even after eight times passages and, as shown in Fig. 16, the functional maturity of vascular endothelial cells such as the cord formation ability and the acetylated low-density lipoprotein uptake ability were confirmed.

In the light of the forgoing, vascular endothelial cells which, at least for eight times subculture procedure,retain the stable proliferating ability and mature function and shows a clear N-cadherin cell membrane localization which can be similarly seen in a living body were prepared from the cynomolgus monkey embryonic stem cells. Example 5

### Induction of Differentiation of a cynomolgus monkey embryonic stem cell into a vascular endothelial cell/a blood cell without using feeder cells (method using serum-free medium)

### (1) Preparation of differentiation medium

Cynomolgus monkey embryonic stem cells were induced to be differentiated using a medium in which cytokines consisting of a final concentration 20 ng/ml of vascular endothelial growth factor (VEGF), a final concentration 20 ng/ml of bone morphogenetic protein-4 (BMP-4), 20 ng of stem cell factor (SCCF), a final concentration 10 ng/ml of Fld3-ligand, a final concentration 20 ng/ml of interleukin 3 (IL 3), and a final concentration 10 ng/ml interleukin 6 (IL 6) were added to a differentiation medium 1-2 {composition: Iscove's modified Dulbecco's medium (IMDM) [manufactured by Sigma Chemical Co.], 15% by weight of KNOCKOUT^{®} SR [manufactured by Invitrogen Corp.], 1 mM β-mercaptoethanol [manufactured by Sigma Chemical Co.], and 2 mM L-glutamine [manufactured by Invitrogen Corp.]}.

### (2) Technique of inducing differentiation

Embroyid body-analogous cellular aggregate cells are prepared by the Hanging/Dropping method using the differentiation medium 1-2 (with addition of cytokine). Specifically, cynomolgus monkey embryonic stem cells are recovered using a cytodetachment solution followed by treatment with a 0.25% trypsin solution [manufactured by Invitrogen Corp.] at 37°C for 5 minutes to allow the cells to be dispersed and individually separated from each other. 3,000 cynomolgus monkey embryonic stem cells are suspended in 30 µl of the differentiation medium 1-2 (with addition of cytokine), and a drop of the suspension is placed on a back side of a lid of a culture dish having the diameter of 10 cm using a micropipette (it is possible to place about 20 to 30 drops on one culture dish). The cells were suspension-cultured at 37°C and 5 vol% CO₂ for 3 days in a CO₂ incubator which was filled with water to prevent the culture from being dried up. Since formation of a cellular aggregate can be visually confirmed after three days, the aggregate was recovered by rinsing a lid surface of the culture dish, and adhesion-cultured at 37°C and 5 vol% CO₂ in a CO₂ incubator using the differentiation medium 1-2 (with addition of cytokine), on a culture dish (diameter 10 cm or 6 cm) coated with 0.1% gelatin [manufactured by Sigma Chemical Co.]. Thereafter, the medium was exchanged every 3 to 4 days. The aggregate of the cynomolgus monkey embryonic stem cells continued to grow with spreading transversally. Unlike an experiment in the presence of bovine fetal serum (Example 4), formation of a sac-like structure was not seen, but after about two weeks, proliferation of an adherent cell and production of a non-adherent cell (spherical cell) were confirmed. The non-adherent cell (spherical cell) was recovered by centrifuging the culture supernatant. On the other hand, the adherent cell was detached and recovered by treatment with a trypsin/EDTA solution [manufactured by Invitrogen Corp.] at 37°C for 5 minutes.

Hemocyte preparation was confirmed by conducting a hematopoietic colony assay of the recovered spherical cell using a colony assay kit (Methocult^{®} GF⁺H4535 (Stemcell Technologies Inc.)) equipped with a semi-solid medium containing methylcellulose(see Fig. 17). Fig. 17 shows Wright-Giemsa staining (A) and special staining (myeloperoxidase staining (B) and esterase double staining (C)) of mature hemocytes prepared from the cynomolgus monkey embryonic stem cells using the serum-free culture (using KNOCKOUT^{®} SR). Preparation of a variety of myeloid lineage cells, that is, cells which are in each of various differentiation stages ranging from a myeloblast to a mature hemocyte (neutrophil and macrophage) were observed.

Regarding the recovered adherent cells, adhesion-culture was conducted using a differentiation medium 1-2 (with addition of cytokine) in a new culture dish (diameter 10 cm or 6 cm) coated with 0.1% gelatin [manufactured by Sigma Chemical Co.]. Thereafter, the cells were detached using a trypsin/EDTA solution every 3 to 4 days, and about one-third of the detached cells were used for the subculture. The result of a flow cytometry study of 3rd passage cells is shown in Fig. 18. Previously, it was thought that iuduction of differentiation into an endothelial cell can not be achieved without using serum, but expressions of VE-cadherin and PECAM1 were confirmed on a cell membrane of a few % or more of the cells(see Fig. 18). This result is the epoch-making outcome, because so far, it has been believed that vascular endothelial cells cannot be prepared from primate embryonic stem cells using serum-free culture and in addition, it was reported that the production efficiency of vascular endothelial cells from primate embryonic stem cells is 2% or less even in the presence of serum.

### Example 6

### "Induction of differentiation into a blood cell (a hematopoietic stem cell and a mature hemocyte)", "production of hematopoietic stroma cells", and "expanded reproduction of hematopoietic cells and hematopoietic stroma cells" from Cynomolgus monkey embryonic stem cells (using bovine fetal serum)

### (1) Medium preparation

Cytokines of a final concentration 50 ng/ml of bone morphogenetic protein-4 (BMP-4), 300 ng/ml of stem cell factor (SCF), a final concentration 300 ng/ml of Flt3-ligand, a final concentration 10 ng/ml of interleukin 3 (IL 3), a final concentration 10 ng/ml of interleukin 6 (IL 6), and a concentration 50 ng/ml of granulocyte colony stimulating factor (G-CCF) were added to a differentiation medium 1-3 {composition: knockout D-MEM (Knockout D-MEM [Invitrogen Corp.], 20% by weight of heat-inactivated bovine fetal serum [PAA Laboratories GmbH], 0.1 mM β-mercaptoethanol [manufactured by Sigma Chemical Co.], 1% non-essential amino acid solution [manufactured by Invitrogen Corp.], and 1 mM L-glutamine [manufactured by Invitrogen Corp.]}.

### (2) Technique of inducing differentiation 1 (step of embryoid body formation)

Cynomolgus monkey embryonic stem cells which were maintained in the undifferentiated stage prepared by Example 1 were treated with collagenase IV (room temperature, 20 min) and subsequently, with chelating agent (non-enzymatic cell dissociation buffer [manufactured by Invitrogen Corp.]) at room temperature for 20 minutes so that the cells were detached from a culture container coated with a Matrigel^{®} matrix. By this procedure, the cynomolgus monkey embryonic stem cells were dissociated and almost separated from each other. The resulting cells were recovered and then, suspension-cultured overnight using a non-adhesive culture container (diameter 6 cm, culture dish etc.) or Hydrocell (manufactured by CellSeed) coated with polyhydroxyethyl methacrylate (poly(2-hydroxyethyl methacrylate), manufactured by Sigma Chemical Co.) using the differentiation medium 1-3. On the next day, the medium was exchanged with the differentiation medium 1-3 (comprising no cytokine) to which cytokine is added, and the suspension culture was further conducted for about 2 weeks to obtain an embryoid body (or embroyid body-analogous cellular aggregate) (see Fig. 19A). During the culture period of 2 weeks, medium exchange was conducted every 3 to 4 days. At the time of medium exchange, an embryoid body (or embroyid body-analogous cellular aggregate) suspended in the medium was recovered in the culture supernatant followed by centrifugation to precipitate cell components. Then, the resulting cells were suspended in a newly prepared differentiation medium 1-3 (with no addition of cytokine), and the resulting suspension was transferred to a newly prepared non-adhesive culture container, and suspension culture was continued.

### (3) Technique of inducing differentiation 2 (step of forming "specific precursor cells" (organization consisting of sac-like structure and spherical cell population))

The formed embryoid body as described above was recovered from the culture supernatant by centrifugation and then, adhesion-cultured at 37°C and 5 vol% CO₂ in a CO₂ incubator using the differentiation medium 1-3 (with addition of cytokine), on a culture dish (24-well multiwell dish) coated with 0.1% gelatin [manufactured by Sigma Chemical Co.]. Thereafter, the medium was exchanged every 3 to 4 days. An aggregate of the cynomolgus monkey embryonic stem cells continued to grow with spreading transversally and, after about two weeks, "specific precursor cells" (organization consisting of sac-like structure and a spherical cell population contained therein) was formed from the area around a center of a region where an aggregate was originally located, as shown in Fig. 19B (one was formed per each aggregate). Fig. 19 shows a phase-contrast micrograph of embryoid body cells prepared from the cynomolgus monkey embryonic stem cell in the presence of bovine fetal serum (A), and a "specific precursor cell" (organization consisting of sac-like structure and spherical cell population), which is a common precursor of a vascular endothelial precursor cell and a blood cell, obtained by adhesion-culturing the embryoid body (B).

### (4) Technique of inducing differentiation 3 (step of expanded reproduction of hemocytes)

By treating a culture dish containing the "specific precursor cells" formed by means of the technique 2 with a trypsin/EDTA solution [manufactured by Invitrogen Corp.], all cells present on the culture dish were detached and recovered, and the adhesion-culture was continued using a differentiation medium 1-3 (with addition of cytokine) on a new culture dish (diameter 6 cm) coated with gelatin. The adherent cells actively proliferated and, after two days, reached confluent. Further, after two days, as shown in Fig. 20, production of the non-adherent cells was obviously observed. Fig. 20 shows a phase-contrast micrograph showing a circumstance in which expanded reproduction of blood cells was achieved. Both a hematopoietic stroma cell (adherent cell) and a hemocyte (non-adherent cell) produced therefrom were confirmed.
Thereafter, the medium was exchanged two times per week to continue the cell culture. When medium was exchanged, the non-adherent cells were centrifuged to recovere them and they were returned to the culture dish. In addition, the adherent cells were detached using a trypsin/EDTA solution [manufactured by Invitrogen Corp.] once per week, and mixed with the non-adherent cells in the culturing supernatant. Then, 1/2 to 1/3 of the resulting mixture was subcultured.

Regarding the non-adherent cells recovered from the culture supernatant, the cells were assessed for a blood cell by confirming (1) the colony forming ability using a hematopoietic colony assay (using Methocult^{®} GF⁺H4535 (Stemcell Technologies Inc.)), (2) morphology observed by Wright-Giemsa staining, (3) the presence or the absence of the specific enzyme activity using special staining such as myeloperoxidase staining (POX staining), esterase double staining, neutrophilic alkaline phosphatase staining (NAP staining) and the like, and (4) expression on a cell surface of various markers (CD34, CD45, CD11b etc.) using flow cytometry. Results are shown in Fig. 21A to Fig. 21F.
Wright-Giemsa staining image (A), and special staining (myeloperoxidase staining (B), esterase double staining (C), neutrophilic alkaline phosphatase staining (D)) of the hemocytes (non-adherent cells) recovered in the above-described expanded reproduction process of a hemocyte are shown. A variety of myeloid lineage cells, that is, cells which are present in each of various differentiation stages ranging from a myeloblast to a mature hemocyte (neutrophil and macrophage) are observed. Further, the results confirmed by flow cytometry regarding expressions of CD34 which is a hematopoietic stem cell marker (E) and CD45 which is a pan-hematopoietic cell marker (F) on these hemocytes (non-adherent cell) are shown.
From Fig. 21, production of cells which were present in each of various differentiation stages of a myeloid lineage of cell (including myeloblast, promyelocyte, myelocyte, metamyelocyte, neutrophil, monocyte, and macrophage) was confirmed. In addition, it was confirmed that expression of a CD45 antigen which is a pan-hematopoietic cell marker was approximately 100% (see Fig. 21F), the hemocyte differentiation efficiency is very high, and an almost pure population consisting of an approximately only hemocyte was obtained. In addition, a CD34-positive (i.e. CD34 and CD45 double positive cell) was present at a few % (see Fig. 21E), and it was confirmed that hematopoietic stem cells (or hematopoietic precursor cells equivalent to hematopoietic stem cells) were prepared.

In the case where only adherent cells were subcultured, only non-adherent cells were subcultured, or both of them were mixed and subcultured, in each case, the same state consisting of an adherent cell and a non-adherent cell as seen before the subculture was reproduced even after the subculture. This situation was stably retained during a culture period until about 80th passage (about 3 months), and production of a various blood cells including from a hematopoietic stem cell to a mature hematocyte was continuously confirmed. In addition, in the case where only adherent cells were frozen, only non-adherent cells were frozen, or both of them were mixed and frozen, in each case, the same state as the state seen before freezing was reproduced even after the cells were thawed and cultured (see Fig. 24). Fig. 24 shows a phase microscope photograph of the situation of expanded production of blood cells, where each of non-adherent cells and adherent cells were freeze-thawed to restart the culture, respectively. In any case, the presence of a hematopoietic stroma cell (adherent cell) and a hemocyte (non-adherent cell) produced therefrom was confirmed to be the same as seen before freezing.

### (5) Technique of inducing differentiation 4 (step of expanded reproduction of hematopoietic stroma cells)

Fig. 22 shows a phase microscope photograph of subculture-capable "hematopoietic stroma cells" prepared by the aforementioned method (A), and the result of flow cytometry analysis on expressions of CD34, and CD45 (B). Both CD45, which is a pan-hematopoietic cell marker, and CD34, which is a hematopoietic cell marker, were almost negative, and it was confirmed that they are non-hematopoietic cells (it is thought that a small amount of a positive cells was caused by being contaminated with hematopoietic stem cells adhered to hematopoietic stroma cells).
Regarding the adherent cells formed by the above-described technique 3, a CD45 antigen which is a pan-hematopoietic cell marker was expressed little (see Fig. 22), and it was confirmed that the adherent cells were a non-hematopoietic cells. Although the cell form was relatively flat in any case, the cells were not homogeneous because they included star-shaped cells, elongated cells and, in some cases, cells having a short pseudopodium and a polynuclear large cells. From the viewpoint of morphology, the state was very similar to an inhomogeneous cell population called as "hematopoietic stroma" which is an adherent cell obtained by culturing a bone marrow blood. As far as these cells are present, hemocytes which are present in various differentiation stages of a bone marrow lineage are continuously produced as described in the technique 3, and in the case where this cell is disappeared, the produced hemocyte tends to be limited to a hematopoietic stem cell (or hematopoietic precursor cell equivalent to hematopoietic stem cell) as described later. Thus, from the functional aspect, the cells are also determined to be hematopoietic stroma cells. In addition, as described above, regarding these adherent cells, the form and the function were stably retained until about 80th passage (culturing for about 3 months), and expanded reproduction of them was possible.
From now on, analyzing these adherent cells from a variety of angles would hopefully contribute to better understanding of a hematopoietic stroma cell, the entity of which has not been known yet, and would produce a great effect in medical transplantation and basic research regarding the hematopoietic mechanism.

### (6) Technique of inducing differentiation 5 (step of expanded reproduction of hematopoietic stem cells)

Fig. 23 shows a phase-contrast micrograph of "CD34-positive and CD45-positive cells" prepared by long term culture (> 100 days) of a subculture-capable "hematopoietic stem/precursor cells" prepared by the above-described method (A). The non-adherent cells and the adherent cells are present as an admixture, but they may be interchangeable, and thus, it is thought that both are equivalent cell populations (e.g. hematopoietic stem cells and equivalent thereof). Fig. 23B shows the result of confirmation of expression of CD34 and CD45 by flow cytometry, regarding the non-adherent cells and the adherent cells. In addition, regarding a human, the hematopoietic stem cell is a "CD34-positive and CD45-positive cell", and the cell is separated and purified from bone marrow blood or umbilical blood and transplanted in the case of medical transplantation of the hematopoietic stem cell.
The adherent cells formed by means of the technique 4 were hematopoietic stroma cells in almost all cases until about 80^{th} passage (culturing for about 3 months), as described above. However, in a period of about 10 passages thereafter, the hematopoietic stroma cells were gradually decreased and, at about 100^{th} passage, these adherent cells were substituted with "uniform cell population which is thick and has a spindle shape" (see Fig. 23A). From flow cytometry analysis on a cell surface marker, the cell was CD34-positive and CD45-positive (see Fig. 23B, second panel of lower column), and was a hematopoietic stem cell (or hematopoietic precursor cell equivalent to hematopoietic stem cell). At the same time, at this stage, almost all of the non-adherent cells became CD34-positive and CD45-positive hematopoietic stem cells (or hematopoietic precursor cells equivalent to hematopoietic stem cells) (see Fig. 23B, second panel of upper column). That is, at this stage, both of the non-adherent cells and the adherent cells became to belong to the same cell linegae called as a hematopoietic stem cell (or a hematopoietic precursor cell equivalent to a hematopoietic stem cell), and it was clear that a hematopoietic stem cell (or a hematopoietic precursor cell equivalent to hematopoietic stem cell) has not only a nature of the non-adherent cell but also a nature of the adherent cell.

Thereafter, the above-described state is stably retained after subculturing exceeding 140 times. In addition, after freeze and thaw, the same state is reproduced. That is, culturing these cells makes it possible to stably expanded reproduce hematopoietic stem cells (or hematopoietic precursor cells equivalent to hematopoietic stem cells). Example 7

### "Induction of differentiation into blood cell" from cynomolgus monkey embryonic stem cells without using feeder cells (using serum-free medium)

### (1) Medium preparation

Cytokines consisting of a final concentration 50 ng/ml of bone morphogenetic protein-4 (BMP-4), 300 ng/ml of stem cell factor (SCF), a final concentration 300 ng/ml of Flt-3 ligand, a final concentration 10 ng/ml of interleukin 3 (IL 3), a final concentration 10 ng/ml of interleukin 6 (IL 6), and a concentration 50 ng/ml of granulocyte colony stimulating factor (G-CSF) were added to a differentiation medium 1-3 {composition: knockout D-MEM (Knockout D-MEM [manufactured by Invitrogen Corp.], 20% by weight of KNOCKOUT^{®} SR [manufactured by Invitrogen Corp.], 0.1 mM β-mercaptoethanol [manufactured by Sigma Chemical Co.], 1% non-essential amino acid solution [manufactured by Invitrogen Corp.], and 1 mM L-glutamine [manufactured by Invitrogen Corp.]}.

### (2) Technique of iducing differentiation 1 (step of embryoid body formation)

The cynomolgus monkey embryonic stem cells which were maintained in undifferentiated stage as prepared by Example 1 were suspension-cultured overnight in a non-adhering culture container (diameter 6 cm culture dish etc.) coated with polyhydroxyethyl methacrylate (poly(2-hydroxyethyl methacrylate), manufactured by Sigma Chemical Co.) or Hydrocell (manufactured by CellSeed), using the differentiation medium 1-4, after they were detached from a culture container coated with a Matrigel^{®} matrix according to the method described in Example 6. On the next day, the medium was exchanged with a differentiation medium 1-4 (with no addition of cytokine) to which the cytokine was added, and the cells were further suspension-cultured for about 2 weeks to prepare an embryiod body (or embroyid body-analogous cellular aggregate). During the culture period of 2 weeks, the medium was exchanged every 3 to 4 days. At the medium exchange, the culture supernatant comprising the embryoid body (or embroyid body-analogous cellular aggregate) suspending in the medium was recovered, followed by centrifuging to precipitate cell components. The resulting cells were suspended in a newly prepared differentiation medium 1-3 (with no addition of cytokine), and the suspension was placed into a newly prepared non-adhering culture container to continue the culture.

### (3) Technique of inducing differentiation 2 (step of "specific precursor cell" formation)

The embryoid body formed by means of the technique 1 was recovered from the culture supernatant by centrifugation, and adhesion-cultured at 37°C and 5 vol% CO₂ in a CO₂ incubator using a differentiation medium 1-4 (with addition of cytokine), on a culture dish (24-well multiwell dish) coated with 0.1% gelatin [manufactured by Sigma Chemical Co.]. Thereafter, the medium was exchanged every 3 to 4 days. An aggregate of the cynomolgus monkey embryonic stem cells continued to grow with spreading transversally and, after about 2 weeks, the same "specific precursor cells" (organization consisting of sac-like structure and spherical cell population contained therein) as that shown in Example 6 were formed from the area around a center of a region where the aggregate was originally located.

### (4) Technique of inducing differentiation 3 (step of expanded reproduction of hemocytes)

By treating a culture dish containing the "specific precursor cells" formed by means of the technique 2 with a trypsin/EDTA solution [manufactured by Invitrogen Corp], all of cells on the culture dish were detached and recovered and then, the culture was continued on a new culture dish (diameter 6 cm) coated with gelatin using the differentiation medium 1-4 (with addition of cytokine). The adherent cells gradually proliferated and, after a few days, reached confluent. When the culture was further continued, production of the non-adherent cells was clearly observed. Fig. 25 shows Wright-Giemsa staining image (A) and special staining (myeloperoxidase staining (B) and esterase double staining (C)) of the hemocytes prepared from the cynomolgus monkey embryonic stem cells under the serum-free condition by the above-described method. A variety of myeloid lineage cells are observed. From Fig. 25, it was confirmed by Wright-Giemsa staining, myeloperoxidase staining (POX staining), esterase double staining or the like, that the non-adherent cells were cells which were present in a variety of differentiation stages of a myeloid lineage cell (including myeloblast, promyelocyte, myelocyte, metamyelocyte, neutrophil, monocyte, and macrophage).

### Example 8

### Induction of differentiation of a human embryonic stem cell into a blood cell (a hematopoietic stem cell and a mature hemocyte) without using feeder cells and reproduction of hemocytes (using bovine fetal serum)

### (1) Preparation of differentiation medium

Cytokines consisting of a final concentration 20 ng/ml of vascular endothelial growth factor (VEGF), a final concentration 20 ng/ml of insulin-like growth factor 2 (IGF2), a final concentration 10 ng/ml of bone morphogenetic protein-4 (BMP-4), a final concentration 5 ng/ml of oncostatin M (OSM), a final concentration 5 ng/ml of fibroblast growth factor (FGF2), 50 ng/ml of stem cell factor (SCF), a final concentration 50 ng/ml of Flt-3 ligand, and a concentration 50 ng/ml of granulocyte colony stimulating factor (G-CSF) were added to a differentiation medium 1-1 {composition: Iscove's modified Dulbecco's medium (IMDM) [manufactured by Sigma Chemical Co.], 15% by weight of heat-inactivating bovine fetal serum [PAA Laboratories GmbH], 1 mM of β-mercaptoethanol [manufactured by Sigma Chemical Co.], and 2 mM of L-glutamine [manufactured by Invitrogen Corp.].

### (2) Technique of inducing differentiation 1 (step of embryoid body formation)

After colonies of human embryonic stem cells were recovered with a cytodetachment solution, suspension-culture was conducted using the differentiation medium (with addition of cytokine) in a non-adhesive culture container (diameter 6 cm culture dish etc.) coated with polyhydroxyethyl methacrylate (poly(2-hydryoxyethyl methacrylate), manufactured by Sigma Chemical Co.), or Hydrocell (manufactured by CellSeed). After 3 to 8 days, an embryoid body (or embroyid body-analogous cellular aggregate) was formed.

### (3) Technique of inducing differentiation 2 (step of formation of "specific precursor cells" (organization consisting of sac-like structure and spherical cell population))

The embryoid body (or embroyid body-analogous cellular aggregate) formed by means of the technique 1 was recovered from the culture supernatant by centrifugation, and adhesion-cultured at 35°C and 5 vol% CO₂ in a CO₂ incubator using the differentiation medium (with addition of cytokine), on a culture dish (culture dish having a diameter of 6 cm or 10 cm) coated with 0.1% gelatin [manufactured by Sigma Chemical Co.]. Thereafter, the medium was exchanged every 3 to 4 days. An aggregate of the human embryonic stem cells continued to grow with spreading transversally and, after about 2 weeks, "specific precursor cells" (organization consisting of sac-like structure and spherical cell population) were formed (one was formed per aggregate) as shown in Fig. 26, from the area around a center of a region where the aggregate was originally located. Fig. 26 shows production of the "specific precursor cells" (organization consisting of sac-like structure and spherical cell population) of vascular endothelial precursor cells and blood cells from the human embryonic stem cells, by the above-described method.

### (4) Technique of inducing differentiation 3 (step of a hemocyte reproduction)

Before spherical cells were completely filled into the "sac-like structure" formed by means of the technique 3, the spherical cells in the inside of the sac-like structure were slowly released into a culture solution by making a slight notch near a bottom of the sac-like structure without destroying a structure of sac-like structure itself. If the spherical cells were completely filled in the sac-like structure, viability of the spherical cells is decreased. The spherical cells were recovered by centrifugation and the medium was exchanged every 3 to 4 days to continue the culture. Regarding the non-adherent cells recovered from the culture supernatant, assessment for a blood cell was conducted by adequately confirming as follows:(1) the colony forming ability by a hematopoietic colony assay (Methocult^{®} GF⁺ H4535 (Stemcell Technologies Inc.)), (2) observation of morphology by Wright-Giemsa staining, (3) the presence or the absence of the specific enzyme activity by special staining such as myeloperoxidase staining (POX staining), esterase double staining, neutrophilic alkaline phosphatase staining (NAP staining) and the like, (4) expression of various markers (CD34, CD45 etc.) on the cell surface by flow cytometry. Results are shown in Figs. 27 and 28.

Fig. 27 shows Wright-Giemsa staining image (A) and special staining (esterase double staining (B), and neutrophilic alkaline phosphatase staining (C)) of the mature hemocytes (non-adherent cells) prepared by the above-described method. A variety of myeloid lineage cells, that is, cells which are present in each of differentiation stages including stages from a myeloblast to a mature hemocyte (netrophil and macrophage) are observed.
Fig. 28 shows the results of expressions of CD34 and CD45 on the non-adherent cells prepared from the human embryo stem cells in the presence of bovine fetal serum by the above-described method, confirmed by flow cytometry. On almost all cells, CD45 which is a pan-hematopoietic cell marker is expressed, and it can be understood that differentiation into hemocytes is induced at the very high efficiency. In addition, since about 10% of CD34-positive cells were detected, it was confirmed that hematopoietic stem cells were also present.
In other words, production of cells which were present in a variety of differentiation stages of myeloid lineage cells (including myeloblast, promyelocyte, myelocyte, metamyelocyte, neutrophil, monocyte, and macrophage) was confirmed (see Fig. 27). In addition, it was confirmed that expression of a CD45 antigen which is a pan-hematopoietic cell marker was found in almost 100% of the cells (see Fig. 28), and the hemocyte differentiation efficiency is very high, and an almost pure population consisting of an approximately only hemocyte was obtained. In addition, about less than 10% of a CD34 positive (i.e. CD34-positive, CD45-positive double positive cell) was present (see Fig. 28), and it was confirmed that a hematopoietic stem cell (or hematopoietic precursor cell equivalent to hematopoietic stem cell) was produced.

In addition, even if the spherical cells were released by cutting the "sac-like structure" finely using a microknife, the opening was rapidly closed. When the culture was continued, the spherical cells were filled in the "sac-like structure" again in about a few days. Then, before the spherical cells were completely filled, the "sac-like structure" was cut finely using a microknife again to release the spherical cells. Such procedure can be performed repeatedly, that is, the non-adherent cells (hemocytes) were continued to be reproduced.

### (5) Technique of inducing differentiation 4 (step of separation and concentration of a specific lineage hemocyte)

Neutrophils were concentrated from the hemocytes prepared by the method described in the technique 3 by a density gradient centrifugation method using Lymphoprep^{®} (manufactured by Sekisui Medical Co., Ltd). When neutrophils were given blue-purple color by neutrophilic alkaline phosphatase staining and observed under a microscope, it was confirmed that almost all cells consist of neutrophil after concentration (B) as compared with before concentration (A), as shown in Fig. 29.
Fig. 29 shows results of concentration of neutrophils from the hemocytes prepared by the above-described method by means of a density gradient centrifugation method using Lymphoprep^{®} (manufactured by Sekisui Medical Co., Ltd). Herein, neutrophils are stained blue purple by neutrophilic alkaline phosphase staining, and almost all cells become neutrophil after concentration (B) as compared with before concentration (A).

### Example 9

### Induction of differentiation of a human embryonic stem cell into a blood cell (a hematopoietic stem cell and a mature hemocyte) without using feeder cells, and reproduction of hemocytes (using serum-free medium)

### (1) Preparation of differentiation medium

For the culture of human embryonic stem cells, cytokines consisting of a final concentration 20 ng/ml of vascular endothelial growth factor (VEGF), a final concentration 20 ng/ml of insulin-like growth factor 2 (IGF2), a final concentration 120 ng/ml of bone morphogenetic protein 4 (BMP-4), a final concentration 5 ng/ml of oncostatin M (OSM), a final concentration 5 ng/ml of fibroblast growth factor 2 (FGF2), 50 ng of stem cell factor (SCF), a final concentration 50 ng/ml of Flt3-ligand, and a concentration 50 ng/ml of granulocyte colony stimulating factor (G-CSF) were added to a differentiation medium 1-2 {composition: Iscove's modified Dulbecco's medium (IMDM) [manufactured by Sigma Chemical Co.], 15% by weight of KNOCKOUT^{®} SR [manufactured by Invitrogen Corp.] 1mM β-mercaptoethanol [manufactured by Sigma Chemical Co.], and 2 mM L-glutamine [manufactured by Invitrogen Corp.]}.

### (2) Technique of inducing differentiation 1 (step of embryoid body formation)

After colonies of human embryonic stem cells were recovered with a cytodetachment solution, suspension-culture was conducted in the differentiation medium (with addition of cytokine) using a non-adhesive culture container (diameter 6 cm culture dish etc.) coated with polyhydroxyethyl methacrylate (poly (2-hydroxyethyl methacrylate), manufactured by Sigma Chemical Co.), or Hydrocell (manufactured by CellSeed). After 3 to 8 days, an embryoid body (or embroyid body-analogous cellular aggregate) was formed.

### (3) Technique of inducing differentiation 2 (step of formation of "specific precursor cells" (organization consisting of sac-like structure and spherical cell population))

The embryoid body (or embroyid body-analogous cellular aggregate) formed by means of the technique 1 was recovered from the culture supernatant by centrifugation, and adhesion-cultured at 37°C and 5 vol% CO₂ in a CO₂ incubator using the differentiation medium (with addition of cytokine) on a culture dish (culture dish of diameter 6 cm or 10 cm) coated with 0.1% gelatin [manufactured by Sigma Chemical Co.]. Thereafter, the medium was exchanged every 3 to 4 days. The aggregate of the human embryonic stem cells continued to grow with spreading transversally and, after about 2 weeks, "specific precursor cells" (organization consisting of sac-like structure and spherical cell population) were formed from the area around a center of a region where the aggregate was originally located.

### (5) Technique of inducing differentiation 3 (step of hemocyte reproduction)

Before spherical cells were completely filled into a "sac-like structure" formed by means of the technique 2, a bottom of a sac-like structure was cut finely using a microknife (Stemcell knife (manufactured by Swemed) etc.) to release the spherical cells contained in the inside of the sac-like structure into the culture supernatant. When the spherical cells are completely filled in a sac-like structure, viability of the spherical cell is decreased. The spherical cells were recovered by centrifuging the culture supernatant, and the medium was exchanged every 3 to 4 days to continue the culture. Regarding the non-adherent cells recovered from the culture supernatant, assessment for a blood cell was conducted by confirming (1) the colony forming ability by a hematopoietic colony assay (using Methocult^{®}) GS⁺ H4535 (Stemcell Technologies Inc.)), (2) observation of morphology by Wright-Giemsa staining, (3) the presence or the absence of the specific enzyme activity by special staining such as myeloperoxidase staining (POX staining), esterase double staining, neutrophilic alkaline phosphatase staining (NAP staining) and the like, and (4) expression of various markers (CD34, CD54 etc.) on the cell surface by flow cytometry. Results are shown in Fig. 31.

Fig. 30 shows a result confirmed by flow cytometry regarding expression of CD45 on the non-adherent cells prepared from the human embryonic stem cells under the serum-free condition (using KNOCKOUZ^{®} SR) by the above-described method. On almost all cells, CD45 which is a genaral hemocyte marker is expressed, and it can be understood that hemocyte differentiation is induced at the very high efficiency. In the light of the foregoing, expression of a CD45 antigen which is a pan-hematopoietic cell marker was almost 100%, and the hemocyte differentiation efficiency was very high using serum free culturing, and an approximately pure population consisting of an approximately only hemocyte was obtained.

### Example 10

### Assessment of pluripotency of a cynomolgus monkey embryonic stem cell

According to the undifferentiation-maintenance culture method of Example 1, cynomolgus monkey embryonic stem cells (1×10⁶) which had been subcultured 22 times were transplanted under a testis membrane of immunodeficient mice (SCID mice). After 8 weeks, tumor formation was visually confirmed in testis of all transplanted mice. These tumors were removed, fixed with formalin, a thin section was prepared to be subjected to hematoxylin/eosin staining (HE staining), and a histological test was conducted.
As a result, as shown in Fig. 32, because the tumor has tridermic components of an ectoderm component (neuroepithelial cell; Fig. a, tooth enamel epithelium; Fig. d), a mesoderm component (smooth muscle; Fig. b, tooth dentine; Fig. d), and an endoderm component (intestinal tract epithelium; Fig. b, secretory gland tissue; Fig. c), it was confirmed that a tumor formed in a testis was teratoma.
Therefore, it was confirmed that the cynomolgus monkey embryonic stem cells which were subcultured to be maintained according to the undifferentiation-maintenance culture method of Example 1 has the teratoma forming ability, and it was demonstrated that the pluripotency was retained.

### Example 11

### Assessment for pluripotency of a human Embryonic stem cell

The human embryonic stem cells (khES-1) (3 × 10⁶) which had been subcultured 20 times by the undifferentiation-maintenance culture method of Example 2 were transplanted into a quadriceps femoris muscle of an immunodeficient mouse (SCID mouse). In all transplanted mice, tumor formation was visually confirmed in a quadriceps femoris muscle after 8 weeks. These tumors were removed, fixed with formalin, a thin section was prepared to be subjected to hematoxylin/eosin staining (HE staining), and a histological test was conducted.
As a result, as shown in Fig. 33, because the tumor has tridermic components of an ectroderm component (neuroendothelial cell; Fig. a, pigment epithelium; Fig. b, sebaceous gland; Fig. h), a mesoderm component (bone; Fig. d, adipocyte; Fig. e, cartilage Fig. f, and an endoderm component (secretory gland; Fig c and Fig. g), it was confirmed that the tumor formed in a quadriceps femoris muscle was teratoma.
Therefore, it was confirmed that the human embryonic stem cells which were subcultured to be maintained by the undifferentiation-maintenance culture method of Example 2 has the teratoma forming ability, and it was demonstrated that the pluripotency is retained.

### Example 12

### Method for inducing differentiation of a cynomolgus monkey embryonic stem cell into a vascular endothelial cell/a blood cell without using feeder cells (method using bovine fetal serum)

Embroyid body-analogous cellular aggregate cells are prepared by the Hanging/Dropping method using the differentiation medium 1-1 (with addition of cytokine) used in Example 4. Specifically, cynomolgus monkey embryonic stem cells are recovered with a cytodetachment solution, and are treated with a 0.25% trypsin solution [manufactured by Invitrogen Corp.] at 37°C for 5 minutes to allow the cells to be dispersed and individually separated from each other. 3,000 cynomolgus monkey embryonic stem cells are suspended in 30 µl of the differentiation medium 1-1 (with addition of cytokine), and a drop of the suspension is placed on a back side of a lid of a culture dish having the diameter of 10 cm using a micropipette (it is possible to place about 20 to 30 drops on one culture dish). The cells were suspension-cultured at 37°C and 5 vol% CO₂ for 3 days in a CO₂ incubator which was filled with water to prevent the culture from being dried up. Since formation of a cellular aggregate can be visually confirmed after three days, the aggregate was recovered by rinsing a lid surface of the culture dish, and adhesion-cultured at 37°C and 5 vol% CO₂ in a CO₂ incubator using the differentiation medium 1-1 (with addition of cytokine), on a culture dish (diameter 10 cm or 6 cm) coated with 0.1% gelatin [manufactured by Sigma Chemical Co.]. Thereafter, the medium was exchanged every 3 to 4 days. The aggregate of the cynomolgus monkey embryonic stem cells continued to grow with spreading transversally and, after about 2 weeks, specific precursor cells (organization consisting of sac-like structure and spherical cell population) were formed from the area around a center of region where the aggregate was originally located (one was formed per aggregate), as is seen in Fig. 12.

Before the spherical cells were completely filled in the sac-like structure, the spherical cells included in the inside of the sac-like structure were slowly releasing into the culture solution by finely cutting a bottom of a sac-like structure without destroying the structure of sac-like structure itself, using a microknife (Stemcell knife, manufactured by SweMed). If spherical cells are completely filled in the sac-like structure, viabilty of the spherical cells are decreased. The spherical cells were recovered by centrifuging the culture supernatant.

The hemocyte production was confirmed by conducting a hematopoietic colony assay of the recovered spherical cells using a colony assay kit equipped with a semisolid medium containing methylcellulose (Methocult^{®}) GF + H4535 (Stemcell Technologies Inc.). Wright-Giemsa staining (A), and special staining (myeloperoxidase staining (B), esterase double staining (C)) of the mature hemocytes produced from the spherical cells were indicated like Fig. 13. A variety of myeloid lineage cells, that is, cells which were present in each of various differentiation stages including from a myeloblast to a mature hemocyte (neutrophil and macrophage) were observed.

Since a cell group constituting a "wall surface" and a "base" of the sac-like structure actively proliferates, a cobblestone-like cell group was expanded all around a sac-structure with time. Regarding these cobblestone cells and the wall surface cells of the sac-like structure, as shown in upper column of Fig. 34, VE-cadherin which is an intercellular adhesion molecule known as a "pan-vascular endothelial cell marker" and a "vascular endothelial cell-specific marker" is expressed at the boundary of all cells. In addition, as reported by Lampugnami et al. (Journal of Cell Biology, Vol. 129, p203-217, 1995), in a cell group of a "front region" in which cell movement is active, cell membrane localization of VE-cadherin became unclear, and an expression of VE-cadherin was found mainly in the cytoplasm (Fig. 34, right column). On a further external side, large flat cells which do not express VE-cadherin were dispersed (Fig. 34, right lower figure, arrow). These "non-vascular endothelial cells" which are present in small numbers are proliferating cell nuclear antigen (PCLA)-negative, and it can be understood that the cells were not in the proliferation. Actually, these VE-cadherin-negatitve "non-vascular endothelial cells" were rapidly expelled by a cobblestone cell which was a VE-cadherin-positive "vascular endothelial cell" with time.

As describe above, when the medium was exchanged and the culture of the sac-like structure was continued, the culture dish was dominated by "vascular endothelial cells having the active proliferating ability" in about a few days. Then, if cells in the culture dish are recovered as an aggregate and subculture of it is continued, it becomes possible to achieve the "expansion of a vascular endothelial cell culture". Specifically, the subculture was conducted as follows. The cells were detached and recovered by a reaction at 37°C for 5 minutes with a trypsin/EDTA solution [manufactured by Invitrogen Corp.], and cultured using a differentiation medium 1-1 (with addition of cytokine) in a new culture dish (diameter 10 cm or 6 cm) coated with 0.1% gelatin [manufactured by Sigma Chemical Co.]. Thereafter, the cells were detached using a trypsin/EDTA solution every 3 to 4 days, and about 1/3 of the cells were continued to be subcultured.

As shown in Fig. 35, it was confirmed by immunostaining that in all of cells subcultured by the above-described procedure, VE-cadherin was expressed at least within the cells. Further, as shown in Fig. 35, N-cadherin, which is an adhesion factor known as being expressed in a vascular endothelial cell, was expressed on almost all cells, as measured by immunostaining, and it was shown that clear cell membrane localization was recognized. As shown in Fig. 31, this is a nature that a primary vascular endothelial cell obtained from a living body has been lost, and thus this is a very interesting finding (see and compare Fig. 35 and Fig. 31).

Further, in order to confirm localization of VE-cadherin on a "cell membrane", analysis by flow cytometry was conducted. As shown in Fig. 36, cells expressing VE-cadherin and PECAM1 which is a mature vascular endothelial cell marker on the "cell membrane" were about 10 to 20% at the initial stage of the subculture, and the ratio of the cells was remarkably increased during the subculture resulting in reaching about 40% at the middle stage of the subculture and about 80% at the late stage of the subculture. Thus, at any stage, the "VE-cadherin/PECAM1-positive mature vascular endothelial cells" are present at much higher concentration than that reported in the previous method (<2%). Although it was reported that because the VE-cadherin-positive vascular endothelial cells rapidly disappear with the subculture according to the previous method, expansion of the culture can not be achieved by the previous method, using the present method, it was made clear that the VE-cadherin/PECAM1-positive cells became concentrated with subculturing, and thus, the present method has a remarkable characteristic which has not been previously reported.

In addition, it was confirmed that a pericyte was totally absent in the vascular endothelial cells which were induced to be differentiated according to the present method, although the vascular endothelial cells prepared by the previous method were contaminated with so large amount of pericytes that it may be impossible to expand the culture of the vascular endothelial cells. Thus, as shown in Fig. 37, expressions of platelet-derived growth factor receptor β (PDGFR β), actin, alpha-2, smooth muscle, and aorta (ACTA2), each of which is a marker of pericyte, are not detected. In addition, as shown in Fig. 38, because expression of Nanog which is a marker of an undifferentiated embryonic stem cell is not recognized, it is confirmed that the culture is not contaminated with undifferentiated embryonic stem cells.

In the light of the foregoing, in the cell population which is induced to be differentiated by the present method, all cells express VE-cadherin at least within the cells and are not contaminated with a pericyte and an undifferentiated ES cell. In addition, "mature vascular endothelial cells" expressing VE-cadherin and PECAM1 on a cell membrane are produced and the purity is 10% or more at the initial stage of the subculture, and reaches near 90% at late stage of the subculture.
By recovering the VE-cadherin-positive cells using a cell sorter at the initial stage of the subculture, the vascular endothelial cells can be concentrated to be highly purified from the initial stage of the subculture. Fig. 39 and Fig. 40 show the results of sorting of the VE-cadherin-positive cell by FACSAria (BD Biosciences) after one time passage of the sac-like structure. As shown in Fig. 39, it was confirmed that cells contained in the VE-cadherin-positive fraction continue a stable expression of VE-cadherin on a cell membrane even if subculturing is repeated. After 5th passage, the VE-cadherin-positive cells were amplified about 160-fold (Fig. 39B). In addition, by immunostaining, localization of VE-cadherin protein was also confirmed at the junction of all cells (Fig. 39C).

On the other hand, as shown in Fig. 40, the cells contained in the VE-cadherin-negative fraction does not express VE-cadherin on the cell membrane, but express VE-cadherin within the cell as described above. They have the cord formation ability (Fig. 40B) and the acetylated low density lipoprotein uptake ability (Fig. 40C). It was confirmed that the cells constitute a cell population committed to a vascular endothelial cell.

In the light of the foregoing, the vascular endothelial cells, which retain the stable proliferating ability and the mature function during the procedure of at least eight times passages and show clear N-cadherin localization on the cell membrane as is seen in a living body, were prepared from cynomolgus monkey embryonic stem cells.

### Example 13

### Transplantation experiment of cynomolgus monkey embryonic stem cell-derived vascular endothelial cells

1 × 10⁶ of C6 rat grioma cells and 1 × 10⁶ of cynomolgus monkey embryonic stem cell-derived vascular endothelial cells (5th passage) were mixed and the mixed cells were cultured overnight. The resulting mixed cells were transplanted under a back skin of an immunodeficient mouse (SCID mouse), while only C6 rat grioma cells were transplanted as a control. As the result, as shown in the following Table 1, a tumor formation was visually confirmed under a skim of all transplanted mice after 3 weeks. These tumor was removed, and measurement of a tumor (size, weight), and apparent observation (Fig. 41a) were conducted. Thereafter, the tumor was fixed with formalin, a thin section was made, and subjected to hematoxylin/eosin staining (HE staining), and a histological test (Fig. 41b) was conducted.

**[Table 1]**

| A novel method for induction of a vascular endothelial cell: assessment of in vivo function | | | | | |
|---|---|---|---|---|---|
| | No. 1 | No. 2 | No. 3 | No. 4 | No. 5 |
| Glioma cell | - | + | + | + | + |
| ES-derived vascular endothelial cell | - | - | + | + | + |
| Macro-observation of tumor (size, color, hardness) | - No tumor formation is recognized | Small White Hard | Medium Hemorrhagic Soft | Large Hemorrhagic Soft | Large Hemorrhagic Soft |
| Size of tumor (cm) | - | 2.0×1.5×1.0 | 2.2×1.5×1.5 | 2.7×2.0×1.5 | 2.5×2.0×1.5 |
| Weight of tumor (g) | - | 1.26 | 1.82 | 2.84 | 3.78 |

In the light of the forgoing, as compared with the case where C6 rat glioma cells alone were transplanted, in the case where mixed culture of C6 rat glioma cells and cynomolgus monkey ES cell-derived vascular endothelial cells were transplanted, the following characteristic was seen: the formed tumor was large, has a color accompanied with dark rouge, is firmly adhered to the periphery, and is easily breeding. In addition, histologically, it was confirmed that the former (the case where C6 rat glioma cells alone were transplanted) shows a solid tumor tissue image consisting of glioma cells (Fig. 41b, left) having little vascular components, while the latter (the case where mixed culture of C6 rat glioma cells and cynomolgus monkey ES cell-derived vascular endothelial cells were transplanted) shows a tumor tissue image rich in vascular components (fill the inner cavity with erythrocyte is recognized) (Fig. 41b, right). Further, in the latter, by immunostaining using anti-human HLA-A, B, C monoclonal antibodies (Clone W6/32, BioLegend) which has been confirmed to widely cross with a primate, it was confirmed that a vascular endothelial cell supporting a vascular inner cavity is stained (Fig. 41c). That is, it was confirmed that vascularization in a glioma tissue was formed by cynomolgus monkey embryonic stem cell-derived vascular endothelial cells.
Therefore, it was demonstrated that vascular endothelial cells prepared from the cynomolgus monkey embryonic stem cells contribute to tumor vascularization in the experiment of transplantation into a mouse.

### Example 14

### Experiment of transplantation of human embryonic stem cell-vascular endothelial cells

Human embryonic stem cells (khES-3) (correspond to one 10 cm culture dish), which were cultured with maintaining the undifferentiated state without using feeder cells and cytokines by the method of Example 2, were detached and recovered with the cytodetachment solution 1 or the cytodetachment solution for primate embryonic stem cell (Reprocell Incorporation) described in Example 2. The resulting cells were suspension-cultured using a generally used low adsorption culture dish (one 6 cm culture dish) in a differentiation medium 1-1 (with addition of cytokine) as described in Example 4 to prepare an embroyid body-analogous cellular aggregate. By adhesion-culturing (culturing on two 10 cm culture dishes) followed by subculturing this cellular aggregate by the method described in Example 4, vascular endothelial cells were induced to be differentiated. It was confirmed that, like the examples using the cynomolgus monkey embryonic stem cells described in Example 4 and Example 12, the vascular endothelial cells were produced from the human embryonic stem cell (20 to 70%) much more efficiently than the previous method, and the ratio of the VE-cadherin/PECAM1 double positive cells were increased accompanying with subculturing (see Fig. 42a). In addition, like the cynomolgus monkey embryonic stem cell-derived endothelial cells, human embryonic stem cell-derived vascular endothelial cells can be subcultured about 8 to 10 times. Further, the functional maturity of the vascular endothelial cells such as Cord formation ability and the acetylated low density lipoprotein uptake ability was confirmed (see Fig. 42b). Then, assessment of the in vivo function of the human embryonic stem cell-derived vascular endothelial cells as prepared above was conducted as follows: a few pieces of about 1 mm square honeycombed collagen sponge were sunk in the suspension solution of the human embryonic stem cell-derived vascular endothelial cells (4th passage) followed by repeating several times pressed and loosened to fill the collagen sponge with the human embryonic stem cell-derived vascular endothelial cells. The cells were cultured for 2 days, and transplanted into an abdominal cavity of an immunodeficient mouse (SCID mouse) (about a few species/animal). After about one month, high-molecular dextran labeled with FITC was injected via a tail vein and, after a few minutes, the collagen sponge was recovered from the abdominal cavity. After formalin fixation, immunostaining was performed using anti-human HLA-A, B, C antibodies (Clone W6/32, BioLegend) and an anti-human PECAM1 antibody (Santa Cruz, sc-8306).

As the result, as shown in Fig. 42c, in the mouse transplanted with the human embryonic cell-derived vascular endothelial cells, a "lumen structure, an inner cavity of which was filled with FITC dextran" was confirmed in the inside of the collagen sponge. The lumen structure was positively stained with both human HLA-A, B, C antibodies (Fig. 42c left), which specifically recognize not mouse cells but cells of a primate including a human, and a human PECAM1 antibody, which is a vascular endothelial cell marker (Fig. 42c, center). In addition, since the human PECAM1 antibody did not stain a vessel structure of a mouse (Fig. 42c, right), it was confirmed that this lumen structure is a "neovascular vessel consisting of human embryonic stem cell-derived vascular endothelial cells" connected to the body circulation.
Therefore, it was confirmed that the vascular endothelial cells prepared from the human embryonic stem cells by the novel method for inducing differentiation into a vascular endothelial cell was directly associated with formation of a "functional neovascular vessel connected to body circulation" (i.e.incorporation into a neovascular vessel) in vivo.

### Example 15

### Analysis of surface marker of a hemocyte

After human embryonic stem cells were induced differentiation by the method of Example 8, cells suspended in the medium on 30th day were recovered and expression of a hemocyte marker, mainly neutrophil, was analyzed by flow cytometry.
As the result, as shown in Fig. 43, expression of CD34 which is a hematopoietic stem/precursor cell marker was low, and expressions of CD54, which is a pan-hematopoietic cell marker, and CD33, which is a pan-leukocyte marker as well as CD11b, which is a neutrophil/monocyte cell marker, were very high (> 90%). In addition, expressions of CD66b and GPI-80, which are a granulocyte marker, were found regarding about 60 to 80% of the cells, and expression of CD16b, which is a neutrophil-specific marker, was found regarding 30% or more of the cells. Further, a lactoferrin, which is contained in a tertiary granule of neutrophil, positive cell was also detected.
Therefore, a hematopoietic cell prepared from the human embryonic stem cell expresses a neutrophil marker at the high rate.

### Example 16

### Experiment of transplantation of human Embryonic stem cell-derived neutrophils

By the method of Example 8, the human embryonic stem cells were induced differentiation and hemocytes (CD66b positive rate is about 60 to 90%) suspending in the medium on 30^{th} day were recovered. About 1×10⁶ cells were intravenously injected into a NOD/SCID/γc^{null} (NOG) mouse in which an air sac had been formed in advance by injecting sterile air subcutaneously. Immediately, zymosan A (1 mg/ml) and human IL-1 β (10 ng/ml) were administered into the air sac and, after 16 hours, the cells were recovered from the air sac, and a positive rate of the human-derived neutrophils was measured by flow cytometry using an anti-human CD66b antibody (Note: it had been confirmed that the antibody reacts with only human neutrophil, but does not react with mouse neutrophil).
As the result, as shown in Fig. 44, in a non-transplanted group, the CD66b-positive cell was not detected, but in a group transplanted with the human ES cell-derived hemocytes, the human CD66b-positive rate was 0.42±0.13%. This value was equivalent to (or more than) a value (0.41%) obtained by the same experiment which was conducted using "leukocyte prepared by coculturing human umbilical blood CD34-positive cells (hematopoietic stem cells) and mouse 0P9 cells (CD66b = 65%)".
Therefore, it was confirmed that neutrophils prepared from the human embryonic stem cells have the migrating ability in vivo.

### Industrial Applicability

According to the present invention, it becomes possible to provide a blood cell and a vascular endothelial cell suitable for a transfusion blood, a transplantation material or the like, stably and at an industrial scale. Since further, the blood cell of the present invention leads to strengthening of natural curing force, its impact on medicine and medical industry is great. Further, taking preparation of a safe transfusion blood, which is substitute of current blood donation, into consideration, possibility of development on giant plant industry is also considered.

## Claims

1. A method for culturing and subculturing primate embryonic stem cells, comprising:
(A) a step of culturing primate embryonic stem cells in a medium containing a protein component without using feeder cells and cytokines in a container coated with an extracellular matrix,
(B) a step of detaching colonies of the embryonic stem cells prepared by the step (A) in the presence of a cytodetachment agent, and
(C) a step of plating the colonies of the embryonic stem cells prepared by the step (B) in a medium containing a protein component without using feeder cells and cytokines in a container coated with an extracellular matrix.

2. The culture and subculture method according to claim 1, wherein in the step (A), the primate embryonic stem cells are cultured until the size of the colony of the primate embryonic stem cells becomes about 2-4 times larger.

3. The culture and subculture method according to claim 1 or 2, wherein the protein component in the step (A) is serum albumin.

4. The culture and subculture method according to any one of claims 1 to 3, wherein the cytodetachment agent in the step (B) is at least one selected from the group consisting of trypsin, collagenase, and dispase.

5. The culture and subculture method according to any one of claims 1 to 4, wherein the extracellular matrix in the step (A) is at least one selected from the group consisting of human collagen, human laminin, human vitronectin, human fibronectin and human serum, as well as a degradation product of them and a synthetic peptide of them.

6. A method for preparing blood cells and/or vascular endothelial precursor cells from a primate embryonic stem cell, comprising:
(A) a step of suspension-culturing primate embryonic stem cells in a medium containing serum or a serum-free medium containing a serum substitute in the presence of a cytokine to prepare an embryoid body or an embroyid body-analogous cellular aggregate,
(B) a step of adhesion-culturing the embryoid body or the embroyid body-analogous cellular aggregate obtained in the step (A) in the presence of the cytokine to prepare specific precursor cells containing non-adherent cells and adherent cells, and
(C) a step of separating the non-adherent cells and the adherent cells from the specific precursor cells obtained in the step (B).

7. The method for preparing vascular endothelial precursor cells and/or blood cells from a primate embryonic stem cell according to claim 6, wherein culturing of the step (A) is performed until an embryoid body-like cell aggregate is formed.

8. The method for preparing vascular endothelial precursor cells and/or blood cells from a primate embryonic stem cell according to claim 6 or 7, wherein the cytokine is at least one selected from the group consisting of vascular endothelial growth factor (VEGF), bone morphogenetic protein 4 (BMP4), stem cell factor (SCF), Flt3-ligand (FL), interleukin 6 (IL6), interleukin 3 (IL3), glanulocyte colony stimulating factor (G-CSF), megakaryocyte proliferation factor (TPO), oncostatin M (OSM), fibroblast growth factor 2 (FGF2) and granulocyte macrophage colony stimulating factor (GM-CSF).

9. The method for preparing vascular endothelial precursor cells and/or blood cells from a primate embryonic stem cell according to any one of claims 6 to 8, wherein a cytodetachment agent is used for separating adherent cells of specific precursor cells in the step (C).

10. The method for preparing vascular endothelial precursor cells and/or blood cells from a primate embryonic stem cell according to claim 9, wherein the cytodetachment agent is at least one selected from the group consisting of trypsin, collagenase, and dispase.

11. A method for preparing blood cells, myeloid lineage cells, hematopoietic stroma cells and/or hematopoietic stem cells from a primate embryonic stem cell, comprising:
(A) a step of suspension-culturing primate embryonic stem cells in a medium containing serum or a serum-free medium containing a serum substitute in the presence of cytokine to prepare an embryoid body or an embroyid body-analogous cellular aggregate,
(B) a step of adhesion-culturing the embryoid body or the embroyid body-analogous cellular aggregate obtained in the step (A) in the presence of a cytokine to produce specific precursor cells containing non-adherent cells and an adherent cells, and
(C) a step of culturing the specific precursor cells obtained in the step (B) together with separating the non-adherent cells.

12. The method for preparing blood cells, myeloid lineage cells, hematopoietic stroma cells and/or hematopoietic stem cells from a primate embryonic stem cell according to claim 11, wherein culturing of the step (A) is performed until an embryoid body is formed.

13. The method for preparing blood cells, myeloid lineage cells, hematopoietic stroma cells and/or hematopoietic stem cells from a primate embryonic stem cell according to claim 11 or 12, wherein the cytokine is at least one selected from the group consisting of vascular endothelial growth factor (VEGF), bone morphogenetic protein 4 (BMP4), stem cell factor (SCF), Flt3-ligand (FL), interleukin 6 (IL6), interleukin 3 (IL3), glanulocyte colony stimulating factor (G-CSF), megakaryocyte proliferation factor (TPO), oncostatin M (OSM), fibroblast growth factor 2 (FGF2) and granulocyte macrophage colony stimulating factor (GM-CSF).

14. The method for preparing blood cells, myeloid lineage cells, hematopoietic stroma cells and/or hematopoietic stem cells from a primate embryonic stem cell according to any one of claims 11 to 13, wherein a cytodetachment agent is used for separating adherent cells of specific precursor cells, in the step (C).

15. The method for preparing blood cells, myeloid lineage cells, hematopoietic stroma cells and/or hematopoietic stem cells from a primate embryonic stem cell according to claim 14, wherein the cytodetachment agent is at least one selected from the group consisting of trypsin, collagenase, and dispase.

16. A substantially isolated vascular endothelial precursor cell which is induced to be differentiated from a primate embryonic stem cell by the method according to any one of claims 6 to 10.

17. A substantially isolated blood cell which is induced to be differentiated from a primate embryonic stem cell by the method according to any one of claims 6 to 14.

18. A substantially isolated hematopoietic stroma cell which is induced to be differentiated from a primate embryonic stem cell by the method according to any one of claims 11 to 14.

19. A substantially isolated hematopoietic stem cell which is induced to be differentiated from a primate embryonic stem cell by the method according to any one of claims 11 to 14.

20. A substantially isolated myeloid lineage cell which is induced to be differentiated from a primate embryonic stem cell by the method according to any one of claims 11 to 14.

21. A composition comprising the substantially isolated vascular endothelial precursor cell, blood cell, hematopoietic stroma cell, or hematopoietic stem cell according to any one of claims 16 to 19.
